# EUROPEAN PATENT APPLICATION

(11) **EP 4 023 678 A1**
(43) Date of publication of application: **06.07.2022**
(21) Application number: 20852367.0
(22) Date of filing: 06.08.2020
(51) Int. Cl.: C07K 19/00, C12N 5/10, A61P 35/00

(54) **CHIMERIC ANTIGEN RECEPTOR AND IMMUNE EFFECTOR CELL EXPRESSING CHIMERIC ANTIGEN RECEPTOR**

(30) Priority: 14.08.2019 CN 201910750137
(71) Applicant: Boyuan Runsheng Pharma (Hangzhou) Co., Ltd., Hangzhou, Zhejiang 310012 (CN)
(72) Inventor: SUN, Chuang, Hangzhou, Zhejiang 310012 (CN); FENG, Xin-Hua, Hangzhou, Zhejiang 310012 (CN); ZHAO, Bin, Hangzhou, Zhejiang 310012 (CN)
(74) Representative: Elzaburu S.L.P.
(86) International application number: PCT/CN2020/107484
(87) International publication number: WO 2021/027687

(57) **Abstract**

Provided are a chimeric antigen receptor capable of targeting B7-H3, a nucleic acid molecule encoding the chimeric antigen receptor, a nucleic acid construct comprising the nucleic acid molecule, an immune effector cell expressing the chimeric antigen receptor, and use thereof. The chimeric antigen receptor comprises an anti-B7-H3 binding domain, a hinge region, a transmembrane domain and a signal transduction domain. Further provided are a composition and a method for diagnosing, treating or preventing tumors that express B7-H3.

SEQUENCE LISTING

<110>  BOYUAN RUNSHENG PHARMA (HANGZHOU) CO., LTD.

<120>  CHIMERIC ANTIGEN RECEPTOR AND IMMUNE EFFECTOR CELL EXPRESSING
CHIMERIC ANTIGEN RECEPTOR

<130>  MTPIEP20069

<140>  PCT/CN2020/107484
<141>  2020-08-06

<150>  CN201910750137.1
<151>  2019-08-14

<160>  130

<170>  SIPOSequenceListing 1.0

<210>  1
<211>  804
<212>  DNA
<213>  Artificial Sequence

<220>
<223>  synthetic nucleic acid

<400>  1
atggagtttg ggctgagctg gctttttctt gtggctattt taaaaggtgt ccagtgctct      60
agagacatcc agatgacaca gagccctagc agcctgtctg ccagcgtggg agacagagtg     120
accatcacct gtcaggcctc tcagaacgtg cggacagccg tggcttggta tcagcagaag     180
cctggcaagg cccctaaggc tctgatctac ctgcctagca acagactgac cggcgtgccc     240
tctagattca gcggctctgg ctctggcacc gacttcacct tcacaatcag cagcctgcag     300
cctgaggata tcgctaccta cttctgcctg caacactgga actacccttt caccttcggc     360
cagggcacca agctggaaat caagggtggt ggtggttctg gtggtggtgg ttctggcggc     420
ggcggctccg gtggtggtgg atccgaggtt cagctgcagc agtctggacc tgagctggtt     480
aagcctggcg cctccgtgaa gatcccttgc aagacaagcg gctacatctt caccagctac     540
tggatccact gggtcaagca gagccacggc aagagcctgg aatggatcgg cagaatctac     600
cccggcgtgg acagcatcta ctacaacgag aagttcaagg gcaaagccac actgaccgcc     660
gacaagagca gcagcacagc ctacatggaa ctgagaagcc tgaccagcga ggataccgcc     720
gtgtacttct gtgccaccat cagctacgac tacgattaca gcatggacta ctggggccag     780
ggcaccaccg tgacagtttc ttct                                            804

<210>  2
<211>  268
<212>  PRT
<213>  Artificial Sequence

<220>
<223>  synthetic protein

<400>  2
Met Glu Phe Gly Leu Ser Trp Leu Phe Leu Val Ala Ile Leu Lys Gly
1               5                   10                  15
Val Gln Cys Ser Arg Asp Ile Gln Met Thr Gln Ser Pro Ser Ser Leu
            20                  25                  30
Ser Ala Ser Val Gly Asp Arg Val Thr Ile Thr Cys Gln Ala Ser Gln
        35                  40                  45
Asn Val Arg Thr Ala Val Ala Trp Tyr Gln Gln Lys Pro Gly Lys Ala
    50                  55                  60
Pro Lys Ala Leu Ile Tyr Leu Pro Ser Asn Arg Leu Thr Gly Val Pro
65                  70                  75                  80
Ser Arg Phe Ser Gly Ser Gly Ser Gly Thr Asp Phe Thr Phe Thr Ile

                    85                  90                  95
Ser Ser Leu Gln Pro Glu Asp Ile Ala Thr Tyr Phe Cys Leu Gln His
            100                 105                 110
Trp Asn Tyr Pro Phe Thr Phe Gly Gln Gly Thr Lys Leu Glu Ile Lys
        115                 120                 125
Gly Gly Gly Gly Ser Gly Gly Gly Gly Ser Gly Gly Gly Gly Ser Gly
    130                 135                 140
Gly Gly Gly Ser Glu Val Gln Leu Gln Gln Ser Gly Pro Glu Leu Val
145                 150                 155                 160
Lys Pro Gly Ala Ser Val Lys Ile Pro Cys Lys Thr Ser Gly Tyr Ile
                165                 170                 175
Phe Thr Ser Tyr Trp Ile His Trp Val Lys Gln Ser His Gly Lys Ser
            180                 185                 190
Leu Glu Trp Ile Gly Arg Ile Tyr Pro Gly Val Asp Ser Ile Tyr Tyr
        195                 200                 205
Asn Glu Lys Phe Lys Gly Lys Ala Thr Leu Thr Ala Asp Lys Ser Ser
    210                 215                 220
Ser Thr Ala Tyr Met Glu Leu Arg Ser Leu Thr Ser Glu Asp Thr Ala
225                 230                 235                 240
Val Tyr Phe Cys Ala Thr Ile Ser Tyr Asp Tyr Asp Tyr Ser Met Asp
                245                 250                 255
Tyr Trp Gly Gln Gly Thr Thr Val Thr Val Ser Ser
            260                 265

<210>  3
<211>  321
<212>  DNA
<213>  Artificial Sequence

<220>
<223>  synthetic nucleic acid

<400>  3
gacatccaga tgacacagag ccctagcagc ctgtctgcca gcgtgggaga cagagtgacc      60
atcacctgtc aggcctctca gaacgtgcgg acagccgtgg cttggtatca gcagaagcct     120
ggcaaggccc ctaaggctct gatctacctg cctagcaaca gactgaccgg cgtgccctct     180
agattcagcg gctctggctc tggcaccgac ttcaccttca caatcagcag cctgcagcct     240
gaggatatcg ctacctactt ctgcctgcaa cactggaact accctttcac cttcggccag     300
ggcaccaagc tggaaatcaa g                                               321

<210>  4
<211>  107
<212>  PRT
<213>  Artificial Sequence

<220>
<223>  synthetic protein

<400>  4
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Val Arg Thr Ala
            20                  25                  30
Val Ala Trp Tyr Gln Gln Lys Pro Gly Lys Ala Pro Lys Ala Leu Ile
        35                  40                  45
Tyr Leu Pro Ser Asn Arg Leu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Phe Cys Leu Gln His Trp Asn Tyr Pro Phe
                85                  90                  95
Thr Phe Gly Gln Gly Thr Lys Leu Glu Ile Lys
            100                 105

<210>  5
<211>  11
<212>  PRT
<213>  Artificial Sequence

<220>
<223>  synthetic protein

<400>  5
Gln Ala Ser Gln Asn Val Arg Thr Ala Val Ala
1               5                   10

<210>  6
<211>  7
<212>  PRT
<213>  Artificial Sequence

<220>
<223>  synthetic protein

<400>  6
Leu Pro Ser Asn Arg Leu Thr
1               5

<210>  7
<211>  9
<212>  PRT
<213>  Artificial Sequence

<220>
<223>  synthetic protein

<400>  7
Leu Gln His Trp Asn Tyr Pro Phe Thr
1               5

<210>  8
<211>  360
<212>  DNA
<213>  Artificial Sequence

<220>
<223>  synthetic nucleic acid

<400>  8
gaggttcagc tgcagcagtc tggacctgag ctggttaagc ctggcgcctc cgtgaagatc        60
ccttgcaaga caagcggcta catcttcacc agctactgga tccactgggt caagcagagc       120
cacggcaaga gcctggaatg gatcggcaga atctaccccg gcgtggacag catctactac       180
aacgagaagt tcaagggcaa agccacactg accgccgaca agagcagcag cacagcctac       240
atggaactga gaagcctgac cagcgaggat accgccgtgt acttctgtgc caccatcagc       300
tacgactacg attacagcat ggactactgg ggccagggca ccaccgtgac agtttcttct       360

<210>  9
<211>  118
<212>  PRT
<213>  Artificial Sequence

<220>
<223>  synthetic protein

<400>  9
Gln Leu Gln Gln Ser Gly Pro Glu Leu Val Lys Pro Gly Ala Ser Val

1               5                   10                  15
Lys Ile Pro Cys Lys Thr Ser Gly Tyr Ile Phe Thr Ser Tyr Trp Ile
            20                  25                  30
His Trp Val Lys Gln Ser His Gly Lys Ser Leu Glu Trp Ile Gly Arg
        35                  40                  45
Ile Tyr Pro Gly Val Asp Ser Ile Tyr Tyr Asn Glu Lys Phe Lys Gly
    50                  55                  60
Lys Ala Thr Leu Thr Ala Asp Lys Ser Ser Ser Thr Ala Tyr Met Glu
65                  70                  75                  80
Leu Arg Ser Leu Thr Ser Glu Asp Thr Ala Val Tyr Phe Cys Ala Thr
                85                  90                  95
Ile Ser Tyr Asp Tyr Asp Tyr Ser Met Asp Tyr Trp Gly Gln Gly Thr
            100                 105                 110
Thr Val Thr Val Ser Ser
        115

<210>  10
<211>  10
<212>  PRT
<213>  Artificial Sequence

<220>
<223>  synthetic protein

<400>  10
Gly Tyr Ile Phe Thr Ser Tyr Trp Ile His
1               5                   10

<210>  11
<211>  17
<212>  PRT
<213>  Artificial Sequence

<220>
<223>  synthetic protein

<400>  11
Arg Ile Tyr Pro Gly Val Asp Ser Ile Tyr Tyr Asn Glu Lys Phe Lys
1               5                   10                  15
Gly

<210>  12
<211>  13
<212>  PRT
<213>  Artificial Sequence

<220>
<223>  synthetic protein

<400>  12
Ala Thr Ile Ser Tyr Asp Tyr Asp Tyr Ser Met Asp Tyr
1               5                   10

<210>  13
<211>  798
<212>  DNA
<213>  Artificial Sequence

<220>
<223>  synthetic nucleic acid

<400>  13

atggagtttg ggctgagctg gctttttctt gtggctattt taaaaggtgt ccagtgctct 60
agagatgttg tgctaactca gtctccagcc accctgtctg tgactccagg agatagcgtc 120
agtctttcct gcagggccag ccaaagtatt agcatcaacc tacactggta tcaacaaaaa 180
tcacatgagt ctccaaggct tctcattaag tatgcttccc agtccatctc tgggatcccc 240
tccaggttca gtggcagtgg atcagggaca gatttcactc tcagtatcca cagtgtggag 300
actgaagatt ttggaatgtt tttctgtcaa cacagtaaca gctggcctct cacgttcggt 360
gctgggacca agctggaaat caaaggtggt ggtggttctg gtggtggtgg ttctggcggc 420
ggcggctccg gtggtggtgg atcccaggtt actctgaaag agtctggccc tgggatattg 480
cagccctccc agaccctcag tctgacttgt tctttctctg ggttttcact gagcacttct 540
ggtatgggtg ttggctggat tcgtcagcca tcacggaagg gtctggagtg gctggcacac 600
atttggtggg atgatgtcaa gcgctataac ccagccctga agagccgact gactatctcc 660
aaggatacct ccagcagcca ggtgttcctc aagatcgcca gtgtggacac tgcggatact 720
gccacatact actgtgctcg aataccccac tggtacttcg atgtctgggg cgcagggacc 780
acggtcaccg tctcctca 798

<210> 14
<211> 266
<212> PRT
<213> Artificial Sequence

<220>
<223> synthetic protein

<400> 14
Met Glu Phe Gly Leu Ser Trp Leu Phe Leu Val Ala Ile Leu Lys Gly
1               5                   10                  15
Val Gln Cys Ser Arg Asp Val Val Leu Thr Gln Ser Pro Ala Thr Leu
            20                  25                  30
Ser Val Thr Pro Gly Asp Ser Val Ser Leu Ser Cys Arg Ala Ser Gln
        35                  40                  45
Ser Ile Ser Ile Asn Leu His Trp Tyr Gln Gln Lys Ser His Glu Ser
    50                  55                  60
Pro Arg Leu Leu Ile Lys Tyr Ala Ser Gln Ser Ile Ser Gly Ile Pro
65                  70                  75                  80
Ser Arg Phe Ser Gly Ser Gly Ser Gly Thr Asp Phe Thr Leu Ser Ile
                85                  90                  95
His Ser Val Glu Thr Glu Asp Phe Gly Met Phe Phe Cys Gln His Ser
            100                 105                 110
Asn Ser Trp Pro Leu Thr Phe Gly Ala Gly Thr Lys Leu Glu Ile Lys
        115                 120                 125
Gly Gly Gly Gly Ser Gly Gly Gly Gly Ser Gly Gly Gly Gly Ser Gly
    130                 135                 140
Gly Gly Gly Ser Gln Val Thr Leu Lys Glu Ser Gly Pro Gly Ile Leu
145                 150                 155                 160
Gln Pro Ser Gln Thr Leu Ser Leu Thr Cys Ser Phe Ser Gly Phe Ser
                165                 170                 175
Leu Ser Thr Ser Gly Met Gly Val Gly Trp Ile Arg Gln Pro Ser Arg
            180                 185                 190
Lys Gly Leu Glu Trp Leu Ala His Ile Trp Trp Asp Asp Val Lys Arg
        195                 200                 205
Tyr Asn Pro Ala Leu Lys Ser Arg Leu Thr Ile Ser Lys Asp Thr Ser
    210                 215                 220
Ser Ser Gln Val Phe Leu Lys Ile Ala Ser Val Asp Thr Ala Asp Thr
225                 230                 235                 240
Ala Thr Tyr Tyr Cys Ala Arg Ile Pro His Trp Tyr Phe Asp Val Trp
                245                 250                 255
Gly Ala Gly Thr Thr Val Thr Val Ser Ser
            260                 265

<210> 15
<211> 321
<212> DNA
<213> Artificial Sequence

<220>
<223>  synthetic nucleic acid

<400>  15
gatgttgtgc taactcagtc tccagccacc ctgtctgtga ctccaggaga tagcgtcagt      60
ctttcctgca gggccagcca aagtattagc atcaacctac actggtatca acaaaaatca     120
catgagtctc caaggcttct cattaagtat gcttcccagt ccatctctgg gatcccctcc     180
aggttcagtg gcagtggatc agggacagat ttcactctca gtatccacag tgtggagact     240
gaagattttg gaatgttttt ctgtcaacac agtaacagct ggcctctcac gttcggtgct     300
gggaccaagc tggaaatcaa a                                               321

<210>  16
<211>  107
<212>  PRT
<213>  Artificial Sequence

<220>
<223>  synthetic protein

<400>  16
Asp Val Val Leu Thr Gln Ser Pro Ala Thr Leu Ser Val Thr Pro Gly
1               5                   10                  15
Asp Ser Val Ser Leu Ser Cys Arg Ala Ser Gln Ser Ile Ser Ile Asn
            20                  25                  30
Leu His Trp Tyr Gln Gln Lys Ser His Glu Ser Pro Arg Leu Leu Ile
        35                  40                  45
Lys Tyr Ala Ser Gln Ser Ile Ser Gly Ile Pro Ser Arg Phe Ser Gly
    50                  55                  60
Ser Gly Ser Gly Thr Asp Phe Thr Leu Ser Ile His Ser Val Glu Thr
65                  70                  75                  80
Glu Asp Phe Gly Met Phe Phe Cys Gln His Ser Asn Ser Trp Pro Leu
                85                  90                  95
Thr Phe Gly Ala Gly Thr Lys Leu Glu Ile Lys
            100                 105

<210>  17
<211>  11
<212>  PRT
<213>  Artificial Sequence

<220>
<223>  synthetic protein

<400>  17
Arg Ala Ser Gln Ser Ile Ser Ile Asn Leu His
1               5                   10

<210>  18
<211>  7
<212>  PRT
<213>  Artificial Sequence

<220>
<223>  synthetic protein

<400>  18
Tyr Ala Ser Gln Ser Ile Ser
1               5

<210>  19
<211>  9
<212>  PRT

<213>  Artificial Sequence

<220>
<223>  synthetic protein

<400>  19
Gln His Ser Asn Ser Trp Pro Leu Thr
1               5

<210>  20
<211>  354
<212>  DNA
<213>  Artificial Sequence

<220>
<223>  synthetic nucleic acid

<400>  20
caggttactc tgaaagagtc tggccctggg atattgcagc cctcccagac cctcagtctg      60
acttgttctt tctctgggtt ttcactgagc acttctggta tgggtgttgg ctggattcgt     120
cagccatcac ggaagggtct ggagtggctg gcacacattt ggtgggatga tgtcaagcgc     180
tataacccag ccctgaagag ccgactgact atctccaagg atacctccag cagccaggtg     240
ttcctcaaga tcgccagtgt ggacactgcg gatactgcca catactactg tgctcgaata     300
ccccactggt acttcgatgt ctggggcgca gggaccacgg tcaccgtctc ctca           354

<210>  21
<211>  118
<212>  PRT
<213>  Artificial Sequence

<220>
<223>  synthetic protein

<400>  21
Gln Val Thr Leu Lys Glu Ser Gly Pro Gly Ile Leu Gln Pro Ser Gln
1               5                   10                  15
Thr Leu Ser Leu Thr Cys Ser Phe Ser Gly Phe Ser Leu Ser Thr Ser
            20                  25                  30
Gly Met Gly Val Gly Trp Ile Arg Gln Pro Ser Arg Lys Gly Leu Glu
        35                  40                  45
Trp Leu Ala His Ile Trp Trp Asp Asp Val Lys Arg Tyr Asn Pro Ala
    50                  55                  60
Leu Lys Ser Arg Leu Thr Ile Ser Lys Asp Thr Ser Ser Ser Gln Val
65                  70                  75                  80
Phe Leu Lys Ile Ala Ser Val Asp Thr Ala Asp Thr Ala Thr Tyr Tyr
                85                  90                  95
Cys Ala Arg Ile Pro His Trp Tyr Phe Asp Val Trp Gly Ala Gly Thr
            100                 105                 110
Thr Val Thr Val Ser Ser
        115

<210>  22
<211>  12
<212>  PRT
<213>  Artificial Sequence

<220>
<223>  synthetic protein

<400>  22
Gly Phe Ser Leu Ser Thr Ser Gly Met Gly Val Gly
1               5                   10

<210>  23
<211>  16
<212>  PRT
<213>  Artificial Sequence

<220>
<223>  synthetic protein

<400>  23
His Ile Trp Trp Asp Asp Val Lys Arg Tyr Asn Pro Ala Leu Lys Ser
1               5                   10                  15

<210>  24
<211>  10
<212>  PRT
<213>  Artificial Sequence

<220>
<223>  synthetic protein

<400>  24
Ala Arg Ile Pro His Trp Tyr Phe Asp Val
1               5                   10

<210>  25
<211>  804
<212>  DNA
<213>  Artificial Sequence

<220>
<223>  synthetic nucleic acid

<400>  25
atggagtttg ggctgagctg gctttttctt gtggctattt taaaaggtgt ccagtgctct      60
agagacattg tgatgaccca gtctcaaaaa ttcatgtcca catcagtagg agacagggtc     120
agcatcacct gcaaggccag tcagaatgtt cgtactgctg tagcctggta tcaacagaaa     180
ccaggccagt ctcctaaagc actgatttac ttggcatcca accggctcac tggagtccct     240
gatcgcttca caggcagtgg atctgggaca gatttcactc tcaccattag caatgtccaa     300
tctgaagacc tggcagatta tttctgtctg caacattgga attatccatt cacgttcggc     360
tcggggacaa agttggaaat aaaaggtggt ggtggttctg gtggtggtgg ttctggcggc     420
ggcggctccg gtggtggtgg atcccaggtc cagctgaagc agtctggagc tgagctggtg     480
aggcctgggg cttcagtgaa tctgtcctgc aagacttctg gatacatctt caccagctac     540
tggattcact gggtaaaaca gaggtctgga cagggccttg agtggattgc aaggatttat     600
cctggagttg atagtattta ttacaatgag aagttcaagg gcaaggccac actgactgca     660
gacaaatcct ccagcactgc ctacatgcag ctcagcagcc tgaaatctga ggactctgct     720
gtctatttct gtgcaactat ctcctatgat tacgactatt ctatggacta ctggggtcaa     780
ggaacctcag tcaccgtctc ctca                                            804

<210>  26
<211>  268
<212>  PRT
<213>  Artificial Sequence

<220>
<223>  synthetic protein

<400>  26
Met Glu Phe Gly Leu Ser Trp Leu Phe Leu Val Ala Ile Leu Lys Gly
1               5                   10                  15
Val Gln Cys Ser Arg Asp Ile Val Met Thr Gln Ser Gln Lys Phe Met
            20                  25                  30
Ser Thr Ser Val Gly Asp Arg Val Ser Ile Thr Cys Lys Ala Ser Gln
        35                  40                  45

Asn Val Arg Thr Ala Val Ala Trp Tyr Gln Gln Lys Pro Gly Gln Ser
    50                  55                  60
Pro Lys Ala Leu Ile Tyr Leu Pro Ser Asn Arg Leu Thr Gly Val Pro
65                  70                  75                  80
Asp Arg Phe Thr Gly Ser Gly Ser Gly Thr Asp Phe Thr Leu Thr Ile
                85                  90                  95
Ser Asn Val Gln Ser Glu Asp Leu Ala Asp Tyr Phe Cys Leu Gln His
            100                 105                 110
Trp Asn Tyr Pro Phe Thr Phe Gly Ser Gly Thr Lys Leu Glu Ile Lys
        115                 120                 125
Gly Gly Gly Gly Ser Gly Gly Gly Gly Ser Gly Gly Gly Gly Ser Gly
    130                 135                 140
Gly Gly Gly Ser Gln Val Gln Leu Lys Gln Ser Gly Ala Glu Leu Val
145                 150                 155                 160
Arg Pro Gly Ala Ser Val Asn Leu Ser Cys Lys Thr Ser Gly Tyr Ile
                165                 170                 175
Phe Thr Ser Tyr Trp Ile His Trp Val Lys Gln Arg Ser Gly Gln Gly
            180                 185                 190
Leu Glu Trp Ile Ala Arg Ile Tyr Pro Gly Val Asp Ser Ile Tyr Tyr
        195                 200                 205
Asn Glu Lys Phe Lys Gly Lys Ala Thr Leu Thr Ala Asp Lys Ser Ser
    210                 215                 220
Ser Thr Ala Tyr Met Gln Leu Ser Ser Leu Lys Ser Glu Asp Ser Ala
225                 230                 235                 240
Val Tyr Phe Cys Ala Thr Ile Ser Tyr Asp Tyr Asp Tyr Ser Met Asp
                245                 250                 255
Tyr Trp Gly Gln Gly Thr Ser Val Thr Val Ser Ser
            260                 265

<210>  27
<211>  321
<212>  DNA
<213>  Artificial Sequence

<220>
<223>  synthetic nucleic acid

<400>  27
gacattgtga tgacccagtc tcaaaaattc atgtccacat cagtaggaga cagggtcagc      60
atcacctgca aggccagtca gaatgttcgt actgctgtag cctggtatca acagaaacca     120
ggccagtctc ctaaagcact gatttacttg ccatccaacc ggctcactgg agtccctgat     180
cgcttcacag gcagtggatc tgggacagat ttcactctca ccattagcaa tgtccaatct     240
gaagacctgg cagattattt ctgtctgcaa cattggaatt atccattcac gttcggctcg     300
gggacaaagt tggaaataaa a                                               321

<210>  28
<211>  107
<212>  PRT
<213>  Artificial Sequence

<220>
<223>  synthetic protein

<400>  28
Asp Ile Val Met Thr Gln Ser Gln Lys Phe Met Ser Thr Ser Val Gly
1               5                   10                  15
Asp Arg Val Ser Ile Thr Cys Lys Ala Ser Gln Asn Val Arg Thr Ala
            20                  25                  30
Val Ala Trp Tyr Gln Gln Lys Pro Gly Gln Ser Pro Lys Ala Leu Ile
        35                  40                  45
Tyr Leu Pro Ser Asn Arg Leu Thr Gly Val Pro Asp Arg Phe Thr Gly
    50                  55                  60
Ser Gly Ser Gly Thr Asp Phe Thr Leu Thr Ile Ser Asn Val Gln Ser

65                  70                  75                  80
Glu Asp Leu Ala Asp Tyr Phe Cys Leu Gln His Trp Asn Tyr Pro Phe
                85                  90                  95
Thr Phe Gly Ser Gly Thr Lys Leu Glu Ile Lys
            100                 105

<210>  29
<211>  11
<212>  PRT
<213>  Artificial Sequence

<220>
<223>  synthetic protein

<400>  29
Lys Ala Ser Gln Asn Val Arg Thr Ala Val Ala
1               5                   10

<210>  30
<211>  7
<212>  PRT
<213>  Artificial Sequence

<220>
<223>  synthetic protein

<400>  30
Leu Pro Ser Asn Arg Leu Thr
1               5

<210>  31
<211>  9
<212>  PRT
<213>  Artificial Sequence

<220>
<223>  synthetic protein

<400>  31
Leu Gln His Trp Asn Tyr Pro Phe Thr
1               5

<210>  32
<211>  360
<212>  DNA
<213>  Artificial Sequence

<220>
<223>  synthetic nucleic acid

<400>  32
caggtccagc tgaagcagtc tggagctgag ctggtgaggc ctggggcttc agtgaatctg      60
tcctgcaaga cttctggata catcttcacc agctactgga ttcactgggt aaaacagagg     120
tctggacagg gccttgagtg gattgcaagg atttatcctg gagttgatag tatttattac     180
aatgagaagt tcaagggcaa ggccacactg actgcagaca aatcctccag cactgcctac     240
atgcagctca gcagcctgaa atctgaggac tctgctgtct atttctgtgc aactatctcc     300
tatgattacg actattctat ggactactgg ggtcaaggaa cctcagtcac cgtctcctca     360

<210>  33
<211>  120
<212>  PRT
<213>  Artificial Sequence

<220>
<223>  synthetic protein

<400>  33
Gln Val Gln Leu Lys Gln Ser Gly Ala Glu Leu Val Arg Pro Gly Ala
1               5                   10                  15
Ser Val Asn Leu Ser Cys Lys Thr Ser Gly Tyr Ile Phe Thr Ser Tyr
            20                  25                  30
Trp Ile His Trp Val Lys Gln Arg Ser Gly Gln Gly Leu Glu Trp Ile
        35                  40                  45
Ala Arg Ile Tyr Pro Gly Val Asp Ser Ile Tyr Tyr Asn Glu Lys Phe
    50                  55                  60
Lys Gly Lys Ala Thr Leu Thr Ala Asp Lys Ser Ser Ser Thr Ala Tyr
65                  70                  75                  80
Met Gln Leu Ser Ser Leu Lys Ser Glu Asp Ser Ala Val Tyr Phe Cys
                85                  90                  95
Ala Thr Ile Ser Tyr Asp Tyr Asp Tyr Ser Met Asp Tyr Trp Gly Gln
            100                 105                 110
Gly Thr Ser Val Thr Val Ser Ser
        115                 120

<210>  34
<211>  10
<212>  PRT
<213>  Artificial Sequence

<220>
<223>  synthetic protein

<400>  34
Gly Tyr Ile Phe Thr Ser Tyr Trp Ile His
1               5                   10

<210>  35
<211>  17
<212>  PRT
<213>  Artificial Sequence

<220>
<223>  synthetic protein

<400>  35
Arg Ile Tyr Pro Gly Val Asp Ser Ile Tyr Tyr Asn Glu Lys Phe Lys
1               5                   10                  15
Gly

<210>  36
<211>  13
<212>  PRT
<213>  Artificial Sequence

<220>
<223>  synthetic protein

<400>  36
Ala Thr Ile Ser Tyr Asp Tyr Asp Tyr Ser Met Asp Tyr
1               5                   10

<210>  37
<211>  807
<212>  DNA
<213>  Artificial Sequence

<220>
<223>  synthetic nucleic acid

<400>  37
atggagtttg ggctgagctg gcttttcttt gtggctattt taaaaggtgt ccagtgctct      60
agacaaattg ttctcaccca gtctccagca atcatgtctg catctccagg ggagaaggtc     120
accatgacct gcagtgccag ctcaagtgta agttacatgc actggtacca gcagaagtca     180
ggcacctccc ccaaaagatg gatttatgac acatccaaac tggcttctgg agtccctgct     240
cgcttcagtg gcagtgggtc tgggacctct tactctctca caatcagcag catggaggct     300
gaagatgctg ccacttatta ctgcctgcag tggagtagta acccgctcac gttcggtgct     360
gggaccaagc tggaaatcaa aggtggtggt ggttctggtg gtggtggttc tggcggcggc     420
ggctccggtg gtggtggatc cgaggtccag ctgcagcagt ctggacctga gctggtgaag     480
cctggggctt cagtgaagat atcctgcaag acttctggat acacattcac tgaatacacc     540
atgcactggg tgaagcagag ccatggaaag agccttgagt ggattggagg tattaatcct     600
aacaatggtg gtactaccta caaccagaaa ttcaagggca aggccacatt gactgtagac     660
aagtcctcca gcacagccta catggagctc cgcagcctga catctgagga ttctgcagtc     720
tattactgtg cgagacggat tactacgatg atacctacgg ggggctttga ctactggggc     780
caaggcacca ctctcacagt ctcctca                                         807

<210>  38
<211>  269
<212>  PRT
<213>  Artificial Sequence

<220>
<223>  synthetic protein

<400>  38
Met Glu Phe Gly Leu Ser Trp Leu Phe Leu Val Ala Ile Leu Lys Gly
1               5                   10                  15
Val Gln Cys Ser Arg Gln Ile Val Leu Thr Gln Ser Pro Ala Ile Met
            20                  25                  30
Ser Ala Ser Pro Gly Glu Lys Val Thr Met Thr Cys Ser Ala Ser Ser
        35                  40                  45
Ser Val Ser Tyr Met His Trp Tyr Gln Gln Lys Ser Gly Thr Ser Pro
    50                  55                  60
Lys Arg Trp Ile Tyr Asp Thr Ser Lys Leu Ala Ser Gly Val Pro Ala
65                  70                  75                  80
Arg Phe Ser Gly Ser Gly Ser Gly Thr Ser Tyr Ser Leu Thr Ile Ser
                85                  90                  95
Ser Met Glu Ala Glu Asp Ala Ala Thr Tyr Tyr Cys Leu Gln Trp Ser
            100                 105                 110
Ser Asn Pro Leu Thr Phe Gly Ala Gly Thr Lys Leu Glu Ile Lys Gly
        115                 120                 125
Gly Gly Gly Ser Gly Gly Gly Gly Ser Gly Gly Gly Gly Ser Gly Gly
    130                 135                 140
Gly Gly Ser Glu Val Gln Leu Gln Gln Ser Gly Pro Glu Leu Val Lys
145                 150                 155                 160
Pro Gly Ala Ser Val Lys Ile Ser Cys Lys Thr Ser Gly Tyr Thr Phe
                165                 170                 175
Thr Glu Tyr Thr Met His Trp Val Lys Gln Ser His Gly Lys Ser Pro
            180                 185                 190
Glu Trp Ile Gly Gly Ile Asn Pro Asn Asn Gly Gly Thr Thr Tyr Asn
        195                 200                 205
Gln Lys Phe Lys Gly Lys Ala Thr Leu Thr Val Asp Lys Ser Ser Ser
    210                 215                 220
Thr Ala Tyr Met Glu Leu Arg Ser Leu Thr Ser Glu Asp Ser Ala Val
225                 230                 235                 240
Tyr Tyr Cys Ala Arg Arg Ile Thr Thr Met Ile Pro Thr Gly Gly Phe
                245                 250                 255
Asp Tyr Trp Gly Gln Gly Thr Thr Leu Thr Val Ser Ser
            260                 265

<210>  39
<211>  318
<212>  DNA
<213>  Artificial Sequence

<220>
<223>  synthetic nucleic acid

<400>  39
caaattgttc tcacccagtc tccagcaatc atgtctgcat ctccagggga gaaggtcacc      60
atgacctgca gtgccagctc aagtgtaagt tacatgcact ggtaccagca gaagtcaggc     120
acctccccca aaagatggat ttatgacaca tccaaactgg cttctggagt ccctgctcgc     180
ttcagtggca gtgggtctgg gacctcttac tctctcacaa tcagcagcat ggaggctgaa     240
gatgctgcca cttattactg cctgcagtgg agtagtaacc cgctcacgtt cggtgctggg     300
accaagctgg aaatcaaa                                                   318

<210>  40
<211>  106
<212>  PRT
<213>  Artificial Sequence

<220>
<223>  synthetic protein

<400>  40
Gln Ile Val Leu Thr Gln Ser Pro Ala Ile Met Ser Ala Ser Pro Gly
1               5                   10                  15
Glu Lys Val Thr Met Thr Cys Ser Ala Ser Ser Ser Val Ser Tyr Met
            20                  25                  30
His Trp Tyr Gln Gln Lys Ser Gly Thr Ser Pro Lys Arg Trp Ile Tyr
        35                  40                  45
Asp Thr Ser Lys Leu Ala Ser Gly Val Pro Ala Arg Phe Ser Gly Ser
    50                  55                  60
Gly Ser Gly Thr Ser Tyr Ser Leu Thr Ile Ser Ser Met Glu Ala Glu
65                  70                  75                  80
Asp Ala Ala Thr Tyr Tyr Cys Leu Gln Trp Ser Ser Asn Pro Leu Thr
                85                  90                  95
Phe Gly Ala Gly Thr Lys Leu Glu Ile Lys
            100                 105

<210>  41
<211>  10
<212>  PRT
<213>  Artificial Sequence

<220>
<223>  synthetic protein

<400>  41
Ser Ala Ser Ser Ser Val Ser Tyr Met His
1               5                   10

<210>  42
<211>  7
<212>  PRT
<213>  Artificial Sequence

<220>
<223>  synthetic protein

<400>  42
Asp Thr Ser Lys Leu Ala Ser

1               5

<210>  43
<211>  9
<212>  PRT
<213>  Artificial Sequence

<220>
<223>  synthetic protein

<400>  43
Leu Gln Trp Ser Ser Asn Pro Leu Thr
1               5

<210>  44
<211>  366
<212>  DNA
<213>  Artificial Sequence

<220>
<223>  synthetic nucleic acid

<400>  44
gaggtccagc tgcagcagtc tggacctgag ctggtgaagc ctggggcttc agtgaagata      60
tcctgcaaga cttctggata cacattcact gaatacacca tgcactgggt gaagcagagc     120
catggaaaga gccctgagtg gattggaggt attaatccta acaatggtgg tactacctac     180
aaccagaaat tcaagggcaa ggccacattg actgtagaca agtcctccag cacagcctac     240
atggagctcc gcagcctgac atctgaggat tctgcagtct attactgtgc gagacggatt     300
actacgatga tacctacggg gggctttgac tactggggcc aaggcaccac tctcacagtc     360
tcctca                                                                366

<210>  45
<211>  122
<212>  PRT
<213>  Artificial Sequence

<220>
<223>  synthetic protein

<400>  45
Glu Val Gln Leu Gln Gln Ser Gly Pro Glu Leu Val Lys Pro Gly Ala
1               5                   10                  15
Ser Val Lys Ile Ser Cys Lys Thr Ser Gly Tyr Thr Phe Thr Glu Tyr
            20                  25                  30
Thr Met His Trp Val Lys Gln Ser His Gly Lys Ser Pro Glu Trp Ile
        35                  40                  45
Gly Gly Ile Asn Pro Asn Asn Gly Gly Thr Thr Tyr Asn Gln Lys Phe
    50                  55                  60
Lys Gly Lys Ala Thr Leu Thr Val Asp Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Ala Arg Arg Ile Thr Thr Met Ile Pro Thr Gly Gly Phe Asp Tyr Trp
            100                 105                 110
Gly Gln Gly Thr Thr Leu Thr Val Ser Ser
        115                 120

<210>  46
<211>  10
<212>  PRT
<213>  Artificial Sequence

<220>

<223>  synthetic protein

<400>  46
Gly Tyr Thr Phe Thr Glu Tyr Thr Met His
1               5                   10

<210>  47
<211>  17
<212>  PRT
<213>  Artificial Sequence

<220>
<223>  synthetic protein

<400>  47
Gly Ile Asn Pro Asn Asn Gly Gly Thr Thr Tyr Asn Gln Lys Phe Lys
1               5                   10                  15
Gly

<210>  48
<211>  15
<212>  PRT
<213>  Artificial Sequence

<220>
<223>  synthetic protein

<400>  48
Ala Arg Arg Ile Thr Thr Met Ile Pro Thr Gly Gly Phe Asp Tyr
1               5                   10                  15

<210>  49
<211>  789
<212>  DNA
<213>  Artificial Sequence

<220>
<223>  synthetic nucleic acid

<400>  49
atggagtttg ggctgagctg gctttttctt gtggctattt taaaaggtgt ccagtgctct      60
agagaaattt tgctcaccca gtctccagca atcatagctg tatctcctgg ggagaaggtc     120
accataacct gcagtgccag ctcaagtgta aattacatga actggtacca acagaaacca     180
ggatcctccc ccaaaatatg gatttttggt atgtccaacc tggcttctgg agttcctgct     240
cgcttcagtg gcagtgggtc tgggacatct ttctctttca caatcaacag catggaggct     300
gaagatgttg ccacttatta ctgtcaccaa aggagtagtt acccatacac gttcggaggg     360
gggaccaagc tggaaatcaa aggtggtggt ggttctggtg gtggtggttc tggcggcggc     420
ggctccggtg gtggtggatc ccagatccag ttggtgcagt ctggacctga attgaagaag     480
cctggagaga cagtcaggat ctcctgcaag gcttctggat ataccttcac aaactatgga     540
atgaactggg tgaagcaggc tccaggaaag ggtttgaagt ggatgggctg gattaatacc     600
tacactggag agccgacata ttctgatgtc ttcaggggac ggtttgcctt ctctttggaa     660
acctctgcca acactgccta tttagagatc agaaaactca aaaatgagga catggctacg     720
tatttctgtg tgagaaatga atacgacgca ccttactggg gccaagggac tctggtcact     780
gtctctgca                                                             789

<210>  50
<211>  263
<212>  PRT
<213>  Artificial Sequence

<220>
<223>  synthetic protein

<400>  50
Met Glu Phe Gly Leu Ser Trp Leu Phe Leu Val Ala Ile Leu Lys Gly
1               5                   10                  15
Val Gln Cys Ser Arg Glu Ile Leu Leu Thr Gln Ser Pro Ala Ile Ile
            20                  25                  30
Ala Val Ser Pro Gly Glu Lys Val Thr Ile Thr Cys Ser Ala Ser Ser
        35                  40                  45
Ser Val Asn Tyr Met Asn Trp Tyr Gln Gln Lys Pro Gly Ser Ser Pro
    50                  55                  60
Lys Ile Trp Ile Phe Gly Met Ser Asn Leu Ala Ser Gly Val Pro Ala
65                  70                  75                  80
Arg Phe Ser Gly Ser Gly Ser Gly Thr Ser Phe Ser Phe Thr Ile Asn
                85                  90                  95
Ser Met Glu Ala Glu Asp Val Ala Thr Tyr Tyr Cys His Gln Arg Ser
            100                 105                 110
Ser Tyr Pro Tyr Thr Phe Gly Gly Gly Thr Lys Leu Glu Ile Lys Gly
        115                 120                 125
Gly Gly Gly Ser Gly Gly Gly Gly Ser Gly Gly Gly Gly Ser Gly Gly
    130                 135                 140
Gly Gly Ser Gln Ile Gln Leu Val Gln Ser Gly Pro Glu Leu Lys Lys
145                 150                 155                 160
Pro Gly Glu Thr Val Arg Ile Ser Cys Lys Ala Ser Gly Tyr Thr Phe
                165                 170                 175
Thr Asn Tyr Gly Met Asn Trp Val Lys Gln Ala Pro Gly Lys Gly Leu
            180                 185                 190
Lys Trp Met Gly Trp Ile Asn Thr Tyr Thr Gly Glu Pro Thr Tyr Ser
        195                 200                 205
Asp Val Phe Arg Gly Arg Phe Ala Phe Ser Leu Glu Thr Ser Ala Asn
    210                 215                 220
Thr Ala Tyr Leu Glu Ile Arg Lys Leu Lys Asn Glu Asp Met Ala Thr
225                 230                 235                 240
Tyr Phe Cys Val Arg Asn Glu Tyr Asp Ala Pro Tyr Trp Gly Gln Gly
                245                 250                 255
Thr Leu Val Thr Val Ser Ala
            260

<210>  51
<211>  318
<212>  DNA
<213>  Artificial Sequence

<220>
<223>  synthetic nucleic acid

<400>  51
gaaattttgc tcacccagtc tccagcaatc atagctgtat ctcctgggga gaaggtcacc      60
ataacctgca gtgccagctc aagtgtaaat tacatgaact ggtaccaaca gaaaccagga     120
tcctccccca aaatatggat ttttggtatg tccaacctgg cttctggagt tcctgctcgc     180
ttcagtggca gtgggtctgg gacatctttc tctttcacaa tcaacagcat ggaggctgaa     240
gatgttgcca cttattactg tcaccaaagg agtagttacc catacacgtt cggagggggg     300
accaagctgg aaatcaaa                                                   318

<210>  52
<211>  106
<212>  PRT
<213>  Artificial Sequence

<220>
<223>  synthetic protein

<400>  52
Glu Ile Leu Leu Thr Gln Ser Pro Ala Ile Ile Ala Val Ser Pro Gly

1               5                   10                  15
Glu Lys Val Thr Ile Thr Cys Ser Ala Ser Ser Ser Val Asn Tyr Met
            20                  25                  30
Asn Trp Tyr Gln Gln Lys Pro Gly Ser Ser Pro Lys Ile Trp Ile Phe
        35                  40                  45
Gly Met Ser Asn Leu Ala Ser Gly Val Pro Ala Arg Phe Ser Gly Ser
    50                  55                  60
Gly Ser Gly Thr Ser Phe Ser Phe Thr Ile Asn Ser Met Glu Ala Glu
65                  70                  75                  80
Asp Val Ala Thr Tyr Tyr Cys His Gln Arg Ser Ser Tyr Pro Tyr Thr
                85                  90                  95
Phe Gly Gly Gly Thr Lys Leu Glu Ile Lys
            100                 105

<210>  53
<211>  10
<212>  PRT
<213>  Artificial Sequence

<220>
<223>  synthetic protein

<400>  53
Ser Ala Ser Ser Ser Val Asn Tyr Met Asn
1               5                   10

<210>  54
<211>  7
<212>  PRT
<213>  Artificial Sequence

<220>
<223>  synthetic protein

<400>  54
Gly Met Ser Asn Leu Ala Ser
1               5

<210>  55
<211>  9
<212>  PRT
<213>  Artificial Sequence

<220>
<223>  synthetic protein

<400>  55
His Gln Arg Ser Ser Tyr Pro Tyr Thr
1               5

<210>  56
<211>  348
<212>  DNA
<213>  Artificial Sequence

<220>
<223>  synthetic nucleic acid

<400>  56
cagatccagt tggtgcagtc tggacctgaa ttgaagaagc ctggagagac agtcaggatc      60
tcctgcaagg cttctggata taccttcaca aactatggaa tgaactgggt gaagcaggct     120
ccaggaaagg gtttgaagtg gatgggctgg attaatacct acactggaga gccgacatat     180
tctgatgtct tcaggggacg gtttgccttc tctttggaaa cctctgccaa cactgcctat     240

ttagagatca gaaaactcaa aaatgaggac atggctacgt atttctgtgt gagaaatgaa 300
tacgacgcac cttactgggg ccaagggact ctggtcactg tctctgca 348

<210> 57
<211> 116
<212> PRT
<213> Artificial Sequence

<220>
<223> synthetic protein

<400> 57
Gln Ile Gln Leu Val Gln Ser Gly Pro Glu Leu Lys Lys Pro Gly Glu
1               5                   10                  15
Thr Val Arg Ile Ser Cys Lys Ala Ser Gly Tyr Thr Phe Thr Asn Tyr
            20                  25                  30
Gly Met Asn Trp Val Lys Gln Ala Pro Gly Lys Gly Leu Lys Trp Met
        35                  40                  45
Gly Trp Ile Asn Thr Tyr Thr Gly Glu Pro Thr Tyr Ser Asp Val Phe
    50                  55                  60
Arg Gly Arg Phe Ala Phe Ser Leu Glu Thr Ser Ala Asn Thr Ala Tyr
65                  70                  75                  80
Leu Glu Ile Arg Lys Leu Lys Asn Glu Asp Met Ala Thr Tyr Phe Cys
                85                  90                  95
Val Arg Asn Glu Tyr Asp Ala Pro Tyr Trp Gly Gln Gly Thr Leu Val
            100                 105                 110
Thr Val Ser Ala
        115

<210> 58
<211> 10
<212> PRT
<213> Artificial Sequence

<220>
<223> synthetic protein

<400> 58
Gly Tyr Thr Phe Thr Asn Tyr Gly Met Asn
1               5                   10

<210> 59
<211> 17
<212> PRT
<213> Artificial Sequence

<220>
<223> synthetic protein

<400> 59
Trp Ile Asn Thr Tyr Thr Gly Glu Pro Thr Tyr Ser Asp Val Phe Arg
1               5                   10                  15
Gly

<210> 60
<211> 9
<212> PRT
<213> Artificial Sequence

<220>
<223> synthetic protein

<400>  60
Val Arg Asn Glu Tyr Asp Ala Pro Tyr
1               5

<210>  61
<211>  816
<212>  DNA
<213>  Artificial Sequence

<220>
<223>  synthetic nucleic acid

<400>  61
atggagtttg ggctgagctg gctttttctt gtggctattt taaaaggtgt ccagtgctct      60
agaaacattg tgttggccca atctccagct tctttggctg tgtctctagg gcagagggcc     120
accatatcct gcagagccag cgaaagtgtt gataattatg gctatagttt tatgcactgg     180
taccagcaga aaccaggaca gtcacccaaa ctcctcatct atcttgcatc caacctagaa     240
tctggggtcc ctgccaggtt cagtggcagt gggtctaggt cagacttcac cctcaccatt     300
gatcctgtgg aggctgatga tgctgcaacc tattactgtc agcaaaatag tgaagatccg     360
tggacgttcg gtggaggcac caggctggaa atcaaaggtg gtggtggttc tggtggtggt     420
ggttctggcg gcggcggctc cggtggtggt ggatcccagg ttcaactcca gcagtctggg     480
gctgtgctgg tgaagcctgg ggcctcagtg aagatgtcct gcaaggcttc tggctacaca     540
tttaccagtt acaatttgca ctggataaag cagacacctg gacagggcct ggaatggatt     600
ggagttattt attcaggaaa tagtgatact tcaaccaatc agaagttcaa aggcaaggcc     660
acattgactg aagacaaatc cgccagtaca gtctacatat atctcagcag cctgacatct     720
gaagactctg cggtctatta ctgtgcaagg ggggaatatg gtaaccacgg gggatttgct     780
tactggggcc aagggactct ggtcactgtc tctgca                               816

<210>  62
<211>  272
<212>  PRT
<213>  Artificial Sequence

<220>
<223>  synthetic protein

<400>  62
Met Glu Phe Gly Leu Ser Trp Leu Phe Leu Val Ala Ile Leu Lys Gly
1               5                   10                  15
Val Gln Cys Ser Arg Asn Ile Val Leu Ala Gln Ser Pro Ala Ser Leu
            20                  25                  30
Ala Val Ser Leu Gly Gln Arg Ala Thr Ile Ser Cys Arg Ala Ser Glu
        35                  40                  45
Ser Val Asp Asn Tyr Gly Tyr Ser Phe Met His Trp Tyr Gln Gln Lys
    50                  55                  60
Pro Gly Gln Ser Pro Lys Leu Leu Ile Tyr Leu Ala Ser Asn Leu Glu
65                  70                  75                  80
Ser Gly Val Pro Ala Arg Phe Ser Gly Ser Gly Ser Arg Ser Asp Phe
                85                  90                  95
Thr Leu Thr Ile Asp Pro Val Glu Ala Asp Asp Ala Ala Thr Tyr Tyr
            100                 105                 110
Cys Gln Gln Asn Ser Glu Asp Pro Trp Thr Phe Gly Gly Gly Thr Arg
        115                 120                 125
Leu Glu Ile Lys Gly Gly Gly Gly Ser Gly Gly Gly Gly Ser Gly Gly
    130                 135                 140
Gly Gly Ser Gly Gly Gly Gly Ser Gln Val Gln Leu Gln Gln Ser Gly
145                 150                 155                 160
Ala Val Leu Val Lys Pro Gly Ala Ser Val Lys Met Ser Cys Lys Ala
                165                 170                 175
Ser Gly Tyr Thr Phe Thr Ser Tyr Asn Leu His Trp Ile Lys Gln Thr
            180                 185                 190
Pro Gly Gln Gly Leu Glu Trp Ile Gly Val Ile Tyr Ser Gly Asn Ser
        195                 200                 205

Asp Thr Ser Thr Asn Gln Lys Phe Lys Gly Lys Ala Thr Leu Thr Glu
    210                 215                 220
Asp Lys Ser Ala Ser Thr Val Tyr Ile Tyr Leu Ser Ser Leu Thr Ser
225                 230                 235                 240
Glu Asp Ser Ala Val Tyr Tyr Cys Ala Arg Gly Glu Tyr Gly Asn His
                245                 250                 255
Gly Gly Phe Ala Tyr Trp Gly Gln Gly Thr Leu Val Thr Val Ser Ala
            260                 265                 270

<210>  63
<211>  333
<212>  DNA
<213>  Artificial Sequence

<220>
<223>  synthetic nucleic acid

<400>  63
aacattgtgt tggcccaatc tccagcttct ttggctgtgt ctctagggca gagggccacc      60
atatcctgca gagccagcga aagtgttgat aattatggct atagttttat gcactggtac     120
cagcagaaac caggacagtc acccaaactc ctcatctatc ttgcatccaa cctagaatct     180
ggggtccctg ccaggttcag tggcagtggg tctaggtcag acttcaccct caccattgat     240
cctgtggagg ctgatgatgc tgcaacctat tactgtcagc aaaatagtga agatccgtgg     300
acgttcggtg gaggcaccag gctggaaatc aaa                                  333

<210>  64
<211>  111
<212>  PRT
<213>  Artificial Sequence

<220>
<223>  synthetic protein

<400>  64
Asn Ile Val Leu Ala Gln Ser Pro Ala Ser Leu Ala Val Ser Leu Gly
1               5                   10                  15
Gln Arg Ala Thr Ile Ser Cys Arg Ala Ser Glu Ser Val Asp Asn Tyr
            20                  25                  30
Gly Tyr Ser Phe Met His Trp Tyr Gln Gln Lys Pro Gly Gln Ser Pro
        35                  40                  45
Lys Leu Leu Ile Tyr Leu Ala Ser Asn Leu Glu Ser Gly Val Pro Ala
    50                  55                  60
Arg Phe Ser Gly Ser Gly Ser Arg Ser Asp Phe Thr Leu Thr Ile Asp
65                  70                  75                  80
Pro Val Glu Ala Asp Asp Ala Ala Thr Tyr Tyr Cys Gln Gln Asn Ser
                85                  90                  95
Glu Asp Pro Trp Thr Phe Gly Gly Gly Thr Arg Leu Glu Ile Lys
            100                 105                 110

<210>  65
<211>  15
<212>  PRT
<213>  Artificial Sequence

<220>
<223>  synthetic protein

<400>  65
Arg Ala Ser Glu Ser Val Asp Asn Tyr Gly Tyr Ser Phe Met His
1               5                   10                  15

<210>  66
<211>  7

<212>  PRT
<213>  Artificial Sequence

<220>
<223>  synthetic protein

<400>  66
Leu Ala Ser Asn Leu Glu Ser
1               5

<210>  67
<211>  9
<212>  PRT
<213>  Artificial Sequence

<220>
<223>  synthetic protein

<400>  67
Gln Gln Asn Ser Glu Asp Pro Trp Thr
1               5

<210>  68
<211>  360
<212>  DNA
<213>  Artificial Sequence

<220>
<223>  synthetic nucleic acid

<400>  68
caggttcaac tccagcagtc tggggctgtg ctggtgaagc ctggggcctc agtgaagatg        60
tcctgcaagg cttctggcta cacatttacc agttacaatt tgcactggat aaagcagaca       120
cctggacagg gcctggaatg gattggagtt atttattcag gaaatagtga tacttcaacc       180
aatcagaagt tcaaaggcaa ggccacattg actgaagaca aatccgccag tacagtctac       240
atatatctca gcagcctgac atctgaagac tctgcggtct attactgtgc aaggggggaa      300
tatggtaacc acgggggatt tgcttactgg ggccaaggga ctctggtcac tgtctctgca      360

<210>  69
<211>  120
<212>  PRT
<213>  Artificial Sequence

<220>
<223>  synthetic protein

<400>  69
Gln Val Gln Leu Gln Gln Ser Gly Ala Val Leu Val Lys Pro Gly Ala
1               5                   10                  15
Ser Val Lys Met Ser Cys Lys Ala Ser Gly Tyr Thr Phe Thr Ser Tyr
            20                  25                  30
Asn Leu His Trp Ile Lys Gln Thr Pro Gly Gln Gly Leu Glu Trp Ile
        35                  40                  45
Gly Val Ile Tyr Ser Gly Asn Ser Asp Thr Ser Thr Asn Gln Lys Phe
    50                  55                  60
Lys Gly Lys Ala Thr Leu Thr Glu Asp Lys Ser Ala Ser Thr Val Tyr
65                  70                  75                  80
Ile Tyr Leu Ser Ser Leu Thr Ser Glu Asp Ser Ala Val Tyr Tyr Cys
                85                  90                  95
Ala Arg Gly Glu Tyr Gly Asn His Gly Gly Phe Ala Tyr Trp Gly Gln
            100                 105                 110
Gly Thr Leu Val Thr Val Ser Ala
        115                 120

<210>  70
<211>  10
<212>  PRT
<213>  Artificial Sequence

<220>
<223>  synthetic protein

<400>  70
Gly Tyr Thr Phe Thr Ser Tyr Asn Leu His
1               5                   10

<210>  71
<211>  17
<212>  PRT
<213>  Artificial Sequence

<220>
<223>  synthetic protein

<400>  71
Val Ile Tyr Ser Gly Asn Ser Asp Thr Ser Thr Asn Gln Lys Phe Lys
1               5                   10                  15
Gly

<210>  72
<211>  13
<212>  PRT
<213>  Artificial Sequence

<220>
<223>  synthetic protein

<400>  72
Ala Arg Gly Glu Tyr Gly Asn His Gly Gly Phe Ala Tyr
1               5                   10

<210>  73
<211>  804
<212>  DNA
<213>  Artificial Sequence

<220>
<223>  synthetic nucleic acid

<400>  73
atggagtttg ggctgagctg gctttttctt gtggctattt taaaaggtgt ccagtgctct      60
agagacattg tgatgaccca gtctcaaaaa ttcatgtcca catcagtagg agacagggtc     120
agcatcacct gcaaggccag tcagaatgtt cgtactgctg tagcctggta tcaacagaaa     180
ccaggccagt ctcctaaagc actgatttac ttggcatcca accggcacac tggagtccct     240
gatcgcttca caggcagtgg atctgggaca gatttcactc tcaccattag caatgtgcaa     300
tctgaagacc tggcagatta tttctgtctg caacattgga attatccatt cacgttcggc     360
tcggggacaa agttggaaat aaaaggtggt ggtggttctg gtggtggtgg ttctggcggc     420
ggcggctccg gtggtggtgg atcccaggtc cagctgaagc agtctggagc tgagctggtg     480
aggcctgggg cttcagtgaa tctgtcctgc aagacttctg gatacatctt caccagctac     540
tggattcact gggtaaaaca gaggtctgga cagggccttg agtggattgc aaggatttat     600
cctggagttg atagtattta ttacaatgag aagttcaagg gcaaggccac actgactgca     660
gacaaatcct ccagcactgc ctacatgcag ctcagcagcc tgaaatctga ggactctgct     720
gtctatttct gtgcaactat ctcctatgat tacgactatt ctatggacta ctggggtcaa     780
ggaacctcag tcaccgtctc ctca                                            804

<210>  74
<211>  268
<212>  PRT
<213>  Artificial Sequence

<220>
<223>  synthetic protein

<400>  74
Met Glu Phe Gly Leu Ser Trp Leu Phe Leu Val Ala Ile Leu Lys Gly
1               5                   10                  15
Val Gln Cys Ser Arg Asp Ile Val Met Thr Gln Ser Gln Lys Phe Met
            20                  25                  30
Ser Thr Ser Val Gly Asp Arg Val Ser Ile Thr Cys Lys Ala Ser Gln
        35                  40                  45
Asn Val Arg Thr Ala Val Ala Trp Tyr Gln Gln Lys Pro Gly Gln Ser
    50                  55                  60
Pro Lys Ala Leu Ile Tyr Leu Pro Ser Asn Arg His Thr Gly Val Pro
65                  70                  75                  80
Asp Arg Phe Thr Gly Ser Gly Ser Gly Thr Asp Phe Thr Leu Thr Ile
                85                  90                  95
Ser Asn Val Gln Ser Glu Asp Leu Ala Asp Tyr Phe Cys Leu Gln His
            100                 105                 110
Trp Asn Tyr Pro Phe Thr Phe Gly Ser Gly Thr Lys Leu Glu Ile Lys
        115                 120                 125
Gly Gly Gly Gly Ser Gly Gly Gly Gly Ser Gly Gly Gly Gly Ser Gly
    130                 135                 140
Gly Gly Gly Ser Gln Val Gln Leu Lys Gln Ser Gly Ala Glu Leu Val
145                 150                 155                 160
Arg Pro Gly Ala Ser Val Asn Leu Ser Cys Lys Thr Ser Gly Tyr Ile
                165                 170                 175
Phe Thr Ser Tyr Trp Ile His Trp Val Lys Gln Arg Ser Gly Gln Gly
            180                 185                 190
Leu Glu Trp Ile Ala Arg Ile Tyr Pro Gly Val Asp Ser Ile Tyr Tyr
        195                 200                 205
Asn Glu Lys Phe Lys Gly Lys Ala Thr Leu Thr Ala Asp Lys Ser Ser
    210                 215                 220
Ser Thr Ala Tyr Met Gln Leu Ser Ser Leu Lys Ser Glu Asp Ser Ala
225                 230                 235                 240
Val Tyr Phe Cys Ala Thr Ile Ser Tyr Asp Tyr Asp Tyr Ser Met Asp
                245                 250                 255
Tyr Trp Gly Gln Gly Thr Ser Val Thr Val Ser Ser
            260                 265

<210>  75
<211>  321
<212>  DNA
<213>  Artificial Sequence

<220>
<223>  synthetic nucleic acid

<400>  75
gacattgtga tgacccagtc tcaaaaattc atgtccacat cagtaggaga cagggtcagc      60
atcacctgca aggccagtca gaatgttcgt actgctgtag cctggtatca acagaaacca     120
ggccagtctc ctaaagcact gatttacttg ccatccaacc ggcacactgg agtccctgat     180
cgcttcacag gcagtggatc tgggacagat ttcactctca ccattagcaa tgtgcaatct     240
gaagacctgg cagattattt ctgtctgcaa cattggaatt atccattcac gttcggctcg     300
gggacaaagt tggaaataaa a                                               321

<210>  76
<211>  107
<212>  PRT

<213>  Artificial Sequence

<220>
<223>  synthetic protein

<400>  76
Asp Ile Val Met Thr Gln Ser Gln Lys Phe Met Ser Thr Ser Val Gly
1               5                   10                  15
Asp Arg Val Ser Ile Thr Cys Lys Ala Ser Gln Asn Val Arg Thr Ala
            20                  25                  30
Val Ala Trp Tyr Gln Gln Lys Pro Gly Gln Ser Pro Lys Ala Leu Ile
        35                  40                  45
Tyr Leu Pro Ser Asn Arg His Thr Gly Val Pro Asp Arg Phe Thr Gly
    50                  55                  60
Ser Gly Ser Gly Thr Asp Phe Thr Leu Thr Ile Ser Asn Val Gln Ser
65                  70                  75                  80
Glu Asp Leu Ala Asp Tyr Phe Cys Leu Gln His Trp Asn Tyr Pro Phe
                85                  90                  95
Thr Phe Gly Ser Gly Thr Lys Leu Glu Ile Lys
            100                 105

<210>  77
<211>  11
<212>  PRT
<213>  Artificial Sequence

<220>
<223>  synthetic protein

<400>  77
Lys Ala Ser Gln Asn Val Arg Thr Ala Val Ala
1               5                   10

<210>  78
<211>  7
<212>  PRT
<213>  Artificial Sequence

<220>
<223>  synthetic protein

<400>  78
Leu Pro Ser Asn Arg His Thr
1               5

<210>  79
<211>  9
<212>  PRT
<213>  Artificial Sequence

<220>
<223>  synthetic protein

<400>  79
Leu Gln His Trp Asn Tyr Pro Phe Thr
1               5

<210>  80
<211>  359
<212>  DNA
<213>  Artificial Sequence

<220>

<223>  synthetic nucleic acid

<400>  80
aggtccagct gaagcagtct ggagctgagc tggtgaggcc tggggcttca gtgaatctgt      60
cctgcaagac ttctggatac atcttcacca gctactggat tcactgggta aaacagaggt     120
ctggacaggg ccttgagtgg attgcaagga tttatcctgg agttgatagt atttattaca     180
atgagaagtt caagggcaag gccacactga ctgcagacaa atcctccagc actgcctaca     240
tgcagctcag cagcctgaaa tctgaggact ctgctgtcta tttctgtgca actatctcct     300
atgattacga ctattctatg gactactggg gtcaaggaac ctcagtcacc gtctcctca      359

<210>  81
<211>  120
<212>  PRT
<213>  Artificial Sequence

<220>
<223>  synthetic protein

<400>  81
Gln Val Gln Leu Lys Gln Ser Gly Ala Glu Leu Val Arg Pro Gly Ala
1               5                   10                  15
Ser Val Asn Leu Ser Cys Lys Thr Ser Gly Tyr Ile Phe Thr Ser Tyr
            20                  25                  30
Trp Ile His Trp Val Lys Gln Arg Ser Gly Gln Gly Leu Glu Trp Ile
        35                  40                  45
Ala Arg Ile Tyr Pro Gly Val Asp Ser Ile Tyr Tyr Asn Glu Lys Phe
    50                  55                  60
Lys Gly Lys Ala Thr Leu Thr Ala Asp Lys Ser Ser Ser Thr Ala Tyr
65                  70                  75                  80
Met Gln Leu Ser Ser Leu Lys Ser Glu Asp Ser Ala Val Tyr Phe Cys
                85                  90                  95
Ala Thr Ile Ser Tyr Asp Tyr Asp Tyr Ser Met Asp Tyr Trp Gly Gln
            100                 105                 110
Gly Thr Ser Val Thr Val Ser Ser
        115                 120

<210>  82
<211>  10
<212>  PRT
<213>  Artificial Sequence

<220>
<223>  synthetic protein

<400>  82
Gly Tyr Ile Phe Thr Ser Tyr Trp Ile His
1               5                   10

<210>  83
<211>  17
<212>  PRT
<213>  Artificial Sequence

<220>
<223>  synthetic protein

<400>  83
Arg Ile Tyr Pro Gly Val Asp Ser Ile Tyr Tyr Asn Glu Lys Phe Lys
1               5                   10                  15
Gly


<210>  84

<211>  13
<212>  PRT
<213>  Artificial Sequence

<220>
<223>  synthetic protein

<400>  84
Ala Thr Ile Ser Tyr Asp Tyr Asp Tyr Ser Met Asp Tyr
1               5                   10

<210>  85
<211>  813
<212>  DNA
<213>  Artificial Sequence

<220>
<223>  synthetic nucleic acid

<400>  85
atggagtttg ggctgagctg gcttttcttt gtggctattt taaaaggtgt ccagtgctct      60
agagatattg tgatgacgca ggctgcattc tccaatccag tcactcttgg aacatcagct     120
tccatctcct gcaggtctag taagagtctc ctccatagta atggcatcac ttatttctat     180
tggtatctgc agaagccagg ccagtctcct cagctcctga tttatcagat gtccagcctt     240
gcctcaggag tcccagacag gttcagtagc agtgggtcag gaactgattt cacactgaga     300
atcagcagag tggaggctga ggatgtgggt gtttattact gtgctcaaaa tctagaactt     360
ccgtacacgt tcggaggggg gaccaagctg gaaatcaaag gtggtggtgg ttctggtggt     420
ggtggttctg gcggcggcgg ctccggtggt ggtggatccc agatccagtt ggtgcagtct     480
ggacctgagg tgaggaggcc tggggagaca gtcaagatct cctgcaaggc ttctggatat     540
actttcacaa actttggaat gaactgggtg aaacaggctc caggaaaggc tttacagtgg     600
atgggctgga taaacaccta cactggtgag ccaacatatg ctgatgactt caagggccgg     660
tttgccttct ctttggaatc ctctgccagc actgcccatt tgcagatcag ctacctcaaa     720
gatgaggaca cggctacata tttctgtgcg agatggtggc ttcaatatgc tttggacaac     780
tggggtcaag gaacctcagt caccgtctcc tca                                  813

<210>  86
<211>  271
<212>  PRT
<213>  Artificial Sequence

<220>
<223>  synthetic protein

<400>  86
Met Glu Phe Gly Leu Ser Trp Leu Phe Leu Val Ala Ile Leu Lys Gly
1               5                   10                  15
Val Gln Cys Ser Arg Asp Ile Val Met Thr Gln Ala Ala Phe Ser Asn
            20                  25                  30
Pro Val Thr Leu Gly Thr Ser Ala Ser Ile Ser Cys Arg Ser Ser Lys
        35                  40                  45
Ser Leu Leu His Ser Asn Gly Ile Thr Tyr Phe Tyr Trp Tyr Leu Gln
    50                  55                  60
Lys Pro Gly Gln Ser Pro Gln Leu Leu Ile Tyr Gln Met Ser Ser Leu
65                  70                  75                  80
Ala Ser Gly Val Pro Asp Arg Phe Ser Ser Ser Gly Ser Gly Thr Asp
                85                  90                  95
Phe Thr Leu Arg Ile Ser Arg Val Glu Ala Glu Asp Val Gly Val Tyr
            100                 105                 110
Tyr Cys Ala Gln Asn Leu Glu Leu Pro Tyr Thr Phe Gly Gly Gly Thr
        115                 120                 125
Lys Leu Glu Ile Lys Gly Gly Gly Gly Ser Gly Gly Gly Gly Ser Gly
    130                 135                 140
Gly Gly Gly Ser Gly Gly Gly Gly Ser Gln Ile Gln Leu Val Gln Ser

145                 150                 155                 160
Gly Pro Glu Val Arg Arg Pro Gly Glu Thr Val Lys Ile Ser Cys Lys
                165                 170                 175
Ala Ser Gly Tyr Thr Phe Thr Asn Phe Gly Met Asn Trp Val Lys Gln
            180                 185                 190
Ala Pro Gly Lys Ala Leu Gln Trp Met Gly Trp Ile Asn Thr Tyr Thr
        195                 200                 205
Gly Glu Pro Thr Tyr Ala Asp Asp Phe Lys Gly Arg Phe Ala Phe Ser
    210                 215                 220
Leu Glu Ser Ser Ala Ser Thr Ala His Leu Gln Ile Ser Tyr Leu Lys
225                 230                 235                 240
Asp Glu Asp Thr Ala Thr Tyr Phe Cys Ala Arg Trp Trp Leu Gln Tyr
                245                 250                 255
Ala Leu Asp Asn Trp Gly Gln Gly Thr Ser Val Thr Val Ser Ser
            260                 265                 270

<210>  87
<211>  336
<212>  DNA
<213>  Artificial Sequence

<220>
<223>  synthetic nucleic acid

<400>  87
gatattgtga tgacgcaggc tgcattctcc aatccagtca ctcttggaac atcagcttcc      60
atctcctgca ggtctagtaa gagtctcctc catagtaatg gcatcactta tttctattgg     120
tatctgcaga agccaggcca gtctcctcag ctcctgattt atcagatgtc cagccttgcc     180
tcaggagtcc cagacaggtt cagtagcagt gggtcaggaa ctgatttcac actgagaatc     240
agcagagtgg aggctgagga tgtgggtgtt tattactgtg ctcaaaatct agaacttccg     300
tacacgttcg gaggggggac caagctggaa atcaaa                               336

<210>  88
<211>  112
<212>  PRT
<213>  Artificial Sequence

<220>
<223>  synthetic protein

<400>  88
Asp Ile Val Met Thr Gln Ala Ala Phe Ser Asn Pro Val Thr Leu Gly
1               5                   10                  15
Thr Ser Ala Ser Ile Ser Cys Arg Ser Ser Lys Ser Leu Leu His Ser
            20                  25                  30
Asn Gly Ile Thr Tyr Phe Tyr Trp Tyr Leu Gln Lys Pro Gly Gln Ser
        35                  40                  45
Pro Gln Leu Leu Ile Tyr Gln Met Ser Ser Leu Ala Ser Gly Val Pro
    50                  55                  60
Asp Arg Phe Ser Ser Ser Gly Ser Gly Thr Asp Phe Thr Leu Arg Ile
65                  70                  75                  80
Ser Arg Val Glu Ala Glu Asp Val Gly Val Tyr Tyr Cys Ala Gln Asn
                85                  90                  95
Leu Glu Leu Pro Tyr Thr Phe Gly Gly Gly Thr Lys Leu Glu Ile Lys
            100                 105                 110

<210>  89
<211>  16
<212>  PRT
<213>  Artificial Sequence

<220>
<223>  synthetic protein

<400>  89
Arg Ser Ser Lys Ser Leu Leu His Ser Asn Gly Ile Thr Tyr Phe Tyr
1               5                   10                  15

<210>  90
<211>  7
<212>  PRT
<213>  Artificial Sequence

<220>
<223>  synthetic protein

<400>  90
Gln Met Ser Ser Leu Ala Ser
1               5

<210>  91
<211>  9
<212>  PRT
<213>  Artificial Sequence

<220>
<223>  synthetic protein

<400>  91
Ala Gln Asn Leu Glu Leu Pro Tyr Thr
1               5

<210>  92
<211>  354
<212>  DNA
<213>  Artificial Sequence

<220>
<223>  synthetic nucleic acid

<400>  92
cagatccagt tggtgcagtc tggacctgag gtgaggaggc ctggggagac agtcaagatc      60
tcctgcaagg cttctggata tactttcaca aactttggaa tgaactgggt gaaacaggct     120
ccaggaaagg ctttacagtg gatgggctgg ataaacacct acactggtga gccaacatat     180
gctgatgact tcaagggccg gtttgccttc tctttggaat cctctgccag cactgcccat     240
ttgcagatca gctacctcaa agatgaggac acggctacat atttctgtgc gagatggtgg     300
cttcaatatg ctttggacaa ctggggtcaa ggaacctcag tcaccgtctc ctca            354

<210>  93
<211>  118
<212>  PRT
<213>  Artificial Sequence

<220>
<223>  synthetic protein

<400>  93
Gln Ile Gln Leu Val Gln Ser Gly Pro Glu Val Arg Arg Pro Gly Glu
1               5                   10                  15
Thr Val Lys Ile Ser Cys Lys Ala Ser Gly Tyr Thr Phe Thr Asn Phe
            20                  25                  30
Gly Met Asn Trp Val Lys Gln Ala Pro Gly Lys Ala Leu Gln Trp Met
        35                  40                  45
Gly Trp Ile Asn Thr Tyr Thr Gly Glu Pro Thr Tyr Ala Asp Asp Phe
    50                  55                  60
Lys Gly Arg Phe Ala Phe Ser Leu Glu Ser Ser Ala Ser Thr Ala His

65                  70                  75                  80
Leu Gln Ile Ser Tyr Leu Lys Asp Glu Asp Thr Ala Thr Tyr Phe Cys
                85                  90                  95
Ala Arg Trp Trp Leu Gln Tyr Ala Leu Asp Asn Trp Gly Gln Gly Thr
            100                 105                 110
Ser Val Thr Val Ser Ser
        115

<210>  94
<211>  10
<212>  PRT
<213>  Artificial Sequence

<220>
<223>  synthetic protein

<400>  94
Gly Tyr Thr Phe Thr Asn Phe Gly Met Asn
1               5                   10

<210>  95
<211>  17
<212>  PRT
<213>  Artificial Sequence

<220>
<223>  synthetic protein

<400>  95
Trp Ile Asn Thr Tyr Thr Gly Glu Pro Thr Tyr Ala Asp Asp Phe Lys
1               5                   10                  15
Gly

<210>  96
<211>  11
<212>  PRT
<213>  Artificial Sequence

<220>
<223>  synthetic protein

<400>  96
Ala Arg Trp Trp Leu Gln Tyr Ala Leu Asp Asn
1               5                   10

<210>  97
<211>  816
<212>  DNA
<213>  Artificial Sequence

<220>
<223>  synthetic nucleic acid

<400>  97
atggagtttg ggctgagctg gctttttctt gtggctattt taaaaggtgt ccagtgctct      60
agaaacattg tgctgaccca atctccagct tctttggctg tgtctctagg gcagagggcc     120
accatatcct gcagagccag tgaaaatgtt gataattatg gcaatagttt tatacactgg     180
taccagcaga aaccaggaca gccacccaaa ctcctcatct atcttgcatc caacctagaa     240
tctggggtcc ctgccaggtt cagtggcagt gggtctaggt cagacttcac cctcaccatt     300
gatcctgtgg aggctgatga tgctgcaacc tattactgtc agcaaaataa tgaggatccg     360
tggacgttcg gtggaggcac caagctggaa atcaaaggtg gtggtggttc tggtggtggt     420
ggttctggcg gcggcggctc cggtggtggt ggatcccagg tccaactgca gcagcctggg     480

gctgagctgg tgaagcctgg ggcctcagtg aagatgtcct gcaaggcttc tggctacata 540
tttaccagtt acaatttgca ctgggtaaaa cagacacctg gacagggcct ggaatggatt 600
ggagttattt attcaggaaa tagtgatact tccaacaatc agaagttcaa aggcaaggcc 660
acattgactg aagacaaatc cgccagcaca gcctacctac acctcagcag cctgacatct 720
gaagactcag cggtctattt ctgtgcaagg ggggaatatg gtaaccacgg ggggtttgct 780
tactggggcc aagggactct ggtcactgtc tctgca 816

<210> 98
<211> 272
<212> PRT
<213> Artificial Sequence

<220>
<223> synthetic protein

<400> 98
Met Glu Phe Gly Leu Ser Trp Leu Phe Leu Val Ala Ile Leu Lys Gly
1               5                   10                  15
Val Gln Cys Ser Arg Asn Ile Val Leu Thr Gln Ser Pro Ala Ser Leu
            20                  25                  30
Ala Val Ser Leu Gly Gln Arg Ala Thr Ile Ser Cys Arg Ala Ser Glu
        35                  40                  45
Asn Val Asp Asn Tyr Gly Asn Ser Phe Ile His Trp Tyr Gln Gln Lys
    50                  55                  60
Pro Gly Gln Pro Pro Lys Leu Leu Ile Tyr Leu Ala Ser Asn Leu Glu
65                  70                  75                  80
Ser Gly Val Pro Ala Arg Phe Ser Gly Ser Gly Ser Arg Ser Asp Phe
                85                  90                  95
Thr Leu Thr Ile Asp Pro Val Glu Ala Asp Asp Ala Ala Thr Tyr Tyr
            100                 105                 110
Cys Gln Gln Asn Asn Glu Asp Pro Trp Thr Phe Gly Gly Gly Thr Lys
        115                 120                 125
Leu Glu Ile Lys Gly Gly Gly Gly Ser Gly Gly Gly Gly Ser Gly Gly
    130                 135                 140
Gly Gly Ser Gly Gly Gly Gly Ser Gln Val Gln Leu Gln Gln Pro Gly
145                 150                 155                 160
Ala Glu Leu Val Lys Pro Gly Ala Ser Val Lys Met Ser Cys Lys Ala
                165                 170                 175
Ser Gly Tyr Ile Phe Thr Ser Tyr Asn Leu His Trp Val Lys Gln Thr
            180                 185                 190
Pro Gly Gln Gly Leu Glu Trp Ile Gly Val Ile Tyr Ser Gly Asn Ser
        195                 200                 205
Asp Thr Ser Asn Asn Gln Lys Phe Lys Gly Lys Ala Thr Leu Thr Glu
    210                 215                 220
Asp Lys Ser Ala Ser Thr Ala Tyr Leu His Leu Ser Ser Leu Thr Ser
225                 230                 235                 240
Glu Asp Ser Ala Val Tyr Phe Cys Ala Arg Gly Glu Tyr Gly Asn His
                245                 250                 255
Gly Gly Phe Ala Tyr Trp Gly Gln Gly Thr Leu Val Thr Val Ser Ala
            260                 265                 270

<210> 99
<211> 333
<212> DNA
<213> Artificial Sequence

<220>
<223> synthetic nucleic acid

<400> 99
aacattgtgc tgacccaatc tccagcttct ttggctgtgt ctctagggca gagggccacc 60
atatcctgca gagccagtga aaatgttgat aattatggca atagttttat acactggtac 120
cagcagaaac caggacagcc acccaaactc ctcatctatc ttgcatccaa cctagaatct 180

ggggtccctg ccaggttcag tggcagtggg tctaggtcag acttcaccct caccattgat 240
cctgtggagg ctgatgatgc tgcaacctat tactgtcagc aaaataatga ggatccgtgg 300
acgttcggtg gaggcaccaa gctggaaatc aaa 333

<210> 100
<211> 111
<212> PRT
<213> Artificial Sequence

<220>
<223> synthetic protein

<400> 100
Asn Ile Val Leu Thr Gln Ser Pro Ala Ser Leu Ala Val Ser Leu Gly
1               5                   10                  15
Gln Arg Ala Thr Ile Ser Cys Arg Ala Ser Glu Asn Val Asp Asn Tyr
            20                  25                  30
Gly Asn Ser Phe Ile His Trp Tyr Gln Gln Lys Pro Gly Gln Pro Pro
        35                  40                  45
Lys Leu Leu Ile Tyr Leu Ala Ser Asn Leu Glu Ser Gly Val Pro Ala
    50                  55                  60
Arg Phe Ser Gly Ser Gly Ser Arg Ser Asp Phe Thr Leu Thr Ile Asp
65                  70                  75                  80
Pro Val Glu Ala Asp Asp Ala Ala Thr Tyr Tyr Cys Gln Gln Asn Asn
                85                  90                  95
Glu Asp Pro Trp Thr Phe Gly Gly Gly Thr Lys Leu Glu Ile Lys
            100                 105                 110

<210> 101
<211> 15
<212> PRT
<213> Artificial Sequence

<220>
<223> synthetic protein

<400> 101
Arg Ala Ser Glu Asn Val Asp Asn Tyr Gly Asn Ser Phe Ile His
1               5                   10                  15

<210> 102
<211> 7
<212> PRT
<213> Artificial Sequence

<220>
<223> synthetic protein

<400> 102
Leu Ala Ser Asn Leu Glu Ser
1               5

<210> 103
<211> 9
<212> PRT
<213> Artificial Sequence

<220>
<223> synthetic protein

<400> 103
Gln Gln Asn Asn Glu Asp Pro Trp Thr
1               5

<210>  104
<211>  360
<212>  DNA
<213>  Artificial Sequence

<220>
<223>  synthetic nucleic acid

<400>  104
caggtccaac tgcagcagcc tggggctgag ctggtgaagc ctggggcctc agtgaagatg      60
tcctgcaagg cttctggcta catatttacc agttacaatt tgcactgggt aaaacagaca     120
cctggacagg gcctggaatg gattggagtt atttattcag gaaatagtga tacttccaac     180
aatcagaagt tcaaaggcaa ggccacattg actgaagaca aatccgccag cacagcctac     240
ctacacctca gcagcctgac atctgaagac tcagcggtct atttctgtgc aaggggggaa     300
tatggtaacc acggggggtt tgcttactgg ggccaaggga ctctggtcac tgtctctgca     360

<210>  105
<211>  120
<212>  PRT
<213>  Artificial Sequence

<220>
<223>  synthetic protein

<400>  105
Gln Val Gln Leu Gln Gln Pro Gly Ala Glu Leu Val Lys Pro Gly Ala
1               5                   10                  15
Ser Val Lys Met Ser Cys Lys Ala Ser Gly Tyr Ile Phe Thr Ser Tyr
            20                  25                  30
Asn Leu His Trp Val Lys Gln Thr Pro Gly Gln Gly Leu Glu Trp Ile
        35                  40                  45
Gly Val Ile Tyr Ser Gly Asn Ser Asp Thr Ser Asn Asn Gln Lys Phe
    50                  55                  60
Lys Gly Lys Ala Thr Leu Thr Glu Asp Lys Ser Ala Ser Thr Ala Tyr
65                  70                  75                  80
Leu His Leu Ser Ser Leu Thr Ser Glu Asp Ser Ala Val Tyr Phe Cys
                85                  90                  95
Ala Arg Gly Glu Tyr Gly Asn His Gly Gly Phe Ala Tyr Trp Gly Gln
            100                 105                 110
Gly Thr Leu Val Thr Val Ser Ala
        115                 120

<210>  106
<211>  10
<212>  PRT
<213>  Artificial Sequence

<220>
<223>  synthetic protein

<400>  106
Gly Tyr Ile Phe Thr Ser Tyr Asn Leu His
1               5                   10

<210>  107
<211>  17
<212>  PRT
<213>  Artificial Sequence

<220>
<223>  synthetic protein

<400>  107
Val Ile Tyr Ser Gly Asn Ser Asp Thr Ser Asn Asn Gln Lys Phe Lys
1               5                   10                  15
Gly

<210>  108
<211>  13
<212>  PRT
<213>  Artificial Sequence

<220>
<223>  synthetic protein

<400>  108
Ala Arg Gly Glu Tyr Gly Asn His Gly Gly Phe Ala Tyr
1               5                   10

<210>  109
<211>  795
<212>  DNA
<213>  Artificial Sequence

<220>
<223>  synthetic nucleic acid

<400>  109
atggagtttg ggctgagctg gctttttctt gtggctattt taaaaggtgt ccagtgctct      60
agagacatcc agatgactca gtctccagcc tccctaactg tatttgtggg ggaaactgtc     120
accatcacat gtcgagcaag tgagaatatt tacagtaatt tagcgtggta tcagcagaaa     180
cagggaaaat ctcctcagct cctggtctat actgcaacaa atttaataga tggtgtgcca     240
tcaaggttca gtggcagtgg gtccggcaca cagtattccc tcaagatcaa cagcctgcag     300
tctgaagatt tcgggagtta ttactgtcaa cattttgggg gtactccggt cacgttcggt     360
gctgggacca agctggaaat caaaggtggt ggtggttctg gtggtggtgg ttctggcggc     420
ggcggctccg gtggtggtgg atcccaggtc caactgcagc agcctggggc agaactggtg     480
aagcctgggg cctcagtgag gatgtcctgc agggcttctg gctacacttt taccagttac     540
aatttgcact gggtaaaaca gacacctgga cagggcctgg aatggattgg ggctattcat     600
ccaggagata attatacttc ctacaatcag attttcaacg acaaggccac attgactgca     660
gacaaatcct ccagcacagc ctacatgcag ctcagtagcc tgacatctga ggactctgcg     720
gtctatttct gtgcaaggaa ttactacggt agtcttgatt actggggcca aggcaccact     780
ctcacagtct cctca                                                      795

<210>  110
<211>  265
<212>  PRT
<213>  Artificial Sequence

<220>
<223>  synthetic protein

<400>  110
Met Glu Phe Gly Leu Ser Trp Leu Phe Leu Val Ala Ile Leu Lys Gly
1               5                   10                  15
Val Gln Cys Ser Arg Asp Ile Gln Met Thr Gln Ser Pro Ala Ser Leu
            20                  25                  30
Thr Val Phe Val Gly Glu Thr Val Thr Ile Thr Cys Arg Ala Ser Glu
        35                  40                  45
Asn Ile Tyr Ser Asn Leu Ala Trp Tyr Gln Gln Lys Gln Gly Lys Ser
    50                  55                  60
Pro Gln Leu Leu Val Tyr Thr Ala Thr Asn Leu Ile Asp Gly Val Pro
65                  70                  75                  80
Ser Arg Phe Ser Gly Ser Gly Ser Gly Thr Gln Tyr Ser Leu Lys Ile
                85                  90                  95

Asn Ser Leu Gln Ser Glu Asp Phe Gly Ser Tyr Tyr Cys Gln His Phe
                100                 105                 110
Trp Gly Thr Pro Val Thr Phe Gly Ala Gly Thr Lys Leu Glu Ile Lys
        115                 120                 125
Gly Gly Gly Gly Ser Gly Gly Gly Gly Ser Gly Gly Gly Gly Ser Gly
    130                 135                 140
Gly Gly Gly Ser Gln Val Gln Leu Gln Gln Pro Gly Ala Glu Leu Val
145                 150                 155                 160
Lys Pro Gly Ala Ser Val Arg Met Ser Cys Arg Ala Ser Gly Tyr Thr
                165                 170                 175
Phe Thr Ser Tyr Asn Leu His Trp Val Lys Gln Thr Pro Gly Gln Gly
                180                 185                 190
Leu Glu Trp Ile Gly Ala Ile His Pro Gly Asp Asn Tyr Thr Ser Tyr
        195                 200                 205
Asn Gln Ile Phe Asn Asp Lys Ala Thr Leu Thr Ala Asp Lys Ser Ser
    210                 215                 220
Ser Thr Ala Tyr Met Gln Leu Ser Ser Leu Thr Ser Glu Asp Ser Ala
225                 230                 235                 240
Val Tyr Phe Cys Ala Arg Asn Tyr Tyr Gly Ser Leu Asp Tyr Trp Gly
                245                 250                 255
Gln Gly Thr Thr Leu Thr Val Ser Ser
                260                 265

<210>  111
<211>  321
<212>  DNA
<213>  Artificial Sequence

<220>
<223>  synthetic nucleic acid

<400>  111
gacatccaga tgactcagtc tccagcctcc ctaactgtat ttgtggggga aactgtcacc      60
atcacatgtc gagcaagtga gaatatttac agtaatttag cgtggtatca gcagaaacag     120
ggaaaatctc ctcagctcct ggtctatact gcaacaaatt taatagatgg tgtgccatca     180
aggttcagtg gcagtgggtc cggcacacag tattccctca agatcaacag cctgcagtct     240
gaagatttcg ggagttatta ctgtcaacat ttttggggta ctccggtcac gttcggtgct     300
gggaccaagc tggaaatcaa a                                               321

<210>  112
<211>  107
<212>  PRT
<213>  Artificial Sequence

<220>
<223>  synthetic protein

<400>  112
Asp Ile Gln Met Thr Gln Ser Pro Ala Ser Leu Thr Val Phe Val Gly
1               5                   10                  15
Glu Thr Val Thr Ile Thr Cys Arg Ala Ser Glu Asn Ile Tyr Ser Asn
            20                  25                  30
Leu Ala Trp Tyr Gln Gln Lys Gln Gly Lys Ser Pro Gln Leu Leu Val
        35                  40                  45
Tyr Thr Ala Thr Asn Leu Ile Asp Gly Val Pro Ser Arg Phe Ser Gly
    50                  55                  60
Ser Gly Ser Gly Thr Gln Tyr Ser Leu Lys Ile Asn Ser Leu Gln Ser
65                  70                  75                  80
Glu Asp Phe Gly Ser Tyr Tyr Cys Gln His Phe Trp Gly Thr Pro Val
                85                  90                  95
Thr Phe Gly Ala Gly Thr Lys Leu Glu Ile Lys
            100                 105

<210>  113
<211>  11
<212>  PRT
<213>  Artificial Sequence

<220>
<223>  synthetic protein

<400>  113
Arg Ala Ser Glu Asn Ile Tyr Ser Asn Leu Ala
1               5                   10

<210>  114
<211>  6
<212>  PRT
<213>  Artificial Sequence

<220>
<223>  synthetic protein

<400>  114
Thr Ala Thr Asn Leu Ile
1               5

<210>  115
<211>  9
<212>  PRT
<213>  Artificial Sequence

<220>
<223>  synthetic protein

<400>  115
Gln His Phe Trp Gly Thr Pro Val Thr
1               5

<210>  116
<211>  351
<212>  DNA
<213>  Artificial Sequence

<220>
<223>  synthetic nucleic acid

<400>  116
caggtccaac tgcagcagcc tggggcagaa ctggtgaagc ctggggcctc agtgaggatg        60
tcctgcaggg cttctggcta cacttttacc agttacaatt tgcactgggt aaaacagaca       120
cctggacagg gcctggaatg gattggggct attcatccag gagataatta tacttcctac       180
aatcagattt tcaacgacaa ggccacattg actgcagaca aatcctccag cacagcctac       240
atgcagctca gtagcctgac atctgaggac tctgcggtct atttctgtgc aaggaattac       300
tacggtagtc ttgattactg gggccaaggc accactctca cagtctcctc a                351

<210>  117
<211>  117
<212>  PRT
<213>  Artificial Sequence

<220>
<223>  synthetic protein

<400>  117
Gln Val Gln Leu Gln Gln Pro Gly Ala Glu Leu Val Lys Pro Gly Ala
1               5                   10                  15

Ser Val Arg Met Ser Cys Arg Ala Ser Gly Tyr Thr Phe Thr Ser Tyr
            20                  25                  30
Asn Leu His Trp Val Lys Gln Thr Pro Gly Gln Gly Leu Glu Trp Ile
        35                  40                  45
Gly Ala Ile His Pro Gly Asp Asn Tyr Thr Ser Tyr Asn Gln Ile Phe
    50                  55                  60
Asn Asp Lys Ala Thr Leu Thr Ala Asp Lys Ser Ser Ser Thr Ala Tyr
65                  70                  75                  80
Met Gln Leu Ser Ser Leu Thr Ser Glu Asp Ser Ala Val Tyr Phe Cys
                85                  90                  95
Ala Arg Asn Tyr Tyr Gly Ser Leu Asp Tyr Trp Gly Gln Gly Thr Thr
            100                 105                 110
Leu Thr Val Ser Ser
        115

<210>  118
<211>  10
<212>  PRT
<213>  Artificial Sequence

<220>
<223>  synthetic protein

<400>  118
Gly Tyr Thr Phe Thr Ser Tyr Asn Leu His
1               5                   10

<210>  119
<211>  17
<212>  PRT
<213>  Artificial Sequence

<220>
<223>  synthetic protein

<400>  119
Ala Ile His Pro Gly Asp Asn Tyr Thr Ser Tyr Asn Gln Ile Phe Asn
1               5                   10                  15
Asp

<210>  120
<211>  10
<212>  PRT
<213>  Artificial Sequence

<220>
<223>  synthetic protein

<400>  120
Ala Arg Asn Tyr Tyr Gly Ser Leu Asp Tyr
1               5                   10

<210>  121
<211>  135
<212>  DNA
<213>  Homo sapiens

<400>  121
accacgacgc cagcgccgcg accaccaaca ccggcgccca ccatcgcgtc gcagcccctg        60
tccctgcgcc cagaggcgtg ccggccagcg gcggggggcg cagtgcacac gagggggctg       120
gacttcgcct gtgat                                                        135

<210>  122
<211>  45
<212>  PRT
<213>  Homo sapiens

<400>  122
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp
        35                  40                  45

<210>  123
<211>  72
<212>  DNA
<213>  Homo sapiens

<400>  123
atctacatct gggcgccctt ggccgggact tgtggggtcc ttctcctgtc actggttatc      60
accctttact gc                                                          72

<210>  124
<211>  24
<212>  PRT
<213>  Homo sapiens

<400>  124
Ile Tyr Ile Trp Ala Pro Leu Ala Gly Thr Cys Gly Val Leu Leu Leu
1               5                   10                  15
Ser Leu Val Ile Thr Leu Tyr Cys
            20

<210>  125
<211>  123
<212>  DNA
<213>  Homo sapiens

<400>  125
aggagtaaga ggagcaggct cctgcacagt gactacatga acatgactcc ccgccgcccc      60
gggcccaccc gcaagcatta ccagccctat gccccaccac gcgacttcgc agcctatcgc     120
tcc                                                                   123

<210>  126
<211>  41
<212>  PRT
<213>  Homo sapiens

<400>  126
Arg Ser Lys Arg Ser Arg Leu Leu His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Ser
        35                  40

<210>  127
<211>  126
<212>  DNA
<213>  Homo sapiens

<400>  127
aaacggggca gaaagaaact cctgtatata ttcaaacaac catttatgag accagtacaa      60

actactcaag aggaagatgg ctgtagctgc cgatttccag aagaagaaga aggaggatgt 120
gaactg 126

<210> 128
<211> 42
<212> PRT
<213> Homo sapiens

<400> 128
Lys Arg Gly Arg Lys Lys Leu Leu Tyr Ile Phe Lys Gln Pro Phe Met
1               5                   10                  15
Arg Pro Val Gln Thr Thr Gln Glu Glu Asp Gly Cys Ser Cys Arg Phe
            20                  25                  30
Pro Glu Glu Glu Glu Gly Gly Cys Glu Leu
        35                  40

<210> 129
<211> 339
<212> DNA
<213> Homo sapiens

<400> 129
agagtgaagt tcagcaggag cgcagacgcc cccgcgtacc agcagggcca gaaccagctc 60
tataacgagc tcaatctagg acgaagagag gagtacgatg ttttggacaa gagacgtggc 120
cgggaccctg agatgggggg aaagccgaga aggaagaacc ctcaggaagg cctgtacaat 180
gaactgcaga aagataagat ggcggaggcc tacagtgaga ttgggatgaa aggcgagcgc 240
cggaggggca aggggcacga tggcctttac cagggtctca gtacagccac caaggacacc 300
tacgacgccc ttcacatgca ggccctgccc cctcgctaa 339

<210> 130
<211> 112
<212> PRT
<213> Homo sapiens

<400> 130
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110

## Description

### Description

This application claims the priority of Chinese patent application CN 201910750137.1 filed with the China National Intellectual Property Administration on August 14, 2019, the entirety of which is incorporated herein by reference.

### Technical Field

The present disclosure relates to the field of biological medicines, and particularly relates to a B7-H3-targeted chimeric antigen receptor, immune effector cells expressing the B7-H3-targeted chimeric antigen receptor, and use thereof.

### Background Art

Cancer is one of the main diseases threatening human health. Common cancer therapies include radiotherapy, chemotherapy, targeted therapy, etc., and currently, immunotherapy is gradually becoming a mainstream therapy. Chimeric antigen receptor-mediated cell therapy is a type of immunotherapy, and CD19-targeted chimeric antigen receptor T-cell therapy has made outstanding achievements in the treatment of B-cell-associated tumors, and has cured some patients (whose tumors do not recur in 5 years). Therefore, these achievements have prompted FDA's approval of the CD19-targeted chimeric antigen receptor T-cell therapy, which promotes the rapid development of this therapy in the field of blood cancer. However, due to the lack of specific targets, the heterogeneity of solid tumor (different tumor cells have different phenotypes), tumor microenvironment, etc., the chimeric antigen receptor-mediated cell therapy didn't perform well for the treatment of solid tumors, and therefore, developing chimeric antigen receptors for new targets is particularly critical to solving this problem. B7-H3 is a membrane protein belonging to the B7 immunomodulatory factor family, which is present in two isoforms of 2Ig-B7-H3 and 4Ig-B7-H3 in the human body, but only in one isoform of 2Ig-B7-H3 in mice. Some literature reported that B7-H3 could reduce the level of type I interferon released by T cells, and could also inhibit the killing function of natural killer cells, and therefore, B7-H3 is regarded as an immunosuppressive factor. Other reports also confirmed that B7-H3 can inhibit diseases such as graft-versus-host reaction, heart transplant rejection, airway inflammation, and experimental autoimmune encephalomyelitis, etc. However, in certain literature, B7-H3 is described as a T-cell costimulatory factor in the in vitro and in vivo autoimmune models.

B7-H3 is expressed at very low level in normal human tissues but is usually overexpressed (ranges from 74%-94%) in tumors. In additions, studies have found that B7-H3 is also highly expressed in tumor-associated blood vessels and tumor stromal fibroblasts. The high expression of B7-H3 is closely related with a low number of tumor-infiltrating lymphocytes, the rapid progression of cancer, and a poor clinical outcome, and is common in cancers such as glioma, pancreatic cancer, prostate cancer, ovarian cancer, lung cancer, and kidney cancer, etc. Because B7-H3 is highly expressed in a variety of solid tumors, it can be a good target for solid tumors. Meanwhile, B7-H3 is expressed on the cell surface, so that chimeric antigen receptor-mediated cell therapy can be used to target B7-H3 to kill tumor cells without damaging normal cells.

### Summary of the Invention

In view of the above, the present disclosure is directed to provide a B7-H3 protein-targeted chimeric antigen receptor (CAR), immune effector cells expressing the CAR, and use thereof in cancer diagnosis, treatment, and prevention.

Specifically, the present disclosure proposes the following technical solutions:
In one aspect, the present disclosure provides a B7-H3-targeted chimeric antigen receptor (CAR), characterized by including: an anti-B7-H3 binding domain, a hinge region, a transmembrane domain, and a signal transduction domain.

In one aspect, in the CAR of the present disclosure, the anti-B7-H3 binding domain comprises an anti-B7-H3 single-chain antibody or antigen-binding portion, and the antibody or antigen-binding portion comprises a light-chain CDR selected from amino acid sequences of SEQ ID NO: 5-7, 17-19, 29-31, 41-43, 53-55, 65-67, 77-79, 89-91, 101-103, 113-115 or any variant thereof; and/or, comprises a heavy-chain CDR selected from amino acid sequences of SEQ ID NO: 10-12, 22-24, 34-36, 46-48, 58-60, 70-72, 82-84, 94-96, 106-108, 118-120 or any variant thereof.

In the CAR according to any one of the preceding aspects, the anti-B7-H3 binding domain comprises an anti-B7-H3 antibody or antigen-binding portion, and the antibody or antigen-binding portion comprises a light-chain CDR1 selected from amino acid sequences of SEQ ID NO: 5, 17, 29, 41, 53, 65, 77, 89, 101, 113 or any variant thereof, a light-chain CDR2 selected from amino acid sequences of SEQ ID NO: 6, 18, 30, 42, 54, 66, 78, 90, 102, 114 or any variant thereof, and a light-chain CDR3 selected from amino acid sequences of SEQ ID NO: 7, 19, 31, 43, 55, 67, 79, 91, 103, 115 or any variant thereof; and/or, comprises a heavy-chain CDR1 selected from amino acid sequences of SEQ ID NO: 10, 22, 34, 46, 58, 70, 82, 94, 106, 118 or any variant thereof, a heavy-chain CDR2 selected from amino acid sequences of SEQ ID NO: 11, 23, 35, 47, 59, 71, 83, 95, 107, 119 or any variant thereof, and a heavy-chain CDR3 selected from amino acid sequences of SEQ ID NO: 12, 24, 36, 48, 60, 72, 84, 96, 108, 120 or any variant thereof.

In the CAR according to any one of the preceding aspects, the anti-B7-H3 binding domain comprises an anti-B7-H3 antibody or antigen-binding portion, and the antibody or antigen-binding portion comprises a light-chain sequence selected from amino acid sequences of SEQ ID NO: 4, 16, 28, 40, 52, 64, 76, 88, 100, 112 or any variant thereof; and/or, comprises a heavy-chain sequence selected from amino acid sequences of SEQ ID NO: 9, 21, 33, 45, 57, 69, 81, 93, 105, 117 or any variant thereof.

In the CAR according to any one of the preceding aspects, the anti-B7-H3 binding domain comprises an anti-B7-H3 antibody (scFv) domain; preferably, the anti-B7-H3 single-chain antibody has an amino acid sequence selected from amino acid sequences of SEQ ID NO: 2, 14, 26, 38, 50, 62, 74, 86, 98, 110 or any variant thereof.

In the CAR according to any one of the preceding aspects, the transmembrane domain is selected from one or more of transmembrane domains of α, β, ζ chains of TCR, CD3y, CD3δ, CD3ε, CD3ζ, CD4, CD5, CD8α, CD9, CD16, CD22, CD27, CD28, CD33, CD37, CD45, CD64, CD80, CD86, CD134, 4-1BB, CD152, CD154, PD-1; preferably, the transmembrane domain is selected from one or more of transmembrane domains of CD8α, CD4, CD45, PD-1, CD154, CD28; preferably, the transmembrane domain is selected from one or more of transmembrane domains of CD8α or CD28; more preferably, the transmembrane domain has an amino acid sequence selected from amino acid sequences of SEQ ID NO: 124 or any variant thereof; the hinge region is selected from one or more of an extracellular hinge region of CD8, a hinge region of IgG1 Fc CH2CH3, a hinge region of IgD, an extracellular hinge region of CD28, a hinge region of IgG4 Fc CH2CH3, and an extracellular hinge region of CD4; preferably, the hinge region is a hinge region of CD8α; more preferably, the hinge region has an amino acid sequence selected from amino acid sequences of SEQ ID NO: 122 or any variant thereof.

In the CAR according to any one of the preceding aspects, the signal transduction domain is selected from one or more of intracellular signaling regions of TCRζ, FcRy, FcRβ, CD3y, CD3δ,CD3ε, CD5, CD22, CD79a, CD79b, CD278(ICOS), CD66d, CD3ζ; preferably, the signal transduction domain is selected from an intracellular signaling region of CD3ζ; more preferably, the signal transduction domain has an amino acid sequence selected from amino acid sequences of SEQ ID NO: 130 or any variant thereof; preferably, the signal transduction domain further comprises one or more costimulatory domains; preferably, the costimulatory domain is selected from one or more of intracellular signaling regions of CARD11, CD2, CD7, CD27, CD28, CD30, CD40, CD54, CD83, OX40, 4-1BB, CD134, CD150, CD152, CD223, CD270, PD-L2, PD-L1, CD278, DAP10, LAT, NKD2C, SLP76, TRIM, FcεRlγ, MyD88, 4-1BB; preferably, the costimulatory domain is selected from intracellular signaling regions of 4-1BB, CD134, CD28, and OX40; preferably, the costimulatory domain is selected from one or more of intracellular signaling regions of 4-1 BB or CD28; more preferably, the costimulatory domain has an amino acid sequence selected from amino acid sequence of SEQ ID NO: 126, 128 or any variant thereof.

In one aspect, the present disclosure provides an isolated nucleic acid molecule, comprising a polynucleotide sequence encoding the CAR according to any one of the preceding aspects; preferably, the nucleic acid molecule has a nucleotide sequence selected from nucleotide sequences of SEQ ID NO: 1, 13, 25, 37, 49, 61, 73, 85, 97, 109 or any variant thereof.

In one aspect, the present disclosure provides a nucleic acid construct, comprising the nucleic acid molecule according to any one of the preceding aspects; preferably, the nucleic acid construct is a viral vector; more preferably, the viral vector is one or more of a retroviral vector, a lentiviral vector, an adenoviral vector, an adeno-associated viral vector.

In one aspect, the present disclosure provides a virus, comprising the nucleic acid molecule according to any one of the preceding aspects, or comprising the nucleic acid construct according to any one of the preceding aspects; preferably, the virus is one or more of a retrovirus, a lentivirus, an adenovirus, an adeno-associated virus.

The present disclosure also provides use of the CAR according to any one of the preceding aspects, the nucleic acid molecule according to any one of the preceding aspects, the nucleic acid construct according to any one of the preceding aspects, or the virus according to any one of the preceding aspects in preparation of genetically modified immune cells targeting tumor cells expressing B7-H3.

In one aspect, the present disclosure provides an isolated host cell, expressing the CAR according to any one of the preceding aspects, or comprising the nucleic acid molecule according to any one of the preceding aspects, or comprising the nucleic acid construct according to any one of the preceding aspects, or comprising the virus according to any one of the preceding aspects; preferably, the host cell is a mammalian cell; more preferably, the host cell is one or more of a T cell, an NK cell or cell line, a γδT cell, an NKT cell, a macrophage or cell line, a PG13 cell line; more preferably, the host cell is a T cell; the most preferably, the host cell is a primarily cultured T cell.

The host cell according to any one of the preceding aspects also expresses other effector molecules that include, but are not limited to, a cytokine, another CAR, a chemokine receptor, an siRNA reducing the expression of PD-1 or a protein blocking PD-L1, a TCR, or a safety switch;
preferably, the cytokine is selected from one or more of TNF-α, TNF-β, VEGF, TPO, NGF-β, PDGF, TGF-α, TGF-β, IGF-I, IGF-II, EPO, M-CSF, IL-1, IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, an interferon, an angiostatin, a thrombospondin, or an endostatin;
preferably, the chemokine receptor is selected from one or more of CCR2, CCR5, CXCR2, CXCR4;
preferably, the safety switch is selected from one or more of HSVTK, VZVTK, iCaspase-9, iCaspase-1, iCaspase-8, truncated EGFR, RQR8.

In one aspect, the present disclosure provides a pharmaceutical composition, comprising one or more of the CAR according to any one of the preceding aspects, the nucleic acid molecule according to any one of the preceding aspects, the nucleic acid construct according to any one of the preceding aspects, the virus according to any one of the preceding aspects, the host cell according to any one of the preceding aspects, and a pharmaceutically acceptable carrier.

The present disclosure also provides use of the CAR according to any one of the preceding aspects, the nucleic acid molecule according to any one of the preceding aspects, the nucleic acid construct according to any one of the preceding aspects, the virus according to any one of the preceding aspect, the host cell according to any one of the preceding aspects, or the pharmaceutical composition in preparation of a drug; preferably, the drug is used to diagnose, treat or prevent one or more of cancer, an inflammatory disease, an autoimmune disease; preferably, the drug is used to diagnose, treat or prevent a tumor expressing B7-H3; more preferably, the drug is used to diagnose, treat or prevent one or more of brain cancer, pancreatic cancer, ovarian cancer, kidney cancer, bladder cancer, skin cancer, stomach cancer, intestinal cancer, head and neck cancer, thyroid cancer, prostate cancer, lung cancer, and Kaposi's sarcoma; more preferably, the brain cancer is selected from one or more of glioblastoma, astrocytoma, meningioma, oligodendroglioma, neuroglioma.

The present disclosure achieves the following beneficial effects:
Mouse models of brain glioma have verified that the B7-H3-targeted chimeric antigen receptor-T cells prepared in the present disclosure could remove tumors in most of the mice, and could prevent tumors from recurring within a certain period; and meanwhile, the survival time of the mice was significantly prolonged, which indicates that the B7-H3-targeted chimeric antigen receptor-T cells of the present disclosure can kill tumor cells in vivo, and can be used as a potential therapy for cancer.

### Brief Description of the Drawings

Fig. 1: the expression of B7-H3 in different tumor cell lines.
Fig. 2: the expression of B7-H3 in brain glioma samples.
Fig. 3: the basic structure of a B7-H3-targeted chimeric antigen receptor, and in the figure, B7-H3.28 CAR represents a chimeric antigen receptor whose costimulatory domain is CD28, and B7-H3.BB CAR represents a chimeric antigen receptor whose costimulatory domain is 4-1BB.
Fig. 4: Fig. 4A: the expression of the humanized antibody 15C11-derived B7-H3-targeted chimeric antigen receptor according to the present disclosure on T cells (from an individual healthy donor); and Fig. 4B: the expression of the humanized antibody 15C11-derived B7-H3-targeted chimeric antigen receptor according to the present disclosure on T cells (from multiple healthy donors).
Fig. 5: the killing effects of humanized antibody 15C11-derived B7-H3-targted chimeric antigen receptor-T cells according to the present disclosure on different types of tumor cells in the co-culture systems in vitro.
Fig. 6: Fig. 6A: comparing with the other chimeric antigen receptors (CAR1 and CAR2) targeting the same antigen, the affinity of the humanized antibody 15C11-derived B7-H3-targeted chimeric antigen receptor to the antigen according to the present disclosure;
and Fig. 6B: comparing with the other chimeric antigen receptors (CAR1 and CAR2) targeting the same antigen when killing tumor cells, the levels of cytokines released by the humanized antibody 15C11-derived B7-H3-targeted chimeric antigen receptor CAR-T cells according to the present disclosure.
Fig. 7: the killing effect of the humanized antibody 15C11-derived B7-H3-targeted chimeric antigen receptor-T cells according to the present disclosure on brain glioma in vivo.
Fig. 8: the increased survival rates of mouse models of brain glioma that are treated with the humanized antibody 15C11-derived B7-H3-targeted chimeric antigen receptor-T cells according to the present disclosure.
Fig. 9: Fig. 9A shows the killing effect of the humanized antibody 15C11-derived B7-H3-targeted chimeric antigen receptor-T cells according to the present disclosure on patient-derived glioma neurospheres in vitro (bright field); and Fig. 9B shows the killing effect of the humanized antibody 15C11-derived B7-H3-targeted chimeric antigen receptor-T cells according to the present disclosure on patient-derived glioma neurospheres in vitro (flow cytometry).
Fig. 10: the humanized antibody 15C11-derived B7-H3-targeted chimeric antigen receptor-T cells according to the present disclosure significantly prolong the survival time of patient-derived glioma neurosphere xenograft mouse models.
Fig. 11: the killing effects of 9 kinds of other murine-derived B7-H3-targeted antigen receptor-T cells on tumor cells in vitro.
Fig. 12: the cytokines released by the 9 kinds of murine-derived B7-H3-targeted antigen receptor-T cells when killing tumors.

### Detailed Description of the Invention

### I. Definitions

Unless other specified, the scientific and technical terms used herein have the same meaning as understood by those skilled in the art. Furthermore, terms related to protein and nucleic acid chemistry, molecular biology, cell and tissue culture, microbiology, and immunology, and experimental steps used herein are the widely used terms and conventional steps in the corresponding art. Meanwhile, in order to provide a better understanding of the present disclosure, the relevant terms will be defined and described below.

It should be understood that the terms are used for the purpose of describing specific embodiments, and are not intended to limit the present disclosure.

As used herein, unless otherwise specified, the term "about", when referring to a measurable value such as an amount and a period of time, is meant to encompass variations of ± 10%, more preferably ± 5%, even more preferably ± 1%, and still more preferably ± 0.1% from the given value, as long as such variations are suitable for practicing the disclosed methods.

As used herein, the term "activation" refers to a state of T cells that are sufficiently stimulated to induce detectable cell proliferation. Activation can also be associated with induced cytokine production and detectable effector function. The terms "activated T cells" and the like refer to T cells undergoing cell division.

As used herein, the term "antibody" refers to an immunoglobulin molecule that specifically binds to an antigen. An antibody may be a natural or recombinant intact immunoglobulin or may be an immune response portion of an intact immunoglobulin. An antibody is generally a tetramer of immunoglobulin molecules. The antibody of the present disclosure can exist in a variety of forms which include, but are not limited to, a polyclonal antibody, a monoclonal antibody, Fv, Fab, F(ab)2, etc., and a single-chain antibody and a humanized antibody (Harlow, et al., 1999, Using Antibodies: A Laboratory Manual, Cold Spring Harbor Laboratory Press, NY; Harlow, et al., 1989, Antibodies: A Laboratory Manual, Cold Spring Harbor, New York; Houston, et al., 1988, Proc. Natl. Acad. Sci., USA 85: 5879-5883; and Bird, et al., 1988, Science 242: 423-426).

As used herein, an "antibody fragment" or "antigen-binding fragment" of an antibody refers to any part of a full-length antibody, which is shorter than the full-length antibody, but at least comprises a partial variable region (e.g., one or more CDRs and/or one or more antigen-binding sites) binding to an antigen of the antibody and thus reserves binding specificity and at least partial specific binding ability of the full-length antibody. Therefore, an antigen-binding fragment refers to an antibody fragment comprising an antigen-binding portion that binds to the same antigen as an antibody from which the antibody fragment is derived. An antibody fragment includes an antibody derivative produced by enzymatic treatment of a full-length antibody, and a derivative produced by synthesis such as a derivative produced by recombination. An antibody comprises antibody fragments. Embodiments of the antibody fragment include, but are not limited to, Fab, Fab', F(ab')2, single-chain Fv (scFv), Fv, dsFv, double-antibody, Fd and Fd' fragments, and other fragments including modified fragments (referring to, for example, Methods in Molecular Biology, Vol 207: Recombinant Antibodies for Cancer Therapy Methods and Protocols (2003); Chapter 1; p3-25, Kipriyanov). The fragments may comprise multiple chains that are linked together, for example, via disulfide bonds and/or via peptide linkers. An antibody fragment generally comprises at least or about 50 amino acids, and typically comprises at least or about 200 amino acids. An antigen-binding fragment includes any antibody fragment that is inserted into an antibody framework (for example, through replacement of a corresponding region) to obtain an antibody that immunospecifically binds (that is, showing Ka of at least or at least about 10⁷-10⁸ M⁻¹) to an antigen. A "functional fragment" or "anti-B7-H3 antibody analogue" is a fragment or analogue that can prevent or substantially reduce the ability of a receptor to bind to a ligand or initiate signal transduction. As used herein, a functional fragment generally has the same meaning as an "antibody fragment", and for an antibody, it may refer to a fragment that can prevent or substantially reduce the ability of a receptor to bind to a ligand or initiate signal transduction, such as Fv, Fab, and F(ab')2. An "Fv" fragment is composed of a dimer (VH-VL dimer) formed by a variable domain of one heavy chain and a variable domain of one light chain by mean of non-covalent binding. In this configuration, 3 CDRs of each variable domain interact to determine target-binding sites on the surface of the VH-VL dimer, which is the same as an intact antibody. The 6 CDRs together confer the target-binding specificity of the intact antibody. However, even a single variable domain (or a half of an Fv that comprises only 3 target-specific CDRs) can still have the ability to recognize and bind to a target.

As used herein, a "monoclonal antibody" refers to a population of identical antibodies, which means that each single antibody molecule in the monoclonal antibody population is identical to other antibody molecules. This property is in contrast to that of a population of polyclonal antibodies, which comprises a variety of antibodies having different sequences. A monoclonal antibody can be prepared by a number of well-known methods (Smith, et al., (2004) J. Clin. Pathol. 57, 912-917; and Nelson, et al., J Clin Pathol (2000), 53, 111-117). For example, a monoclonal antibody can be prepared by immortalizing B cells, for example, by fusing with myeloma cells to generate a hybridoma cell line or by infecting B cells with a virus such as EBV. Recombinant technologies can also be used to prepare an antibody from a clonal population of host cells in vitro by transforming the host cells with a plasmid carrying an artificial sequence of nucleotides encoding the antibody.

As used herein, a "heavy chain" of an antibody refers to the larger chain in the two types of polypeptide chains that exist in all antibody molecules in their autogenetic conformations. As used herein, a "light chain" of an antibody refers to the smaller chain in the two types of polypeptide chains that exist in all antibody molecules in their autogenetic conformations, and κ and λ light chains refer to the two major isoforms of a light chain of an antibody.

As used herein, the term "scFv" refers to a fusion protein that comprises at least one antibody fragment comprising a variable region of a light chain and at least one antibody fragment comprising a variable region of a heavy chain, the variable regions of the light chain and the heavy chain are linked via a short flexible polypeptide linker, and can be expressed as a single-chain polypeptide, the scFv reserves the specificity of an intact antibody from which it is derived. Unless otherwise specified, as used herein, scFv can comprise the VL and VH variable regions in any order (e.g., relative to the N-terminal end and the C-terminal end of a polypeptide), and scFv can comprise VL-linker-VH or can comprise VH-linker-VL.

As used herein, the term "gene synthesis" refers to the production by using a recombinant DNA technology or the acquisition by using a synthetic DNA or amino acid sequence technology available and well known in the art.

As used herein, the term "antigen" or "Ag" is defined as a molecule that elicits an immune response, which may involve antibody production, or activation of specific immunocompetent cells, or both. Those skilled in the art will understand that any macromolecule, actually including all proteins or peptides, can be used as an antigen. In addition, an antigen can be derived from recombinant or genomic DNA. Those skilled in the art will understand that any DNA, comprising a nucleotide sequence or partial nucleotide sequence encoding a protein that elicits an immune response, encodes an "antigen" as used herein. In addition, those skilled in the art will understand that an antigen is not necessarily encoded separately by a full-length nucleotide sequence of a gene. It is readily apparent that the present invention includes, but is not limited to, use of more than one partial nucleotide sequences of a gene, and such nucleotide sequences are arranged in different combinations to elicit a desired immune response. In addition, those skilled in the art will understand that an antigen is not necessarily encoded by a "gene". It is readily apparent that an antigen can be produced, synthesized or derived from a biological sample. The biological sample may include, but is not limited to, a tissue sample, a tumor sample, a cell, and a biological fluid.

As used herein, the term "anti-tumor effect" refers to a biological effect that can be clearly indicated by reduction in tumor volume, reduction in the number of tumor cells, reduction in the number of metastases, increase in life expectancy, or improvements of various physiological symptoms associated with cancer. An "anti-tumor effect" can also be clearly indicated by the ability of the peptide, the polynucleotide, the cell or the antibody of the present disclosure to prevent a tumor from forming at the first position.

As used herein, the term "cancer" is defined as a disease characterized by rapid and uncontrolled growth of aberrant cells. Cancer cells can spread locally or spread to other parts of the body via the blood flow and the lymphatic system. Examples of various cancers include, but are not limited to, brain cancer (e.g., astrocytoma, meningioma, oligodendroglioma, and neuroglioma), pancreatic cancer, ovarian cancer, kidney cancer, bladder cancer, skin cancer, stomach cancer, intestinal cancer, head and neck cancer, thyroid cancer, prostate cancer, lung cancer, Kaposi's sarcoma, etc.

As used herein, examples of the term "inflammatory disease" include, but are not limited to, asthma, encephalitis, inflammatory bowel disease, chronic obstructive pulmonary disease (COPD), allergy, septic shock, pulmonary fibrosis, undifferentiated spondyloarthropathy, undifferentiated osteoarthritis, arthritis, inflammatory osteolysis, and chronic inflammation caused by chronic viral infection and bacterial infection.

As used herein, examples of the term "autoimmune disease" include, but are not limited to, alopecia areata, ankylosing spondylitis, antiphospholipid syndrome, autoimmune Addison's disease, adrenal autoimmune disease, autoimmune hemolytic anemia, autoimmune hepatitis, autoimmune oophoritis and orchitis, autoimmune thrombocytopenia, Behçet's disease, bullous pemphigoid, cardiomyopathy, celiac sprue, dermatitis, chronic fatigue immune dysfunction syndrome (CFIDS), chronic inflammatory demyelinating polyneuropathy, Churg-Strauss syndrome, cicatricial pemphigoid, CREST syndrome, cold agglutinin disease, Crohn's disease, discoid lupus erythematosus, idiopathic mixed cryoglobulinemia, fibromyalgia-fibromyositis, glomerulonephritis, Graves' disease, Guillain-Barre syndrome, Hashimoto's thyroiditis, idiopathic pulmonary fibrosis, idiopathic thrombocytopenic purpura (ITP), IgA neuropathy, juvenile arthritis, oral lichen planus, lupus erythematosus, Ménière's disease, mixed connective tissue disease, multiple sclerosis, Type I or immune-mediated diabetes, myasthenia gravis, pemphigus vulgaris, pernicious anemia, polyarteritis nodosa, polychondritis, polyglandular syndrome, polymyalgia rheumatica, polymyositis and dermatomyositis, primary agammaglobulinemia, primary biliary cholangitis, psoriasis, psoriatic arthritis, Raynaud's disease, Reiter's syndrome, rheumatoid arthritis, sarcoidosis, scleroderma, Sjögren's syndrome, catalepsy, systemic lupus erythematosus, lupus erythematosus, polyarteritis, temporal arteritis/giant cell arteritis, ulcerative colitis, uveitis, and vasculitis such as dermatitis herpetiformis vasculitis, vitiligo, and Wegener's granulomatosis. Certain autoimmune diseases are associated with an inflammatory state. Therefore, there is overlap between symptoms considered to be autoimmune diseases and inflammatory diseases. Therefore, certain autoimmune diseases can also be called inflammatory diseases. As used herein, the term "costimulatory ligand" includes molecules on antigen-presenting cells (e.g., APCs, dendritic cells, and B cells) that specifically bind to associated costimulatory molecules on T cells to provide the primary signal by, for example, binding a TCR/CD3 complex to a peptide-loaded MHC molecule and provide a signal that mediates a T-cell response, which includes, but is not limited to, proliferation, activation, differentiation, etc. A costimulatory ligand may include, but is not limited to, CD7, B7-1 (CD80), B7-2 (CD86), PD-L1, PD-L2, 4-1BBL, OX40L, an inducible costimulatory ligand (ICOS-L), an intercellular adhesion molecule (ICAM), CD30L, CD40, CD70, CD83, HLA-G, MICA, MICB, HVEM, a lymphotoxin-β receptor, 3/TR6, ILT3, ILT4, HVEM, an agonist or antibody that binds to a Toll ligand receptor, and a ligand that specifically binds to B7-H3. A costimulatory ligand also includes, particularly, an antibody that specifically binds to costimulatory molecules existing on T cells, such as, but not limited to, antibodies that specifically bind to CD27, CD28, 4-1BB, OX40, CD30, CD40, PD-1, ICOS, a lymphocyte function-associated antigen-1 (LFA-1), CD2, CD7, LIGHT, NKG2C, B7-H3, and CD83.

As used herein, the term "costimulatory molecule" refers to an associated binding partner on a T cell that specifically binds to a costimulatory ligand to mediate a costimulatory response of the T cell, such as, but not limited to, proliferation, and the costimulatory molecule includes, but is not limited to, an MHC-class I molecule, and BTLA and Toll ligand receptors.

As used herein, the term "costimulatory signal" refers to a signal that binds to the primary signal for example, through TCR/CD3 ligation, to elicit T-cell proliferation and/or up-regulation or down-regulation of key molecules.

"Encoding" refers to the inherent property of specific sequences of nucleotides in a polynucleotide, such as a gene, cDNA, and mRNA, to serve as a template for synthesis of other polymers and macromolecules in the biological process, the polymers and macromolecules have any one of a defined sequence of a nucleotide (i.e., rRNA, tRNA, and mRNA) or a defined sequence of an amino acid and biological properties resulting therefrom. Therefore, the transcription and translation of mRNA corresponding to a gene produces proteins in cells or other biological systems, which means that the gene encodes proteins. Both of the coding strand whose nucleotide sequence is identical to the mRNA sequence, which is usually provided in a sequence listing, and the non-coding strand, which serves as a template for transcribing a gene or cDNA, can be referred to as encoding proteins or other products of that gene or cDNA.

As used herein, the term "endogenous" refers to any substance derived from an organism, cell, tissue or system, or produced within an organism, cell, tissue or system.

As used herein, the term "exogenous" refers to any substance introduced into an organism, cell, tissue or system, or produced outside an organism, cell, tissue or system.

"Expression" is defined as the transcription and/or translation of a specific nucleotide sequence driven by its promoter.

As used herein, the term "nucleic acid construct" refers to a vector comprising a recombinant polynucleotide that comprises an expression control sequence operably linked to a nucleotide sequence to be expressed. The nucleic acid construct comprises sufficient cis-acting elements for expressing; and other elements for expressing can be provided by host cells or provided in an expression system in vitro. The nucleic acid construct includes those known in the art, such as a cosmid, a plasmid (e.g., exposed to or comprised in a liposome), and a virus (e.g., a lentivirus, a retrovirus, an adenovirus, and an adeno-associated virus) that are incorporated into a recombinant polynucleotide.

"Homology" refers to the sequence similarity or sequence identity of two peptides or two nucleic acid molecules. When sites in two compared sequences are occupied by the same base or amino acid monomer subunit, for example, if a site in each of two DNA molecules is occupied by an adenine, the molecules are homologous at that site. Percentage of the homology of two sequences is calculated by a function of the number of matched or homologous sites co-owned by two sequences/the number of compared sites×100. For example, two sequences are 60% homologous if 6 out of 10 sites in the two sequences are matched or homologous. For example, two DNA sequences ATTGCC and TATGGC are 50% homologous. Usually, two sequences are compared to obtain the maximum homology.

As used herein, the term "immunoglobulin" or "Ig" is defined as a class of proteins that achieve an antibody effect. An antibody expressed by B cells are sometimes known as BCR (B-cell receptor) or an antigen receptor. It includes 5 members of IgA, IgG, IgM, IgD, and IgE in the class of proteins. IgA is a primary antibody existing in body secretions such as saliva, tears, breast milk, a gastrointestinal secretion, and mucinous secretions in the respiratory tract and the urogenital tract. IgG is the most common circulating antibody. IgM is a predominant immunoglobulin produced during the primary immune response of most subjects. It is the most effective immunoglobulin in agglutination, complement fixation, and other antibody responses, which plays a very important role in combating bacteria and viruses. IgD is an immunoglobulin without the known antibody function, which can be used as an antigen receptor. IgE is an immunoglobulin that mediates immediate hypersensitivity after exposure to an allergen by eliciting mediator release from mast cells and basophils.

"Isolation" refers to transformation or emigration from the natural state. For example, a nucleic acid or peptide naturally existing in a living animal is not "isolated", but the same nucleic acid or peptide that is partially or completely "isolated" from the coexisting substance in its natural state is "isolated". An isolated nucleic acid or protein can exist in a substantially purified form, or, for example, can exist in a non-natural environment such as host cells.

Unless otherwise specified, a "polynucleotide sequence encoding an amino acid sequence" includes all nucleotide sequences that are degenerate versions of each other and encode the same amino acid sequence. A phrase nucleotide sequence encoding a protein or RNA may also include introns, to the extent that the nucleotide sequence encoding the protein may include (one or more) introns in some versions.

As used herein, the term "operable linkage" refers to a functional linkage between a regulatory sequence and a heterologous nucleic acid sequence, which produces the expression of the latter. For example, if a first nucleic acid sequence is in a functional relationship with a second nucleic acid sequence, the first nucleic acid and the second nucleic acid sequence are operably linked. For example, if a promoter affects the transcription or expression of a coding sequence, the promoter is operably linked to the coding sequence. Usually, operably linked DNA sequences are contiguous, and two protein-coding regions are necessarily be linked in the same reading frame.

The term "overexpressed" tumor antigen or "overexpression" of a tumor antigen is intended to indicate the abnormal expression level of the tumor antigen in cells from a disease region such as a solid tumor in a specific tissue or organ of a patient, relative to the expression level of normal cells from a tissue or an organ. Patients with solid tumors or haematological malignancies characterized by tumor antigen overexpression can be determined by standard assays known in the art.

"Parenteral" administration of an immunogenic composition includes, such as, subcutaneous (s.c), intravenous (i.v.), intramuscular (i.m.) or intrasternal injection, or injection technologies. As used herein, the terms "patient", "subject", "individual" and the like are interchangeable, and refer to any animal or cells thereof that, whether in vitro or in situ, comply with the methods described herein. In some nonrestrictive implementation modes, a patient, subject or individual refers to human.

As used herein, the term "polynucleotide" is defined as a nucleotide chain. In addition, a nucleic acid is a polymer of nucleotides. Therefore, as used herein, the nucleic acid and the polynucleotide are interchangeable. Those skilled in the art have the general knowledge that a nucleic acid is a polynucleotide that can be hydrolyzed into monomeric "nucleotides". A monomeric nucleotide can be hydrolyzed into nucleosides. As used herein, the polynucleotide includes, but is not limited to, all nucleic acid sequences obtained by any available method in the art, and the methods include, but are not limited to, a recombination method, that is, nucleic acid sequences are cloned from a recombination library or a genome of a cell by a common cloning technology, PCR^{™} or the like, and a synthesis method.

As used herein, the terms "peptide", "polypeptide", and "protein" are interchangeable, and refer to a compound composed of amino acid residues covalently linked via peptide bonds. A protein or peptide necessarily comprises at least two amino acids, and there is no limit to the maximum number of amino acids that can include a sequence of a protein or peptide. A polypeptide includes any peptide or protein including two or more amino acids linked to each other via peptide bonds. As used herein, this term refers to a short chain that is usually known as, such as, a peptide, an oligopeptide, and an oligomer in the art; and a relative long chain that is usually known as a protein in the art and has many types. A "polypeptide" includes, such as, a biologically active fragment, substantially homologous polypeptides, oligopeptide, a homodimer, a heterodimer, a variant of a polypeptide, a modified polypeptide, a derivative, an analogue, a fusion protein, etc. A polypeptide includes a natural peptide, a recombinant peptide, a synthesized peptide, or a combination thereof.

As used herein, the term "safety switch" refers to an engineered protein that is designed to prevent potential toxicity of cell therapy or prevent adverse effects by other methods. In some cases, the expression of a safety switch protein is conditionally controlled to address safety issues of an engineered cell to be transplanted that have permanently incorporated a gene encoding the safety switch protein into a genome of the cell. Such conditional control may be variable and may include control through micromolecule-mediated post-translational activation and tissue specificity and/or on-time transcriptional control. The safety switch can mediate induction of apoptosis, inhibition of protein synthesis, DNA replication, growth retardation, transcription, and post-transcription gene control, and/or antibody-mediated depletion. In some cases, the safety switch protein is activated by an exogenous molecule (e.g., a prodrug), and triggers cells to be treated to undergo apoptosis and/or death upon activation. Embodiments of the safety switch protein include, but are not limited to, HSVTK, VZVTK, iCaspase-9, iCaspase-1, iCaspase-8, truncated EGFR, RQR8, etc. In such strategy, a prodrug administrated in the case of adverse event is activated by a suicide gene product to kill transduced cells.

As used herein, the term "promoter" is defined as a DNA sequence that is required by initiation of the specific transcription of a polynucleotide sequence, is recognized by a synthesis mechanism of cells or guides the synthesis mechanism.

As used herein, the term "promoter/regulatory sequence" refers to a nucleic acid sequence required by the expression of a gene product that can be operably linked to the promoter/regulatory sequence. In some examples, this sequence may be a core promoter sequence, and in other examples, this sequence may also comprise an enhancer sequence and other regulatory elements that are required by the expression of a gene product. A promoter/regulatory sequence may be, such as, a sequence expressing a gene product in a tissue specificity manner.

A "constitutive" promoter is a nucleotide sequence that, when operably linked to a polynucleotide encoding or specifying a gene product, enable a gene product to be produced in a cell under most or all cellular physiological conditions.

A "inducible" promoter is a nucleotide sequence that, when operably linked to a polynucleotide encoding or specifying a gene product, enable a gene product to be produced in a cell substantially only when an inducer corresponding to the promoter exists in the cell. A "tissue-specific" promoter is a nucleotide sequence that, when operably linked to a polynucleotide encoding a gene or specified by a gene, enable a gene product to be produced in a cell substantially only when the cell is corresponding to the tissue type of the promoter.

As used herein, the "specific binding" or "immunospecific binding" of an antibody or antigen-binding fragment thereof is interchangeable, and refers to the ability of the antibody or antigen-binding fragment to form one or more non-covalent bonds with an alloantigen through non-covalent interactions between the antibody and antibody-binding sites of the antigen. The antigen may be an isolated antigen or an antigen existing in a tumor cell. Usually, an antibody that immunospecifically binds (or specifically binds) to an antigen binds the antigen according to an affinity constant Ka of about 1×10⁷ M⁻¹ or 1×10⁸ M⁻¹ or above (or according to a dissociation constant (Kd) of 1×10⁻⁷ M or 1×10⁻⁸ M or below). An affinity constant can be determined by a standard kinetic method for antibody responses, such as immunoassay, surface plasmon resonance (SPR) (Rich and Myszka (2000) Curr. Opin. Biotechnol 11: 54; and Englebienne (1998) Analyst. 123: 1599), isothermal titration calorimetry (ITC), and other kinetic interaction measurement methods known in the art (referring to, such as, Paul, ed., Fundamental Immunology, 2nd ed., Raven Press, New York, pages 332-336 (1989); and referring to the U.S. patent application 7,229,619 which discloses exemplary SPR and ITC for calculating the binding affinity of an antibody). Instruments and methods for detecting and monitoring binding rates in real time are known and commercially available in the market (referring to BiaCore 2000, Biacore AB, Upsala, Sweden and GE Healthcare Life Sciences; Malmqvist (2000) Biochem. Soc. Trans. 27: 335).

As used herein, a "substantially purified" cell refer to a cell that substantially does not comprise other types of cells. A substantially purified cell also refers to a cell that has been isolated from other types of cells with which it is normally associated in its naturally occurring state. In some examples, a substantially purified cell mass refers to a homogeneous cell mass. In other examples, this term simply refers to a cell that has been isolated from other types of cells with which it is normally associated in its native state. In some embodiments, cells are cultured in vitro. In other embodiments, cells are not cultured in vitro.

As used herein, the term "therapeutic" refers to treatment and/or prevention. A therapeutic effect is achieved through disease inhibition, alleviation or eradication.

As used herein, the term "therapeutically effective amount" refers to the mount of an object compound which will elicit a biological or medical response of a tissue, a system or an object sought by a researcher, a veterinarian, a medical doctor or other clinicians. The term "therapeutically effective amount" includes the following amount of a compound: when administrated, it is sufficient to prevent the development of one or more of signs or symptoms of a disorder or disease to be treated, or alleviate one or more of signs or symptoms of the disorder or disease to be treated. The therapeutically effective amount is adjusted according to compounds, diseases and their severities, and age, weight, etc. of subjects to be treated. As used herein, the term "treatment" of a disease refers to reduction in the frequency or severity of at least one sign or symptom of a disease or disorder experienced by a subject.

As used herein, the term "transfection" or "transformation" or "transduction" refers to the process by which exogenous nucleic acids are transferred or introduced into host cells. "Transfected" or "transformed" or "transduced" cells are cells that have been transfected, transformed or transduced with exogenous nucleic acids. These cells include primary object cells and their progeny.

As used herein, the term "vector" is a substance composition that comprises isolated nucleic acids and can be used to transfer the isolated nucleic acids into cells. Many vectors are known in the art, which include, but are not limited to, a linear polynucleotide, a polynucleotide associated with an ionic or amphiphilic compound, a plasmid, and a virus. Therefore, the term "vector" includes an autonomously replicating plasmid or virus. This term should also be construed to include a non-plasmid and non-viral compound that facilitates transfer of nucleic acids into cells, such as a poly-lysine compound and a liposome. Examples of the viral vector include, but not are limited to, an adenoviral vector, an adeno-associated viral vector, a retroviral vector, etc.

### II. Specific embodiments

In one aspect, the present disclosure provides a B7-H3-targeted chimeric antigen receptor, and immune effector cells expressing the B7-H3-targeted chimeric antigen receptor; and specifically, the immune effector cells expressing the B7-H3-targeted chimeric antigen receptor are T cells expressing the B7-H3-targeted chimeric antigen receptor.

In one aspect, the B7-H3-targeted chimeric antigen receptor (CAR) of the present disclosure comprises an anti-B7-H3 binding domain, a hinge region, a transmembrane domain, and a signal transduction domain.

In the CAR according to any one of the preceding aspects, the anti-B7-H3 binding domain comprises an anti-B7-H3 antibody or antigen-binding portion, and the antibody or antigen-binding portion comprises a light-chain CDR selected from amino acid sequences of SEQ ID NO: 5-7, 17-19, 29-31, 41-43, 53-55, 65-67, 77-79, 89-91, 101-103, 113-115 or any variant thereof; and/or, comprises a heavy-chain CDR selected from amino acid sequences of SEQ ID NO: 10-12, 22-24, 34-36, 46-48, 58-60, 70-72, 82-84, 94-96, 106-108, 118-120 or any variant thereof.

In the CAR according to any one of the preceding aspects, the anti-B7-H3 binding domain comprises an anti-B7-H3 antibody or antigen-binding portion, and the antibody or antigen-binding portion comprises a light-chain CDR1 selected from amino acid sequences of SEQ ID NO: 5, 17, 29, 41, 53, 65, 77, 89, 101, 113 or any variant thereof, a light-chain CDR2 selected from amino acid sequences of SEQ ID NO: 6, 18, 30, 42, 54, 66, 78, 90, 102, 114 or any variant thereof, and a light-chain CDR3 selected from amino acid sequences of SEQ ID NO: 7, 19, 31, 43, 55, 67, 79, 91, 103, 115 or any variant thereof; and/or, comprises a heavy-chain CDR1 selected from amino acid sequences of SEQ ID NO: 10, 22, 34, 46, 58, 70, 82, 94, 106, 118 or any variant thereof, a heavy-chain CDR2 selected from amino acid sequences of SEQ ID NO: 11, 23, 35, 47, 59, 71, 83, 95, 107, 119 or any variant thereof, and a heavy-chain CDR3 selected from amino acid sequences of SEQ ID NO: 12, 24, 36, 48, 60, 72, 84, 96, 108, 120 or any variant thereof.

In the CAR according to any one of the preceding aspects, the anti-B7-H3 binding domain comprises an anti-B7-H3 antibody or antigen-binding portion, and the antibody or antigen-binding portion comprises a light chain sequence selected from amino acid sequences of SEQ ID NO: 4, 16, 28, 40, 52, 64, 76, 88, 100, 112 or any variant thereof; and/or, comprises a heavy chain sequence selected from amino acid sequences of SEQ ID NO: 9, 21, 33, 45, 57, 69, 81, 93, 105, 117 or any variant thereof.

In the CAR according to any one of the preceding aspects, the anti-B7-H3 binding domain comprises an anti-B7-H3 single-chain antibody (scFv).

In the CAR according to any one of the preceding aspects, the anti-B7-H3 single-chain antibody is derived from a monoclonal antibody produced against a B7-H3 protein fragment.

In the CAR according to any one of the preceding aspects, the anti-B7-H3 single-chain antibody has an amino acid sequence selected from amino acid sequences of SEQ ID NO: 2, 14, 26, 38, 50, 62, 74, 86, 98, 110 or any variant thereof.

In one aspect, the anti-B7-H3 binding domain and the anti-B7-H3 single-chain antibody part according to any one of the preceding aspects have at least more than 60%, 65%, 70%, 75%, 80%, 85%, 90%, 95%, 96%, 97%, 98%, 99% or preceding sequence identity.

In the CAR according to any one of the preceding aspects, the transmembrane domain is selected from one or more transmembrane domains of α, β, ζ chains of TCR, CD3y, CD3δ, CD3ε, CD3ζ, CD4, CD5, CD8α, CD9, CD16, CD22, CD27, CD28, CD33, CD37, CD45, CD64, CD80, CD86, CD134, 4-1BB, CD152, CD154, PD-1; preferably, the transmembrane domain is selected from one or more transmembrane domains of CD8α, CD4, CD45, PD-1, CD154, CD28; more preferably, the transmembrane domain is selected from one or more transmembrane domains of CD8α or CD28; more preferably, the transmembrane domain has an amino acid sequence selected from amino acid sequence of SEQ ID NO: 124 or any variant thereof.

In the CAR according to any one of the preceding aspects, the hinge region is selected from one or more of an extracellular hinge region of CD8, a hinge region of IgG1 Fc CH2CH3, a hinge region of IgD, an extracellular hinge region of CD28, a hinge region of IgG4 Fc CH2CH3, and an extracellular hinge region of CD4; preferably, the hinge region is a hinge region of CD8α; more preferably, the hinge region has an amino acid sequence selected from amino acid sequences of SEQ ID NO: 122 or any variant thereof.

In the CAR according to any one of the preceding aspects, the signal transduction domain is selected from one or more intracellular signaling regions of TCRζ, FcRy, FcRβ, CD3γ, CD3δ, CD3ε, CD5, CD22, CD79a, CD79b, CD278 (ICOS), CD66d, CD3ζ; preferably, the signal transduction domain is selected from an intracellular signaling region of CD3ζ; more preferably, the signal transduction domain has an amino acid sequence selected from amino acid sequences of SEQ ID NO: 130 or any variant thereof.

In the CAR according to any one of the preceding aspects, the signal transduction domain further comprises one or more costimulatory domains; preferably, the costimulatory domain is selected from one or more intracellular signaling regions of CARD11, CD2, CD7, CD27, CD28, CD30, CD40, CD54, CD83, OX40, 4-1BB, CD134, CD150, CD152, CD223, CD270, PD-L2, PD-L1, CD278, DAP10, LAT, NKD2C, SLP76, TRIM, FcεRIγ, MyD88, 41BBL; more preferably, the costimulatory domain is selected from intracellular signaling regions of 4-1BB, CD134, CD28, OX40; more preferably, the costimulatory domain is selected from one or more intracellular signaling regions of 4-1BB or CD28; more preferably, the costimulatory domain has an amino acid sequence selected from amino acid sequences of SEQ ID NO: 126, 128 or any variant thereof.

In another aspect, the present disclosure also provides a B7-H3-targeted single-chain antibody (scFv) domain; specifically, the scFv is derived from a monoclonal antibody produced against a B7-H3 protein fragment; more specifically, the scFv has an amino acid sequence selected from amino acid sequences of SEQ ID NO: 2, 14, 26, 38, 50, 62, 74, 86, 98, 110 or any variant thereof.

In one aspect, an encoding gene of the B7-H3-targeted scFv comprises a nucleotide sequence selected from nucleotide sequences of SEQ ID NO: 1, 13, 25, 37, 49, 61, 73, 85, 97, 109 or any variant thereof.

In one aspect, the present disclosure provides a nucleic acid molecule encoding the CAR or scFv according to any one of the preceding aspects; preferably, the nucleic acid molecule encoding the scFv has a nucleotide sequence selected from nucleotide sequences of SEQ ID NO: 1, 13, 25, 37, 49, 61, 73, 85, 97, 109 or a nucleic acid molecule with at least more than 60%, 65%, 70%, 75%, 80%, 85%, 90%, 95%, 96%, 97%, 98%, 99% or preceding sequence identity.

In one aspect, the present disclosure provides a nucleic acid construct, comprising the nucleic acid molecule according to any one of the preceding aspects; preferably, the nucleic acid construct is a viral vector; more preferably, the viral vector is selected from one or more of a retroviral vector, a lentiviral vector, an adenoviral vector.

In one aspect, the present disclosure provides a virus, comprising the nucleic acid molecule according to any one of the preceding aspects, or comprising the nucleic acid construct according to any one of the preceding aspects; and preferably, the virus is selected from one or more of a retrovirus, a lentivirus, an adenovirus, an adeno-associated virus.

In one aspect, the present disclosure provides an isolated host cell, expressing the CAR according to any one of the preceding aspects, or comprising the nucleic acid construct according to any one of the preceding aspects, or comprising the virus according to any one of the preceding aspects; preferably, the host cell is a mammalian cell; more preferably, the host cell is a T cell, an NK cell or cell line, a γδT cell, an NKT cell, a macrophage or cell line, a PG13 cell line; more preferably, the host cell is a T cell; the most preferably, the host cell is a primarily cultured T cell.

In one aspect, the host cell also expresses other effector molecules that include, but are not limited to, a cytokine, another CAR, a chemokine receptor, an siRNA reducing the expression of PD-1 or a protein blocking PD-L1, a TCR or a safety switch; preferably, the cytokine is selected from one or more of TNF-α, TNF-β, VEGF, TPO, NGF-β, PDGF, TGF-α, TGF-β, IGF-I, IGF-II, EPO, M-CSF, IL-1, IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, an interferon, an angiostatin, a thrombospondin, an endostatin; more preferably, the chemokine receptor is selected from one or more of CCR2, CCR5, CXCR2, CXCR4; preferably, the safety switch is selected from one or more of HSVTK, VZVTK, iCaspase-9, iCaspase-1, iCaspase-8, truncated EGFR, RQR8.

In one aspect, the present disclosure provides an immune effector cell expressing the B7-H3-targeted chimeric antigen receptor; specifically, the present disclosure provides a T cell expressing the B7-H3-targeted chimeric antigen receptor (CAR-T); more specifically, the T cell is an activated T cell; more specifically, the activation of the T cell is completed by stimulation with an CD3 and/or CD28 antibodies.

In one aspect, the present disclosure provides a pharmaceutical composition, comprising one or more of the CAR, the nucleic acid molecule, the nucleic acid construct, the virus, the cell according to any one of the preceding aspects, and a pharmaceutically acceptable carrier.

It should be understood that the therapeutic agent of the present disclosure will be administrated together with an appropriate pharmaceutically acceptable carrier, an excipient, and other reagents that are incorporated into the formulation to provide improved transfer, delivery, tolerability, etc. A large number of suitable formulations can be found in the pharmacopoeia known to all pharmaceutical chemists: Remington's Pharmaceutical Science (15th edition, Mack Publishing Company, Easton, Pa. (1975)), especially Chapter 87 of Blaug, Seymour therein. These formulations include, for example, powders, pastes, ointments, gels, waxes, oils, lipids, lipid-comprising (cation or anion) carriers (e.g., Lipofectin^{™}), DNA conjugates, anhydrous slurries, oil-in-water and water-in-oil emulsions, emulsion polyethylene glycols (polyethylene glycols of various molecular weights), semi-solid gels, and semi-solid mixtures comprising polyethylene glycols. Any above mixture can be applicable to the treatment or therapy of the present disclosure under the condition that active ingredients in the formulation will be not inactivated by the formulation and the formulation is physiologically compatible and tolerant during administration.

As used herein, the term "pharmaceutically acceptable carrier" is intended to include any and all solvents, dispersion media, coatings, antibacterial agents and antifungal agents, isotonic agents and absorption delaying agents, etc. that are compatible with pharmaceutical administration. Appropriate pharmaceutically acceptable carriers are described in the latest edition of Remington's Pharmaceutical Sciences, which is a standard reference in the art and is incorporated herein by reference. Preferred examples of these carriers or diluents include, but are not limited to, water, saline, a Ringer's solution, a glucose solution, and 1-10% human serum albumin. Liposomes and non-aqueous carriers, such as fixed oils, can also be used. The use of these media and reagents for pharmaceutically active substances is well known in the art. In addition to any conventional media or reagents that are incompatible with the antibody, its use in compositions is contemplated.

Further, the pharmaceutical composition of the present disclosure can be used to treat inflammatory factor-associated diseases such as cancer, inflammatory diseases, autoimmune diseases, etc.

In one aspect, the CAR, the nucleic acid molecule, the nucleic acid construct, the virus, the cell, and the pharmaceutical composition according to any one of the preceding aspects are used to prepare a drug for diagnosing, treating or preventing cancer.

In one embodiment, the drug can be used as a therapeutic agent. Such reagent is usually used to diagnose, treat, relieve and/or prevent diseases or pathology associated with abnormal B7-H3 expression, activity and/or signal transduction of a subject. A subject, such as a human patient suffering from (or at risk of or developing) a disease or disorder associated with abnormal B7-H3 expression, activity and/or signal transduction, such as cancer or other neoplastic disorders, can be identified by a standard method and applied with a therapeutic regimen. An antibody or antibody fragment that specifically binds to a target antigen (e.g., B7-H3) is used as the chimeric antigen receptor (CAR) of the present disclosure, preferably, an antibody with high specificity and high affinity to its target antigen (e.g., B7-H3) is prepared into the CAR of the present invention, and preferably, further, the prepared CAR is used to genetically modify immune cells (e.g., T cells). The expression, activity and/or signal transduction function of a target (e.g., B7-H3) can be eliminated, inhibited or hindered by administrating the CAR and/or genetically modified immune cells of the present disclosure. The binding of a target (e.g., B7-H3) to its naturally bound endogenous ligand can be eliminated or inhibited or hindered by administrating the CAR and/or genetically modified immune cells of the present disclosure. For example, the B7-H3 expression, activity and/or signal transduction can be regulated, blocked, inhibited, reduced, antagonized, neutralized, or hindered in other manners by administrating the CAR and/or genetically modified immune cells of the present disclosure. In some embodiments, in order to treat a disease or disorder associated with abnormal B7-H3 expression, an anti-B7-H3 antibody heavy-chain and light-chain CDR antibody or antibody fragment can be prepared into the CAR and/or genetically modified immune cells of the present disclosure, and then the CAR and/or genetically modified immune cells are administered to a subject. In one embodiment, the disease or disorder associated with abnormal B7-H3 expression may be cancer.

In order to clearly and concisely describe the present disclosure, features are described herein as part of the same or separate embodiments. However, it will be understood that the scope of the present disclosure may include embodiments comprising combinations of all or some of the described features.

### Examples

### Example 1: detection of the expression of B7-H3 in tumors

In order to determine the expression levels of the antigen B7-H3 in tumors, we first selected different tumor cell lines for detection. Meanwhile, to further verify the expression of B7-H3 in primary tumors, we detected B7-H3 expression levels in sections of brain glioma tissues and normal tissues of multiple patients.

Method: tumor cells were collected, washed with 1x PBS three times, stained with an B7-H3 antibody 15C11 on ices for 30 min, washed with 1x PBS, added with an APC-conjugated goat-anti-mouse secondary antibody, incubated at room temperature for 20 min, and washed with 1x PBS, and the expression of B7-H3 on tumor cells was determined by using flow cytometry.

Immunohistochemistry staining: the expression of B7-H3 in primary tumors was detected by immunohistochemistry staining the sections of brain glioma tissues and normal tissues of multiple patients with the 15C11 antibodies.

The paraffin sections were deparaffinized with dimethylbenzene and rewatered with ethyl alcohol, and antigens were repaired with sodium citrate. Then, the sections were blocked in serums for 1 h. The sections were stained with the B7-H3 antibodies 15C11 at 4°C overnight, and then incubated with horseradish peroxidase-conjugated goat-anti-mouse secondary antibodies for 30 min. The sections were washed, developed with DAB substrates, and then stained with hematoxylin. After being stained, the sections were observed and photographed under a microscope.

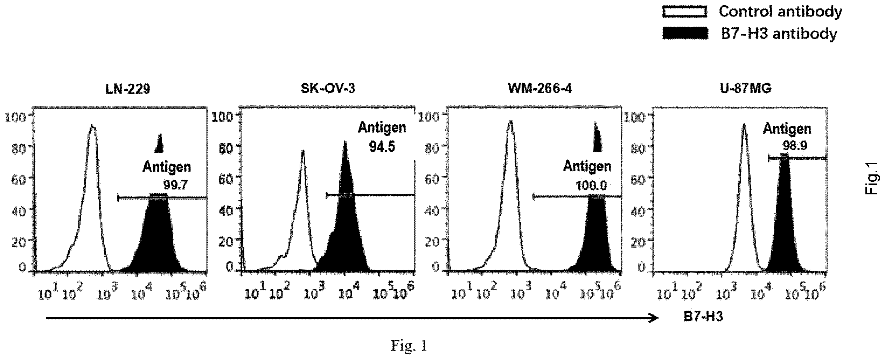

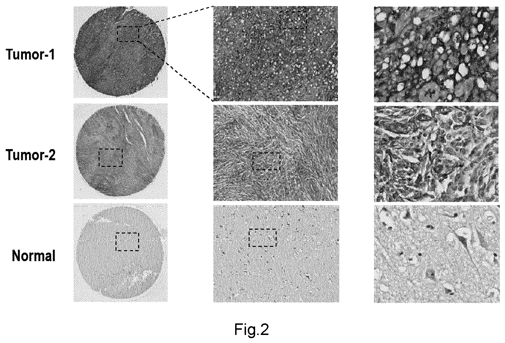

Results: As shown in Fig. 1, B7-H3 is highly expressed in the detected cell lines of several types of tumors, such as skin cancer (WM-266-4), ovarian cancer (SK-OV-3) and glioma (LN-229, U-87MG). Fig. 2 showed that in the glioma samples of all patients, different levels of B7-H3 expression is detected, and in normal tissues adjacent to the tumor and normal brain tissues, B7-H3 is only expressed in a few cells at very low levels. It indicates that B7-H3 could be a specific target for solid tumor therapy.

### Example 2: cell culture

Cell lines used in the present disclosure, including 293T cells, were cultured in IMDM media comprising 10% FBS, 1% GlutaMAX, and 1% two types of antibiotics.

Cell culture media for WM-266-4, SK-OV-3, LN-229, LN-18, U-87MG were RPMI640 or DMEM with 10% FBS.

A retrovirus encoding GFP was prepared by transiently transfecting 293T cells with a three-plasmid system (GFS-GFP, MMLV-gag-pol, and pVSV-G). GFP-positive cell lines were prepared by infecting with the retrovirus encoding GFP. A lentivirus encoding Luciferase (Luc) was prepared by transiently transfecting 293T cells with Lenti-Luciferase, pspAX2, and pVSV-G, and a retrovirus encoding GFP-firefly luciferase (GFP-FFLuc) was prepared by transiently transfecting 293T cells with GFS-GFP-FFLuc, MMLV-gag-pol, and pVSV-G. Tumor cell lines for mice were prepared by infecting cells with the lentivirus encoding Luc or the retrovirus encoding GFP-FFLuc.

### Example 3: construction of chimeric antigen receptor

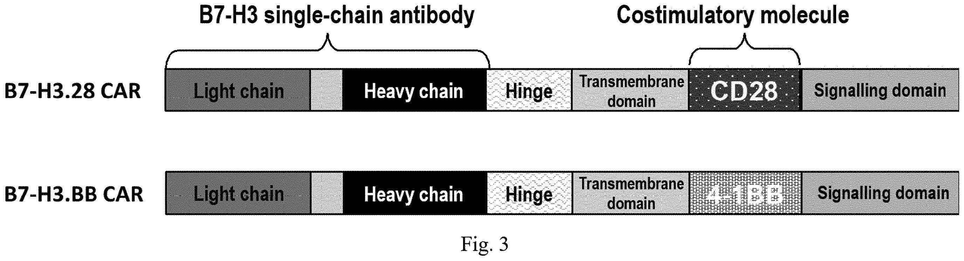

A chimeric antigen receptor was composed of a single-chain antibody domain, a CD8α hinge region, a CD8α transmembrane domain, a CD28 or 4-1 BB costimulatory factor domain, and a CD3ζ signal transduction domain (as shown in Fig. 3). The single-chain antibody (scFv) domain of the chimeric antigen receptors were derived from antibodies against B7-H3 that were produced by different monoclonal hybridoma cell clones, with the amino acid sequences as shown in SEQ ID NO: 2, 14, 26, 38, 50, 62, 74, 86, 98 or 110. The chimeric antigen receptors were generated by gene synthesis and then cloned into a retroviral vector. The correctness of the sequences of the chimeric antigen receptors were confirmed by sequencing.

**Table 1 Sequence listing**

| |
|---|
| SEQ ID NO. 1: a nucleic acid sequence of a 15C11 single-chain antibody region |
| ATGGAGTTTGGGCTGAGCTGGCTTTTTCTTGTGGCTATTTTAAAAGGTGTCCAGTGCTCT AGAGACATCCAGATGACACAGAGCCCTAGCAGCCTGTCTGCCAGCGTGGGAGACAGAG TGACCATCACCTGTCAGGCCTCTCAGAACGTGCGGACAGCCGTGGCTTGGTATCAGCA GAAGCCTGGCAAGGCCCCTAAGGCTCTGATCTACCTGCCTAGCAACAGACTGACCGGC GTGCCCTCTAGATTCAGCGGCTCTGGCTCTGGCACCGACTTCACCTTCACAATCAGCAG CCTGCAGCCTGAGGATATCGCTACCTACTTCTGCCTGCAACACTGGAACTACCCTTTCA CCTTCGGCCAGGGCACCAAGCTGGAAATCAAGGGTGGTGGTGGTTCTGGTGGTGGTG GTTCTGGCGGCGGCGGCTCCGGTGGTGGTGGATCCGAGGTTCAGCTGCAGCAGTCTG GACCTGAGCTGGTTAAGCCTGGCGCCTCCGTGAAGATCCCTTGCAAGACAAGCGGCTA CATCTTCACCAGCTACTGGATCCACTGGGTCAAGCAGAGCCACGGCAAGAGCCTGGAA TGGATCGGCAGAATCTACCCCGGCGTGGACAGCATCTACTACAACGAGAAGTTCAAGG GCAAAGCCACACTGACCGCCGACAAGAGCAGCAGCACAGCCTACATGGAACTGAGAAG CCTGACCAGCGAGGATACCGCCGTGTACTTCTGTGCCACCATCAGCTACGACTACGATT ACAGCATGGACTACTGGGGCCAGGGCACCACCGTGACAGTTTCTTCT |
| SEQ ID NO. 2: a protein sequence of the 15C11 single-chain antibody region |
| MEFGLSWLFLVAILKGVQCSRDIQMTQSPSSLSASVGDRVTITCQASQNVRTAVAWYQQKP GKAPKALIYLPSNRLTGVPSRFSGSGSGTDFTFTISSLQPEDIATYFCLQHWNYPFTFGQGT KLEIKGGGGSGGGGSGGGGSGGGGSEVQLQQSGPELVKPGASVKIPCKTSGYIFTSYWIH WVKQSHGKSLEWIGRIYPGVDSIYYNEKFKGKATLTADKSSSTAYMELRSLTSEDTAVYFCAT ISYDYDYSMDYWGQGTTVTVSS |
| SEQ ID NO. 3: a nucleic acid sequence of a light chain of the 15C11 single-chain antibody |
| GACATCCAGATGACACAGAGCCCTAGCAGCCTGTCTGCCAGCGTGGGAGACAGAGTGA CCATCACCTGTCAGGCCTCTCAGAACGTGCGGACAGCCGTGGCTTGGTATCAGCAGAA GCCTGGCAAGGCCCCTAAGGCTCTGATCTACCTGCCTAGCAACAGACTGACCGGCGTG CCCTCTAGATTCAGCGGCTCTGGCTCTGGCACCGACTTCACCTTCACAATCAGCAGCCT GCAGCCTGAGGATATCGCTACCTACTTCTGCCTGCAACACTGGAACTACCCTTTCACCTT CGGCCAGGGCACCAAGCTGGAAATCAAG |
| SEQ ID NO. 4: a protein sequence of the light chain of the 15C11 single-chain antibody |
| DIQMTQSPSSLSASVGDRVTITCQASQNVRTAVAWYQQKPGKAPKALIYLPSNRLTGVPSRF SGSGSGTDFTFTISSLQPEDIATYFCLQHWNYPFTFGQGTKLEIK |
| SEQ ID NO. 5: a protein sequence of CDR1 of the light chain of the 15C11 single-chain antibody QASQNVRTAVA |
| SEQ ID NO. 6: a protein sequence of CDR2 of the light chain of the 15C11 single-chain antibody LPSNRLT |
| SEQ ID NO. 7: a protein sequence of CDR3 of the light chain of the 15C11 single-chain antibody LQHWNYPFT |
| SEQ ID NO. 8: a nucleic acid sequence of a heavy chain of the 15C11 single-chain antibody |
| GAGGTTCAGCTGCAGCAGTCTGGACCTGAGCTGGTTAAGCCTGGCGCCTCCGTGAAGA TCCCTTGCAAGACAAGCGGCTACATCTTCACCAGCTACTGGATCCACTGGGTCAAGCAG AGCCACGGCAAGAGCCTGGAATGGATCGGCAGAATCTACCCCGGCGTGGACAGCATCT ACTACAACGAGAAGTTCAAGGGCAAAGCCACACTGACCGCCGACAAGAGCAGCAGCAC AGCCTACATGGAACTGAGAAGCCTGACCAGCGAGGATACCGCCGTGTACTTCTGTGCC ACCATCAGCTACGACTACGATTACAGCATGGACTACTGGGGCCAGGGCACCACCGTGAC AGTTTCTTCT |
| SEQ ID NO. 9: a protein sequence of a heavy chain of the 15C11 single-chain antibody |
| QLQQSGPELVKPGASVKIPCKTSGYIFTSYWIHWVKQSHGKSLEWIGRIYPGVDSIYYNEKF KGKATLTADKSSSTAYMELRSLTSEDTAVYFCATISYDYDYSMDYWGQGTTVTVSS |
| SEQ ID NO. 10: a protein sequence of CDR1 of the heavy chain of the 15C11 single-chain antibody GYIFTSYWIH |
| SEQ ID NO. 11: a protein sequence of CDR2 of the heavy chain of the 15C11 single-chain antibody RIYPGVDSIYYNEKFKG |
| SEQ ID NO. 12: a protein sequence of CDR3 of the heavy chain of the 15C11 single-chain antibody ATISYDYDYSMDY |
| SEQ ID NO. 13: a nucleic acid sequence of a 53E9D7 single-chain antibody |
| ATGGAGTTTGGGCTGAGCTGGCTTTTTCTTGTGGCTATTTTAAAAGGTGTCCAGTGCTCT AGAGATGTTGTGCTAACTCAGTCTCCAGCCACCCTGTCTGTGACTCCAGGAGATAGCGT CAGTCTTTCCTGCAGGGCCAGCCAAAGTATTAGCATCAACCTACACTGGTATCAACAAAA ATCACATGAGTCTCCAAGGCTTCTCATTAAGTATGCTTCCCAGTCCATCTCTGGGATCCC CTCCAGGTTCAGTGGCAGTGGATCAGGGACAGATTTCACTCTCAGTATCCACAGTGTGG AGACTGAAGATTTTGGAATGTTTTTCTGTCAACACAGTAACAGCTGGCCTCTCACGTTCG GTGCTGGGACCAAGCTGGAAATCAAAGGTGGTGGTGGTTCTGGTGGTGGTGGTTCTGG CGGCGGCGGCTCCGGTGGTGGTGGATCCCAGGTTACTCTGAAAGAGTCTGGCCCTGG GATATTGCAGCCCTCCCAGACCCTCAGTCTGACTTGTTCTTTCTCTGGGTTTTCACTGAG CACTTCTGGTATGGGTGTTGGCTGGATTCGTCAGCCATCACGGAAGGGTCTGGAGTGG CTGGCACACATTTGGTGGGATGATGTCAAGCGCTATAACCCAGCCCTGAAGAGCCGACT GACTATCTCCAAGGATACCTCCAGCAGCCAGGTGTTCCTCAAGATCGCCAGTGTGGACA CTGCGGATACTGCCACATACTACTGTGCTCGAATACCCCACTGGTACTTCGATGTCTGGG GCGCAGGGACCACGGTCACCGTCTCCTCA |
| SEQ ID NO. 14: a protein sequence of the 53E9D7 single-chain antibody |
| MEFGLSWLFLVAILKGVQCSRDVVLTQSPATLSVTPGDSVSLSCRASQSISINLHWYQQKSH ESPRLLIKYASQSISGIPSRFSGSGSGTDFTLSIHSVETEDFGMFFCQHSNSWPLTFGAGTKL EIKGGGGSGGGGSGGGGSGGGGSQVTLKESGPGILQPSQTLSLTCSFSGFSLSTSGMGV GWIRQPSRKGLEWLAHIWWDDVKRYNPALKSRLTISKDTSSSQVFLKIASVDTADTATYYCA RIPHWYFDVWGAGTTVTVSS |
| GATGTTGTGCTAACTCAGTCTCCAGCCACCCTGTCTGTGACTCCAGGAGATAGCGTCAG TCTTTCCTGCAGGGCCAGCCAAAGTATTAGCATCAACCTACACTGGTATCAACAAAAATC ACATGAGTCTCCAAGGCTTCTCATTAAGTATGCTTCCCAGTCCATCTCTGGGATCCCCTC CAGGTTCAGTGGCAGTGGATCAGGGACAGATTTCACTCTCAGTATCCACAGTGTGGAGA CTGAAGATTTTGGAATGTTTTTCTGTCAACACAGTAACAGCTGGCCTCTCACGTTCGGTG CTGGGACCAAGCTGGAAATCAAA SEQ ID NO. 15: a nucleic acid sequence of a light chain of the 53E9D7 single-chain antibody |
| SEQ ID NO. 16: a protein sequence of the light chain of the 53E9D7 single-chain antibody |
| DVVLTQSPATLSVTPGDSVSLSCRASQSISINLHWYQQKSHESPRLLIKYASQSISGIPSRFS GSGSGTDFTLSIHSVETEDFGMFFCQHSNSWPLTFGAGTKLEIK |
| SEQ ID NO. 17: a protein sequence of CDR1 of the light chain of the 53E9D7 single-chain antibody RASQSISINLH |
| SEQ ID NO. 18: a protein sequence of CDR2 of the light chain of the 53E9D7 single-chain antibody YASQSIS |
| SEQ ID NO. 19: a protein sequence of CDR3 of the light chain of the 53E9D7 single-chain antibody QHSNSWPLT |
| SEQ ID NO. 20: a nucleic acid sequence of a heavy chain of the 53E9D7 single-chain antibody |
| CAGGTTACTCTGAAAGAGTCTGGCCCTGGGATATTGCAGCCCTCCCAGACCCTCAGTCT GACTTGTTCTTTCTCTGGGTTTTCACTGAGCACTTCTGGTATGGGTGTTGGCTGGATTCG TCAGCCATCACGGAAGGGTCTGGAGTGGCTGGCACACATTTGGTGGGATGATGTCAAG CGCTATAACCCAGCCCTGAAGAGCCGACTGACTATCTCCAAGGATACCTCCAGCAGCCA GGTGTTCCTCAAGATCGCCAGTGTGGACACTGCGGATACTGCCACATACTACTGTGCTC GAATACCCCACTGGTACTTCGATGTCTGGGGCGCAGGGACCACGGTCACCGTCTCCTC A |
| SEQ ID NO. 21: a protein sequence of the heavy chain of the 53E9D7 single-chain antibody |
| QVTLKESGPGILQPSQTLSLTCSFSGFSLSTSGMGVGWIRQPSRKGLEWLAHIWWDDVKR YNPALKSRLTISKDTSSSQVFLKIASVDTADTATYYCARIPHWYFDVWGAGTTVTVSS |
| SEQ ID NO. 22: a protein sequence of CDR1 of the heavy chain of the 53E9D7 single-chain antibody GFSLSTSGMGVG |
| SEQ ID NO. 23: a protein sequence of CDR2 of the heavy chain of the 53E9D7 single-chain antibody HIWWDDVKRYNPALKS |
| SEQ ID NO. 24: a protein sequence of CDR3 of the heavy chain of the 53E9D7 single-chain antibody ARIPHWYFDV |
| SEQ ID NO. 25: a nucleic acid sequence of a 15C11C3 single-chain antibody |
| ATGGAGTTTGGGCTGAGCTGGCTTTTTCTTGTGGCTATTTTAAAAGGTGTCCAGTGCTCT AGAGACATTGTGATGACCCAGTCTCAAAAATTCATGTCCACATCAGTAGGAGACAGGGT CAGCATCACCTGCAAGGCCAGTCAGAATGTTCGTACTGCTGTAGCCTGGTATCAACAGA AACCAGGCCAGTCTCCTAAAGCACTGATTTACTTGCCATCCAACCGGCTCACTGGAGTC CCTGATCGCTTCACAGGCAGTGGATCTGGGACAGATTTCACTCTCACCATTAGCAATGT CCAATCTGAAGACCTGGCAGATTATTTCTGTCTGCAACATTGGAATTATCCATTCACGTTC GGCTCGGGGACAAAGTTGGAAATAAAAGGTGGTGGTGGTTCTGGTGGTGGTGGTTCTG GCGGCGGCGGCTCCGGTGGTGGTGGATCCCAGGTCCAGCTGAAGCAGTCTGGAGCTG AGCTGGTGAGGCCTGGGGCTTCAGTGAATCTGTCCTGCAAGACTTCTGGATACATCTTC ACCAGCTACTGGATTCACTGGGTAAAACAGAGGTCTGGACAGGGCCTTGAGTGGATTG CAAGGATTTATCCTGGAGTTGATAGTATTTATTACAATGAGAAGTTCAAGGGCAAGGCCA CACTGACTGCAGACAAATCCTCCAGCACTGCCTACATGCAGCTCAGCAGCCTGAAATCT GAGGACTCTGCTGTCTATTTCTGTGCAACTATCTCCTATGATTACGACTATTCTATGGACT ACTGGGGTCAAGGAACCTCAGTCACCGTCTCCTCA |
| SEQ ID NO. 26: a protein sequence of the 15C11C3 single-chain antibody |
| MEFGLSWLFLVAILKGVQCSRDIVMTQSQKFMSTSVGDRVSITCKASQNVRTAVAWYQQKP GQSPKALIYLPSNRLTGVPDRFTGSGSGTDFTLTISNVQSEDLADYFCLQHWNYPFTFGSG TKLEIKGGGGSGGGGSGGGGSGGGGSQVQLKQSGAELVRPGASVNLSCKTSGYIFTSYWI HWVKQRSGQGLEWIARIYPGVDSIYYNEKFKGKATLTADKSSSTAYMQLSSLKSEDSAVYFC ATISYDYDYSMDYWGQGTSVTVSS |
| SEQ ID NO. 27: a nucleic acid sequence of the 15C11C3 single-chain antibody |
| GACATTGTGATGACCCAGTCTCAAAAATTCATGTCCACATCAGTAGGAGACAGGGTCAG CATCACCTGCAAGGCCAGTCAGAATGTTCGTACTGCTGTAGCCTGGTATCAACAGAAAC CAGGCCAGTCTCCTAAAGCACTGATTTACTTGCCATCCAACCGGCTCACTGGAGTCCCT GATCGCTTCACAGGCAGTGGATCTGGGACAGATTTCACTCTCACCATTAGCAATGTCCAA |
| TCTGAAGACCTGGCAGATTATTTCTGTCTGCAACATTGGAATTATCCATTCACGTTCGGCT CGGGGACAAAGTTGGAAATAAAA |
| SEQ ID NO. 28: a protein sequence of a light chain of the 15C11C3 single-chain antibody |
| DIVMTQSQKFMSTSVGDRVSITCKASQNVRTAVAWYQQKPGQSPKALIYLPSNRLTGVPDR FTGSGSGTDFTLTISNVQSEDLADYFCLQHWNYPFTFGSGTKLEIK |
| SEQ ID NO. 29: a protein sequence of CDR1 of the light chain of the 15C11C3 single-chain antibody KASQNVRTAVA |
| SEQ ID NO. 30: a protein sequence of CDR2 of the light chain of the 15C11C3 single-chain antibody LPSNRLT |
| SEQ ID NO. 31: a protein sequence of CDR3 of the light chain of the 15C11C3 single-chain antibody LQHWNYPFT |
| SEQ ID NO. 32: a nucleic acid sequence of a heavy chain of the 15C11C3 single-chain antibody |
| CAGGTCCAGCTGAAGCAGTCTGGAGCTGAGCTGGTGAGGCCTGGGGCTTCAGTGAAT CTGTCCTGCAAGACTTCTGGATACATCTTCACCAGCTACTGGATTCACTGGGTAAAACAG AGGTCTGGACAGGGCCTTGAGTGGATTGCAAGGATTTATCCTGGAGTTGATAGTATTTAT TACAATGAGAAGTTCAAGGGCAAGGCCACACTGACTGCAGACAAATCCTCCAGCACTGC CTACATGCAGCTCAGCAGCCTGAAATCTGAGGACTCTGCTGTCTATTTCTGTGCAACTAT CTCCTATGATTACGACTATTCTATGGACTACTGGGGTCAAGGAACCTCAGTCACCGTCTC CTCA |
| SEQ ID NO. 33: a protein sequence of the heavy chain of the 15C11C3 single-chain antibody |
| QVQLKQSGAELVRPGASVNLSCKTSGYIFTSYWIHWVKQRSGQGLEWIARIYPGVDSIYYN EKFKGKATLTADKSSSTAYMQLSSLKSEDSAVYFCATISYDYDYSMDYWGQGTSVTVSS |
| SEQ ID NO. 34: a protein sequence of CDR1 of the heavy chain of the 15C11C3 single-chain antibody GYIFTSYWIH |
| SEQ ID NO. 35: a protein sequence of CDR2 of the heavy chain of the 15C11C3 single-chain antibody RIYPGVDSIYYNEKFKG |
| SEQ ID NO. 36: a protein sequence of CDR3 of the heavy chain of the 15C11C3 single-chain antibody ATISYDYDYSMDY |
| SEQ ID NO. 37: a nucleic acid sequence of a 6E4D9 single-chain antibody |
| ATGGAGTTTGGGCTGAGCTGGCTTTTTCTTGTGGCTATTTTAAAAGGTGTCCAGTGCTCT AGACAAATTGTTCTCACCCAGTCTCCAGCAATCATGTCTGCATCTCCAGGGGAGAAGGT CACCATGACCTGCAGTGCCAGCTCAAGTGTAAGTTACATGCACTGGTACCAGCAGAAGT CAGGCACCTCCCCCAAAAGATGGATTTATGACACATCCAAACTGGCTTCTGGAGTCCCT GCTCGCTTCAGTGGCAGTGGGTCTGGGACCTCTTACTCTCTCACAATCAGCAGCATGGA GGCTGAAGATGCTGCCACTTATTACTGCCTGCAGTGGAGTAGTAACCCGCTCACGTTCG GTGCTGGGACCAAGCTGGAAATCAAAGGTGGTGGTGGTTCTGGTGGTGGTGGTTCTGG CGGCGGCGGCTCCGGTGGTGGTGGATCCGAGGTCCAGCTGCAGCAGTCTGGACCTGA GCTGGTGAAGCCTGGGGCTTCAGTGAAGATATCCTGCAAGACTTCTGGATACACATTCA CTGAATACACCATGCACTGGGTGAAGCAGAGCCATGGAAAGAGCCCTGAGTGGATTGG AGGTATTAATCCTAACAATGGTGGTACTACCTACAACCAGAAATTCAAGGGCAAGGCCAC ATTGACTGTAGACAAGTCCTCCAGCACAGCCTACATGGAGCTCCGCAGCCTGACATCTG AGGATTCTGCAGTCTATTACTGTGCGAGACGGATTACTACGATGATACCTACGGGGGGCT TTGACTACTGGGGCCAAGGCACCACTCTCACAGTCTCCTCA |
| SEQ ID NO. 38: a protein sequence of the 6E4D9 single-chain antibody |
| MEFGLSWLFLVAILKGVQCSRQIVLTQSPAIMSASPGEKVTMTCSASSSVSYMHWYQQKSG TSPKRWIYDTSKLASGVPARFSGSGSGTSYSLTISSMEAEDAATYYCLQWSSNPLTFGAGT KLEIKGGGGSGGGGSGGGGSGGGGSEVQLQQSGPELVKPGASVKISCKTSGYTFTEYTM HWVKQSHGKSPEWIGGINPNNGGTTYNQKFKGKATLTVDKSSSTAYMELRSLTSEDSAVYY CARRITTMIPTGGFDYWGQGTTLTVSS |
| SEQ ID NO. 39: a nucleic acid sequence of the 6E4D9 single-chain antibody |
| CAAATTGTTCTCACCCAGTCTCCAGCAATCATGTCTGCATCTCCAGGGGAGAAGGTCAC CATGACCTGCAGTGCCAGCTCAAGTGTAAGTTACATGCACTGGTACCAGCAGAAGTCAG GCACCTCCCCCAAAAGATGGATTTATGACACATCCAAACTGGCTTCTGGAGTCCCTGCT CGCTTCAGTGGCAGTGGGTCTGGGACCTCTTACTCTCTCACAATCAGCAGCATGGAGG CTGAAGATGCTGCCACTTATTACTGCCTGCAGTGGAGTAGTAACCCGCTCACGTTCGGT GCTGGGACCAAGCTGGAAATCAAA |
| SEQ ID NO. 40: a protein sequence of a light chain of the 6E4D9 single-chain antibody |
| QIVLTQSPAIMSASPGEKVTMTCSASSSVSYMHWYQQKSGTSPKRWIYDTSKLASGVPARF SGSGSGTSYSLTISSMEAEDAATYYCLQWSSNPLTFGAGTKLEIK |
| SEQ ID NO. 41: a protein sequence of CDR1 of the light chain of the 6E4D9 single-chain antibody SASSSVSYMH |
| SEQ ID NO. 42: a protein sequence of CDR2 of the light chain of the 6E4D9 single-chain antibody DTSKLAS |
| SEQ ID NO. 43: a protein sequence of CDR3 of the light chain of the 6E4D9 single-chain antibody LQWSSNPLT |
| SEQ ID NO. 44: a nucleic acid sequence of a heavy chain of the 6E4D9 single-chain antibody |
| GAGGTCCAGCTGCAGCAGTCTGGACCTGAGCTGGTGAAGCCTGGGGCTTCAGTGAAG ATATCCTGCAAGACTTCTGGATACACATTCACTGAATACACCATGCACTGGGTGAAGCAG AGCCATGGAAAGAGCCCTGAGTGGATTGGAGGTATTAATCCTAACAATGGTGGTACTACC TACAACCAGAAATTCAAGGGCAAGGCCACATTGACTGTAGACAAGTCCTCCAGCACAGC CTACATGGAGCTCCGCAGCCTGACATCTGAGGATTCTGCAGTCTATTACTGTGCGAGAC GGATTACTACGATGATACCTACGGGGGGCTTTGACTACTGGGGCCAAGGCACCACTCTC ACAGTCTCCTCA |
| SEQ ID NO. 45: a protein sequence of the heavy chain of the 6E4D9 single-chain antibody |
| EVQLQQSGPELVKPGASVKISCKTSGYTFTEYTMHWVKQSHGKSPEWIGGINPNNGGTTY NQKFKGKATLTVDKSSSTAYMELRSLTSEDSAVYYCARRITTMIPTGGFDYWGQGTTLTVSS |
| SEQ ID NO. 46: a protein sequence of CDR1 of the heavy chain of the 6E4D9 single-chain antibody GYTFTEYTMH |
| SEQ ID NO. 47: a protein sequence of CDR2 of the heavy chain of the 6E4D9 single-chain antibody GINPNNGGTTYNQKFKG |
| SEQ ID NO. 48: a protein sequence of CDR3 of the heavy chain of the 6E4D9 single-chain antibody ARRITTMIPTGGFDY |
| SEQ ID NO. 49: a nucleic acid sequence of a 31F3E5 single-chain antibody |
| ATGGAGTTTGGGCTGAGCTGGCTTTTTCTTGTGGCTATTTTAAAAGGTGTCCAGTGCTCT AGAGAAATTTTGCTCACCCAGTCTCCAGCAATCATAGCTGTATCTCCTGGGGAGAAGGT CACCATAACCTGCAGTGCCAGCTCAAGTGTAAATTACATGAACTGGTACCAACAGAAACC AGGATCCTCCCCCAAAATATGGATTTTTGGTATGTCCAACCTGGCTTCTGGAGTTCCTGC TCGCTTCAGTGGCAGTGGGTCTGGGACATCTTTCTCTTTCACAATCAACAGCATGGAGG CTGAAGATGTTGCCACTTATTACTGTCACCAAAGGAGTAGTTACCCATACACGTTCGGAG |
| GGGGGACCAAGCTGGAAATCAAAGGTGGTGGTTCTGGTGGTGGTTCTGGCG GCGGCGGCTCCGGTGGTGGTGGATCCCAGATCCAGTTGGTGCAGTCTGGACCTGAATT GAAGAAGCCTGGAGAGACAGTCAGGATCTCCTGCAAGGCTTCTGGATATACCTTCACAA ACTATGGAATGAACTGGGTGAAGCAGGCTCCAGGAAAGGGTTTGAAGTGGATGGGCTG GATTAATACCTACACTGGAGAGCCGACATATTCTGATGTCTTCAGGGGACGGTTTGCCTT CTCTTTGGAAACCTCTGCCAACACTGCCTATTTAGAGATCAGAAAACTCAAAAATGAGGA CATGGCTACGTATTTCTGTGTGAGAAATGAATACGACGCACCTTACTGGGGCCAAGGGA CTCTGGTCACTGTCTCTGCA |
| SEQ ID NO. 50: a protein sequence of the 31F3E5 single-chain antibody |
| MEFGLSWLFLVAILKGVQCSREILLTQSPAIIAVSPGEKVTITCSASSSVNYMNWYQQKPGSS PKIWIFGMSNLASGVPARFSGSGSGTSFSFTINSMEAEDVATYYCHQRSSYPYTFGGGTKL EIKGGGGSGGGGSGGGGSGGGGSQIQLVQSGPELKKPGETVRISCKASGYTFTNYGMNW VKQAPGKGLKWMGWINTYTGEPTYSDVFRGRFAFSLETSANTAYLEIRKLKNEDMATYFCV RNEYDAPYWGQGTLVTVSA |
| GAAATTTTGCTCACCCAGTCTCCAGCAATCATAGCTGTATCTCCTGGGGAGAAGGTCAC CATAACCTGCAGTGCCAGCTCAAGTGTAAATTACATGAACTGGTACCAACAGAAACCAGG ATCCTCCCCCAAAATATGGATTTTTGGTATGTCCAACCTGGCTTCTGGAGTTCCTGCTCG CTTCAGTGGCAGTGGGTCTGGGACATCTTTCTCTTTCACAATCAACAGCATGGAGGCTG AAGATGTTGCCACTTATTACTGTCACCAAAGGAGTAGTTACCCATACACGTTCGGAGGGG GGACCAAGCTGGAAATCAAA SEQ ID NO. 51: a nucleic acid sequence of a light chain of the 31F3E5 single-chain antibody |
| SEQ ID NO. 52: a protein sequence of a light chain of the 31F3E5 single-chain antibody |
| EILLTQSPAIIAVSPGEKVTITCSASSSVNYMNWYQQKPGSSPKIWIFGMSNLASGVPARFSG SGSGTSFSFTINSMEAEDVATYYCHQRSSYPYTFGGGTKLEIK |
| SEQ ID NO. 53: a protein sequence of CDR1 of the light chain of the 31F3E5 single-chain antibody SASSSVNYMN |
| SEQ ID NO. 54: a protein sequence of CDR2 of the light chain of the 31F3E5 single-chain antibody GMSNLAS |
| SEQ ID NO. 55: a protein sequence of CDR3 of the light chain of the 31F3E5 single-chain antibody HQRSSYPYT |
| SEQ ID NO. 56: a nucleic acid sequence of a heavy chain of the 31F3E5 single-chain antibody |
| CAGATCCAGTTGGTGCAGTCTGGACCTGAATTGAAGAAGCCTGGAGAGACAGTCAGGA TCTCCTGCAAGGCTTCTGGATATACCTTCACAAACTATGGAATGAACTGGGTGAAGCAGG CTCCAGGAAAGGGTTTGAAGTGGATGGGCTGGATTAATACCTACACTGGAGAGCCGACA TATTCTGATGTCTTCAGGGGACGGTTTGCCTTCTCTTTGGAAACCTCTGCCAACACTGC CTATTTAGAGATCAGAAAACTCAAAAATGAGGACATGGCTACGTATTTCTGTGTGAGAAAT GAATACGACGCACCTTACTGGGGCCAAGGGACTCTGGTCACTGTCTCTGCA |
| SEQ ID NO. 57: a protein sequence of the heavy chain of the 31F3E5 single-chain antibody |
| QIQLVQSGPELKKPGETVRISCKASGYTFTNYGMNWVKQAPGKGLKWMGWINTYTGEPTY SDVFRGRFAFSLETSANTAYLEIRKLKNEDMATYFCVRNEYDAPYWGQGTLVTVSA |
| SEQ ID NO. 58: a protein sequence of CDR1 of the heavy chain of the 31F3E5 single-chain antibody GYTFTNYGMN |
| SEQ ID NO. 59: a protein sequence of CDR2 of the heavy chain of the 31F3E5 single-chain antibody WINTYTGEPTYSDVFRG |
| SEQ ID NO. 60: a protein sequence of CDR3 of the heavy chain of the 31F3E5 single-chain antibody VRNEYDAPY |
| SEQ ID NO. 61: a nucleic acid sequence of a 21H2E11 single-chain antibody |
| ATGGAGTTTGGGCTGAGCTGGCTTTTTCTTGTGGCTATTTTAAAAGGTGTCCAGTGCTCT AGAAACATTGTGTTGGCCCAATCTCCAGCTTCTTTGGCTGTGTCTCTAGGGCAGAGGGC CACCATATCCTGCAGAGCCAGCGAAAGTGTTGATAATTATGGCTATAGTTTTATGCACTGG TACCAGCAGAAACCAGGACAGTCACCCAAACTCCTCATCTATCTTGCATCCAACCTAGAA TCTGGGGTCCCTGCCAGGTTCAGTGGCAGTGGGTCTAGGTCAGACTTCACCCTCACCA TTGATCCTGTGGAGGCTGATGATGCTGCAACCTATTACTGTCAGCAAAATAGTGAAGATC CGTGGACGTTCGGTGGAGGCACCAGGCTGGAAATCAAAGGTGGTGGTTCTGGTG GTGGTGGTTCTGGCGGCGGCGGCTCCGGTGGTGGTGGATCCCAGGTTCAACTCCAGC AGTCTGGGGCTGTGCTGGTGAAGCCTGGGGCCTCAGTGAAGATGTCCTGCAAGGCTTC TGGCTACACATTTACCAGTTACAATTTGCACTGGATAAAGCAGACACCTGGACAGGGCCT GGAATGGATTGGAGTTATTTATTCAGGAAATAGTGATACTTCAACCAATCAGAAGTTCAAA GGCAAGGCCACATTGACTGAAGACAAATCCGCCAGTACAGTCTACATATATCTCAGCAGC |
| CTGACATCTGAAGACTCTGCGGTCTATTACTGTGCAAGGGGGGAATATGGTAACCACGG GGGATTTGCTTACTGGGGCCAAGGGACTCTGGTCACTGTCTCTGCA |
| SEQ ID NO. 62: a protein sequence of the 21H2E11 single-chain antibody |
| MEFGLSWLFLVAILKGVQCSRNIVLAQSPASLAVSLGQRATISCRASESVDNYGYSFMHWY QQKPGQSPKLLIYLASNLESGVPARFSGSGSRSDFTLTIDPVEADDAATYYCQQNSEDPWT FGGGTRLEIKGGGGSGGGGSGGGGSGGGGSQVQLQQSGAVLVKPGASVKMSCKASGYT FTSYNLHWIKQTPGQGLEWIGVIYSGNSDTSTNQKFKGKATLTEDKSASTVYIYLSSLTSEDS AVYYCARGEYGNHGGFAYWGQGTLVTVSA |
| SEQ ID NO. 63: a nucleic acid sequence of the 21H2E11 single-chain antibody |
| AACATTGTGTTGGCCCAATCTCCAGCTTCTTTGGCTGTGTCTCTAGGGCAGAGGGCCAC CATATCCTGCAGAGCCAGCGAAAGTGTTGATAATTATGGCTATAGTTTTATGCACTGGTAC CAGCAGAAACCAGGACAGTCACCCAAACTCCTCATCTATCTTGCATCCAACCTAGAATCT GGGGTCCCTGCCAGGTTCAGTGGCAGTGGGTCTAGGTCAGACTTCACCCTCACCATTG ATCCTGTGGAGGCTGATGATGCTGCAACCTATTACTGTCAGCAAAATAGTGAAGATCCGT GGACGTTCGGTGGAGGCACCAGGCTGGAAATCAAA |
| SEQ ID NO. 64: a protein sequence of a light chain of the 21H2E11 single-chain antibody |
| NIVLAQSPASLAVSLGQRATISCRASESVDNYGYSFMHWYQQKPGQSPKLLIYLASNLESGV PARFSGSGSRSDFTLTIDPVEADDAATYYCQQNSEDPWTFGGGTRLEIK |
| SEQ ID NO. 65: a protein sequence of CDR1 of the light chain of the 21H2E11 single-chain antibody RASESVDNYGYSFMH |
| SEQ ID NO. 66: a protein sequence of CDR2 of the light chain of the 21H2E11 single-chain antibody LASNLES |
| SEQ ID NO. 67: a protein sequence of CDR3 of the light chain of the 21H2E11 single-chain antibody QQNSEDPWT |
| SEQ ID NO. 68: a nucleic acid sequence of a heavy chain of the 21H2E11 single-chain antibody |
| CAGGTTCAACTCCAGCAGTCTGGGGCTGTGCTGGTGAAGCCTGGGGCCTCAGTGAAGA TGTCCTGCAAGGCTTCTGGCTACACATTTACCAGTTACAATTTGCACTGGATAAAGCAGA CACCTGGACAGGGCCTGGAATGGATTGGAGTTATTTATTCAGGAAATAGTGATACTTCAA CCAATCAGAAGTTCAAAGGCAAGGCCACATTGACTGAAGACAAATCCGCCAGTACAGTC |
| TACATATATCTCAGCAGCCTGACATCTGAAGACTCTGCGGTCTATTACTGTGCAAGGGGG GAATATGGTAACCACGGGGGATTTGCTTACTGGGGCCAAGGGACTCTGGTCACTGTCTC TGCA |
| SEQ ID NO. 69: a protein sequence of a heavy chain of the 21H2E11 single-chain antibody |
| QVQLQQSGAVLVKPGASVKMSCKASGYTFTSYNLHWIKQTPGQGLEWIGVIYSGNSDTST NQKFKGKATLTEDKSASTVYIYLSSLTSEDSAVYYCARGEYGNHGGFAYWGQGTLVTVSA |
| SEQ ID NO. 70: a protein sequence of CDR1 of the heavy chain of the 21H2E11 single-chain antibody GYTFTSYNLH |
| SEQ ID NO. 71: a protein sequence of CDR2 of the heavy chain of the 21H2E11 single-chain antibody VIYSGNSDTSTNQKFKG |
| SEQ ID NO. 72: a protein sequence of CDR3 of the heavy chain of the 21H2E11 single-chain antibody ARGEYGNHGGFAY |
| SEQ ID NO. 73: a nucleic acid sequence of a 24A11A11 single-chain antibody |
| ATGGAGTTTGGGCTGAGCTGGCTTTTTCTTGTGGCTATTTTAAAAGGTGTCCAGTGCTCT AGAGACATTGTGATGACCCAGTCTCAAAAATTCATGTCCACATCAGTAGGAGACAGGGT CAGCATCACCTGCAAGGCCAGTCAGAATGTTCGTACTGCTGTAGCCTGGTATCAACAGA AACCAGGCCAGTCTCCTAAAGCACTGATTTACTTGCCATCCAACCGGCACACTGGAGTC CCTGATCGCTTCACAGGCAGTGGATCTGGGACAGATTTCACTCTCACCATTAGCAATGT GCAATCTGAAGACCTGGCAGATTATTTCTGTCTGCAACATTGGAATTATCCATTCACGTTC GGCTCGGGGACAAAGTTGGAAATAAAAGGTGGTGGTGGTTCTGGTGGTGGTGGTTCTG GCGGCGGCGGCTCCGGTGGTGGTGGATCCCAGGTCCAGCTGAAGCAGTCTGGAGCTG AGCTGGTGAGGCCTGGGGCTTCAGTGAATCTGTCCTGCAAGACTTCTGGATACATCTTC ACCAGCTACTGGATTCACTGGGTAAAACAGAGGTCTGGACAGGGCCTTGAGTGGATTG CAAGGATTTATCCTGGAGTTGATAGTATTTATTACAATGAGAAGTTCAAGGGCAAGGCCA CACTGACTGCAGACAAATCCTCCAGCACTGCCTACATGCAGCTCAGCAGCCTGAAATCT GAGGACTCTGCTGTCTATTTCTGTGCAACTATCTCCTATGATTACGACTATTCTATGGACT ACTGGGGTCAAGGAACCTCAGTCACCGTCTCCTCA |
| SEQ ID NO. 74: a protein sequence of the 24A11A11 single-chain antibody |
| MEFGLSWLFLVAILKGVQCSRDIVMTQSQKFMSTSVGDRVSITCKASQNVRTAVAWYQQKP GQSPKALIYLPSNRHTGVPDRFTGSGSGTDFTLTISNVQSEDLADYFCLQHWNYPFTFGSG |
| TKLEIKGGGGSGGGGSGGGGSGGGGSQVQLKQSGAELVRPGASVNLSCKTSGYIFTSYWI HWVKQRSGQGLEWIARIYPGVDSIYYNEKFKGKATLTADKSSSTAYMQLSSLKSEDSAVYFC ATISYDYDYSMDYWGQGTSVTVSS |
| SEQ ID NO. 75: a nucleic acid sequence of a light chain of the 24A11A11 single-chain antibody |
| GACATTGTGATGACCCAGTCTCAAAAATTCATGTCCACATCAGTAGGAGACAGGGTCAG CATCACCTGCAAGGCCAGTCAGAATGTTCGTACTGCTGTAGCCTGGTATCAACAGAAAC CAGGCCAGTCTCCTAAAGCACTGATTTACTTGCCATCCAACCGGCACACTGGAGTCCCT GATCGCTTCACAGGCAGTGGATCTGGGACAGATTTCACTCTCACCATTAGCAATGTGCAA TCTGAAGACCTGGCAGATTATTTCTGTCTGCAACATTGGAATTATCCATTCACGTTCGGCT CGGGGACAAAGTTGGAAATAAAA |
| SEQ ID NO. 76: a protein sequence of the light chain of the 24A11A11 single-chain antibody |
| DIVMTQSQKFMSTSVGDRVSITCKASQNVRTAVAWYQQKPGQSPKALIYLPSNRHTGVPDR FTGSGSGTDFTLTISNVQSEDLADYFCLQHWNYPFTFGSGTKLEIK |
| SEQ ID NO. 77: a protein sequence of CDR1 of the light chain of the 24A11A11 single-chain antibody KASQNVRTAVA |
| SEQ ID NO. 78: a protein sequence of CDR2 of the light chain of the 24A11A11 single-chain antibody LPSNRHT |
| SEQ ID NO. 79: a protein sequence of CDR3 of the light chain of the 24A11A11 single-chain antibody LQHWNYPFT |
| SEQ ID NO. 80: a nucleic acid sequence of a heavy chain of the 24A11A11 single-chain antibody |
| AGGTCCAGCTGAAGCAGTCTGGAGCTGAGCTGGTGAGGCCTGGGGCTTCAGTGAATCT GTCCTGCAAGACTTCTGGATACATCTTCACCAGCTACTGGATTCACTGGGTAAAACAGAG GTCTGGACAGGGCCTTGAGTGGATTGCAAGGATTTATCCTGGAGTTGATAGTATTTATTA CAATGAGAAGTTCAAGGGCAAGGCCACACTGACTGCAGACAAATCCTCCAGCACTGCC TACATGCAGCTCAGCAGCCTGAAATCTGAGGACTCTGCTGTCTATTTCTGTGCAACTATC TCCTATGATTACGACTATTCTATGGACTACTGGGGTCAAGGAACCTCAGTCACCGTCTCC TCA |
| SEQ ID NO. 81: a protein sequence of the heavy chain of the 24A11A11 single-chain |
| antibody |
| QVQLKQSGAELVRPGASVNLSCKTSGYIFTSYWIHWVKQRSGQGLEWIARIYPGVDSIYYN EKFKGKATLTADKSSSTAYMQLSSLKSEDSAVYFCATISYDYDYSMDYWGQGTSVTVSS |
| SEQ ID NO. 82: a protein sequence of CDR1 of the heavy chain of the 24A11A11 single-chain antibody GYIFTSYWIH |
| SEQ ID NO. 83: a protein sequence of CDR2 of the heavy chain of the 24A11A11 single-chain antibody RIYPGVDSIYYNEKFKG |
| SEQ ID NO. 84: a protein sequence of CDR3 of the heavy chain of the 24A11A11 single-chain sequence ATISYDYDYSMDY |
| SEQ ID NO. 85: a nucleic acid sequence of a 42B5C12 single-chain antibody |
| ATGGAGTTTGGGCTGAGCTGGCTTTTTCTTGTGGCTATTTTAAAAGGTGTCCAGTGCTCT AGAGATATTGTGATGACGCAGGCTGCATTCTCCAATCCAGTCACTCTTGGAACATCAGCT TCCATCTCCTGCAGGTCTAGTAAGAGTCTCCTCCATAGTAATGGCATCACTTATTTCTATT GGTATCTGCAGAAGCCAGGCCAGTCTCCTCAGCTCCTGATTTATCAGATGTCCAGCCTT GCCTCAGGAGTCCCAGACAGGTTCAGTAGCAGTGGGTCAGGAACTGATTTCACACTGA GAATCAGCAGAGTGGAGGCTGAGGATGTGGGTGTTTATTACTGTGCTCAAAATCTAGAA CTTCCGTACACGTTCGGAGGGGGGACCAAGCTGGAAATCAAAGGTGGTGGTGGTTCTG GTGGTGGTGGTTCTGGCGGCGGCGGCTCCGGTGGTGGTGGATCCCAGATCCAGTTGG TGCAGTCTGGACCTGAGGTGAGGAGGCCTGGGGAGACAGTCAAGATCTCCTGCAAGG CTTCTGGATATACTTTCACAAACTTTGGAATGAACTGGGTGAAACAGGCTCCAGGAAAG GCTTTACAGTGGATGGGCTGGATAAACACCTACACTGGTGAGCCAACATATGCTGATGA CTTCAAGGGCCGGTTTGCCTTCTCTTTGGAATCCTCTGCCAGCACTGCCCATTTGCAGA TCAGCTACCTCAAAGATGAGGACACGGCTACATATTTCTGTGCGAGATGGTGGCTTCAAT ATGCTTTGGACAACTGGGGTCAAGGAACCTCAGTCACCGTCTCCTCA |
| SEQ ID NO. 86: a protein sequence of the 42B5C12 single-chain antibody |
| MEFGLSWLFLVAILKGVQCSRDIVMTQAAFSNPVTLGTSASISCRSSKSLLHSNGITYFYWYL QKPGQSPQLLIYQMSSLASGVPDRFSSSGSGTDFTLRISRVEAEDVGVYYCAQNLELPYTF GGGTKLEIKGGGGSGGGGSGGGGSGGGGSQIQLVQSGPEVRRPGETVKISCKASGYTFT NFGMNWVKQAPGKALQWMGWINTYTGEPTYADDFKGRFAFSLESSASTAHLQISYLKDED TATYFCARWWLQYALDNWGQGTSVTVSS |
| SEQ ID NO. 87: a nucleic acid sequence of a light chain of the 42B5C12 single-chain antibody |
| GATATTGTGATGACGCAGGCTGCATTCTCCAATCCAGTCACTCTTGGAACATCAGCTTCC ATCTCCTGCAGGTCTAGTAAGAGTCTCCTCCATAGTAATGGCATCACTTATTTCTATTGGT ATCTGCAGAAGCCAGGCCAGTCTCCTCAGCTCCTGATTTATCAGATGTCCAGCCTTGCC TCAGGAGTCCCAGACAGGTTCAGTAGCAGTGGGTCAGGAACTGATTTCACACTGAGAAT CAGCAGAGTGGAGGCTGAGGATGTGGGTGTTTATTACTGTGCTCAAAATCTAGAACTTC CGTACACGTTCGGAGGGGGGACCAAGCTGGAAATCAAA |
| SEQ ID NO. 88: a protein sequence of the light chain of the 42B5C12 single-chain antibody DIVMTQAAFSNPVTLGTSAS ISCRSSKSLLHSNG ITYFYWYLQKPGQSPQLLIYQMSSLASG VPDRFSSSGSGTDFTLRISRVEAEDVGVYYCAQNLELPYTFGGGTKLEIK |
| SEQ ID NO. 89: a protein sequence of CDR1 of the light chain of the 42B5C12 single-chain antibody RSSKSLLHSNGITYFY |
| SEQ ID NO. 90: a protein sequence of CDR2 of the light chain of the 42B5C12 single-chain antibody QMSSLAS |
| SEQ ID NO. 91: a protein sequence of CDR3 of the light chain of the 42B5C12 single-chain antibody AQNLELPYT |
| SEQ ID NO. 92: a nucleic acid sequence of a heavy chain of the 42B5C12 single-chain antibody |
| CAGATCCAGTTGGTGCAGTCTGGACCTGAGGTGAGGAGGCCTGGGGAGACAGTCAAG ATCTCCTGCAAGGCTTCTGGATATACTTTCACAAACTTTGGAATGAACTGGGTGAAACAG GCTCCAGGAAAGGCTTTACAGTGGATGGGCTGGATAAACACCTACACTGGTGAGCCAAC ATATGCTGATGACTTCAAGGGCCGGTTTGCCTTCTCTTTGGAATCCTCTGCCAGCACTG CCCATTTGCAGATCAGCTACCTCAAAGATGAGGACACGGCTACATATTTCTGTGCGAGAT GGTGGCTTCAATATGCTTTGGACAACTGGGGTCAAGGAACCTCAGTCACCGTCTCCTCA |
| SEQ ID NO. 93: a protein sequence of the heavy chain of the 42B5C12 single-chain antibody |
| QIQLVQSGPEVRRPGETVKISCKASGYTFTNFGMNWVKQAPGKALQWMGWINTYTGEPTY ADDFKGRFAFSLESSASTAHLQISYLKDEDTATYFCARWWLQYALDNWGQGTSVTVSS |
| SEQ ID NO. 94: a protein sequence of CDR1 of the heavy chain of the 42B5C12 single-chain antibody GYTFTNFGMN |
| SEQ ID NO. 95: a protein sequence of CDR2 of the heavy chain of the 42B5C12 single-chain antibody WINTYTGEPTYADDFKG |
| SEQ ID NO. 96: a protein sequence of CDR3 of the heavy chain of the 42B5C12 single-chain antibody ARWWLQYALDN |
| SEQ ID NO. 97: a nucleic sequence of a 14A7A8 single-chain antibody |
| ATGGAGTTTGGGCTGAGCTGGCTTTTTCTTGTGGCTATTTTAAAAGGTGTCCAGTGCTCT AGAAACATTGTGCTGACCCAATCTCCAGCTTCTTTGGCTGTGTCTCTAGGGCAGAGGGC CACCATATCCTGCAGAGCCAGTGAAAATGTTGATAATTATGGCAATAGTTTTATACACTGG TACCAGCAGAAACCAGGACAGCCACCCAAACTCCTCATCTATCTTGCATCCAACCTAGAA TCTGGGGTCCCTGCCAGGTTCAGTGGCAGTGGGTCTAGGTCAGACTTCACCCTCACCA TTGATCCTGTGGAGGCTGATGATGCTGCAACCTATTACTGTCAGCAAAATAATGAGGATC CGTGGACGTTCGGTGGAGGCACCAAGCTGGAAATCAAAGGTGGTGGTGGTTCTGGTG GTGGTGGTTCTGGCGGCGGCGGCTCCGGTGGTGGTGGATCCCAGGTCCAACTGCAGC AGCCTGGGGCTGAGCTGGTGAAGCCTGGGGCCTCAGTGAAGATGTCCTGCAAGGCTT CTGGCTACATATTTACCAGTTACAATTTGCACTGGGTAAAACAGACACCTGGACAGGGCC TGGAATGGATTGGAGTTATTTATTCAGGAAATAGTGATACTTCCAACAATCAGAAGTTCAA AGGCAAGGCCACATTGACTGAAGACAAATCCGCCAGCACAGCCTACCTACACCTCAGCA GCCTGACATCTGAAGACTCAGCGGTCTATTTCTGTGCAAGGGGGGAATATGGTAACCAC GGGGGGTTTGCTTACTGGGGCCAAGGGACTCTGGTCACTGTCTCTGCA |
| SEQ ID NO. 98: a protein sequence of the 14A7A8 single-chain antibody |
| MEFGLSWLFLVAILKGVQCSRNIVLTQSPASLAVSLGQRATISCRASENVDNYGNSFIHWYQ QKPGQPPKLLIYLASNLESGVPARFSGSGSRSDFTLTIDPVEADDAATYYCQQNNEDPWTF GGGTKLEIKGGGGSGGGGSGGGGSGGGGSQVQLQQPGAELVKPGASVKMSCKASGYIF TSYNLHWVKQTPGQGLEWIGVIYSGNSDTSNNQKFKGKATLTEDKSASTAYLHLSSLTSEDS AVYFCARGEYGNHGGFAYWGQGTLVTVSA |
| SEQ ID NO. 99: a nucleic sequence of a light chain of the 14A7A8 single-chain antibody |
| AACATTGTGCTGACCCAATCTCCAGCTTCTTTGGCTGTGTCTCTAGGGCAGAGGGCCAC CATATCCTGCAGAGCCAGTGAAAATGTTGATAATTATGGCAATAGTTTTATACACTGGTAC CAGCAGAAACCAGGACAGCCACCCAAACTCCTCATCTATCTTGCATCCAACCTAGAATCT GGGGTCCCTGCCAGGTTCAGTGGCAGTGGGTCTAGGTCAGACTTCACCCTCACCATTG |
| ATCCTGTGGAGGCTGATGATGCTGCAACCTATTACTGTCAGCAAAATAATGAGGATCCGT GGACGTTCGGTGGAGGCACCAAGCTGGAAATCAAA |
| SEQ ID NO. 100: a protein sequence of the light chain of the 14A7A8 single-chain antibody |
| NIVLTQSPASLAVSLGQRATISCRASENVDNYGNSFIHWYQQKPGQPPKLLIYLASNLESGVP ARFSGSGSRSDFTLTIDPVEADDAATYYCQQNNEDPWTFGGGTKLEIK |
| SEQ ID NO. 101: a protein sequence of CDR1 of the light chain of the 14A7A8 single-chain antibody RASENVDNYGNSFIH |
| SEQ ID NO. 102: a protein sequence of CDR2 of the light chain of the 14A7A8 single-chain antibody LASNLES |
| SEQ ID NO. 103: a protein sequence of CDR3 of the light chain of the 14A7A8 single-chain antibody QQNNEDPWT |
| SEQ ID NO. 104: a nucleic acid sequence of a heavy chain of the 14A7A8 single-chain antibody |
| CAGGTCCAACTGCAGCAGCCTGGGGCTGAGCTGGTGAAGCCTGGGGCCTCAGTGAAG ATGTCCTGCAAGGCTTCTGGCTACATATTTACCAGTTACAATTTGCACTGGGTAAAACAG ACACCTGGACAGGGCCTGGAATGGATTGGAGTTATTTATTCAGGAAATAGTGATACTTCC AACAATCAGAAGTTCAAAGGCAAGGCCACATTGACTGAAGACAAATCCGCCAGCACAGC CTACCTACACCTCAGCAGCCTGACATCTGAAGACTCAGCGGTCTATTTCTGTGCAAGGG GGGAATATGGTAACCACGGGGGGTTTGCTTACTGGGGCCAAGGGACTCTGGTCACTGT CTCTGCA |
| SEQ ID NO. 105: a protein sequence of the heavy chain of the 14A7A8 single-chain antibody |
| QVQLQQPGAELVKPGASVKMSCKASGYIFTSYNLHWVKQTPGQGLEWIGVIYSGNSDTSN NQKFKGKATLTEDKSASTAYLHLSSLTSEDSAVYFCARGEYGNHGGFAYWGQGTLVTVSA |
| SEQ ID NO. 106: a protein sequence of CDR1 of the heavy chain of the 14A7A8 single-chain antibody GYIFTSYNLH |
| SEQ ID NO. 107: a protein sequence of CDR2 of the heavy chain of the 14A7A8 single-chain antibody VIYSGNSDTSNNQKFKG |
| SEQ ID NO. 108: a protein sequence of CDR3 of the heavy chain of the 14A7A8 single-chain antibody ARGEYGNHGGFAY |
| SEQ ID NO. 109: a nucleic acid sequence of a 41C10E7 single-chain antibody |
| ATGGAGTTTGGGCTGAGCTGGCTTTTTCTTGTGGCTATTTTAAAAGGTGTCCAGTGCTCT AGAGACATCCAGATGACTCAGTCTCCAGCCTCCCTAACTGTATTTGTGGGGGAAACTGT CACCATCACATGTCGAGCAAGTGAGAATATTTACAGTAATTTAGCGTGGTATCAGCAGAA ACAGGGAAAATCTCCTCAGCTCCTGGTCTATACTGCAACAAATTTAATAGATGGTGTGCC ATCAAGGTTCAGTGGCAGTGGGTCCGGCACACAGTATTCCCTCAAGATCAACAGCCTGC AGTCTGAAGATTTCGGGAGTTATTACTGTCAACATTTTTGGGGTACTCCGGTCACGTTCG GTGCTGGGACCAAGCTGGAAATCAAAGGTGGTGGTGGTTCTGGTGGTGGTGGTTCTGG CGGCGGCGGCTCCGGTGGTGGTGGATCCCAGGTCCAACTGCAGCAGCCTGGGGCAG AACTGGTGAAGCCTGGGGCCTCAGTGAGGATGTCCTGCAGGGCTTCTGGCTACACTTT TACCAGTTACAATTTGCACTGGGTAAAACAGACACCTGGACAGGGCCTGGAATGGATTG GGGCTATTCATCCAGGAGATAATTATACTTCCTACAATCAGATTTTCAACGACAAGGCCAC ATTGACTGCAGACAAATCCTCCAGCACAGCCTACATGCAGCTCAGTAGCCTGACATCTG AGGACTCTGCGGTCTATTTCTGTGCAAGGAATTACTACGGTAGTCTTGATTACTGGGGCC AAGGCACCACTCTCACAGTCTCCTCA |
| SEQ ID NO. 110: a protein sequence of the 41C10E7 single-chain antibody |
| MEFGLSWLFLVAILKGVQCSRDIQMTQSPASLTVFVGETVTITCRASENIYSNLAWYQQKQG KSPQLLVYTATNLIDGVPSRFSGSGSGTQYSLKINSLQSEDFGSYYCQHFWGTPVTFGAGT KLEIKGGGGSGGGGSGGGGSGGGGSQVQLQQPGAELVKPGASVRMSCRASGYTFTSYN LHWVKQTPGQGLEWIGAIHPGDNYTSYNQIFNDKATLTADKSSSTAYMQLSSLTSEDSAVYF CARNYYGSLDYWGQGTTLTVSS |
| SEQ ID NO. 111: a nucleic acid of a light chain of the 41C10E7 single-chain antibody |
| GACATCCAGATGACTCAGTCTCCAGCCTCCCTAACTGTATTTGTGGGGGAAACTGTCAC CATCACATGTCGAGCAAGTGAGAATATTTACAGTAATTTAGCGTGGTATCAGCAGAAACA GGGAAAATCTCCTCAGCTCCTGGTCTATACTGCAACAAATTTAATAGATGGTGTGCCATC AAGGTTCAGTGGCAGTGGGTCCGGCACACAGTATTCCCTCAAGATCAACAGCCTGCAG TCTGAAGATTTCGGGAGTTATTACTGTCAACATTTTTGGGGTACTCCGGTCACGTTCGGT GCTGGGACCAAGCTGGAAATCAAA |
| SEQ ID NO. 112: a protein sequence of the light chain of the 41C10E7 single-chain antibody |
| DIQMTQSPASLTVFVGETVTITCRASENIYSNLAWYQQKQGKSPQLLVYTATNLIDGVPSRFS GSGSGTQYSLKINSLQSEDFGSYYCQHFWGTPVTFGAGTKLEIK |
| SEQ ID NO. 113: a protein sequence of CDR1 of the light chain of the 41C10E7 single-chain antibody RASENIYSNLA |
| SEQ ID NO. 114: a protein sequence of CDR2 of the light chain of the 41C10E7 single-chain antibody TATNLI |
| SEQ ID NO. 115: a protein sequence of CDR3 of the light chain of the 41C10E7 single-chain antibody QHFWGTPVT |
| SEQ ID NO. 116: a nucleic acid sequence of a heavy chain of the 41C10E7 single-chain antibody |
| CAGGTCCAACTGCAGCAGCCTGGGGCAGAACTGGTGAAGCCTGGGGCCTCAGTGAGG ATGTCCTGCAGGGCTTCTGGCTACACTTTTACCAGTTACAATTTGCACTGGGTAAAACAG ACACCTGGACAGGGCCTGGAATGGATTGGGGCTATTCATCCAGGAGATAATTATACTTCC TACAATCAGATTTTCAACGACAAGGCCACATTGACTGCAGACAAATCCTCCAGCACAGC CTACATGCAGCTCAGTAGCCTGACATCTGAGGACTCTGCGGTCTATTTCTGTGCAAGGA ATTACTACGGTAGTCTTGATTACTGGGGCCAAGGCACCACTCTCACAGTCTCCTCA |
| SEQ ID NO. 117: a protein sequence of the heavy chain of the 41C10E7 single-chain antibody |
| QVQLQQPGAELVKPGASVRMSCRASGYTFTSYNLHWVKQTPGQGLEWIGAIHPGDNYTSY NQIFNDKATLTADKSSSTAYMQLSSLTSEDSAVYFCARNYYGSLDYWGQGTTLTVSS |
| SEQ ID NO. 118: a protein sequence of CDR1 of the heavy chain of the 41C10E7 single-chain antibody GYTFTSYNLH |
| SEQ ID NO. 119: a protein sequence of CDR2 of the heavy chain of the 41C10E7 single-chain antibody AIHPGDNYTSYNQIFND |
| SEQ ID NO. 120: a protein sequence of CDR3 of the heavy chain of the 41C10E7 single-chain antibody ARNYYGSLDY |
| SEQ ID NO. 121: a nucleic acid sequence of a hinge region of CD8α |
| ACCACGACGCCAGCGCCGCGACCACCAACACCGGCGCCCACCATCGCGTCGCAGCCC CTGTCCCTGCGCCCAGAGGCGTGCCGGCCAGCGGCGGGGGCGCAGTGCACACGAG GGGGCTGGACTTCGCCTGTGAT |
| SEQ ID NO. 122: a protein sequence of the hinge region of CD8α TTTPAPRPPTPAPTIASQPLSLRPEACRPAAGGAVHTRGLDFACD |
| SEQ ID NO. 123: a nucleic acid sequence of a transmembrane region of CD8α |
| ATCTACATCTGGGCGCCCTTGGCCGGGACTTGTGGGGTCCTTCTCCTGTCACTGGTTAT CACCCTTTACTGC |
| SEQ ID NO. 124: a protein sequence of the transmembrane region of CD8α IYIWAPLAGTCGVLLLSLVITLYC |
| SEQ ID NO. 125: a nucleic acid sequence of a CD28 costimulatory factor |
| AGGAGTAAGAGGAGCAGGCTCCTGCACAGTGACTACATGAACATGACTCCCCGCCGCC CCGGGCCCACCCGCAAGCATTACCAGCCCTATGCCCCACCACGCGACTTCGCAGCCTA TCGCTCC |
| SEQ ID NO. 126: a protein sequence of the CD28 costimulatory factor RSKRSRLLHSDYMNMTPRRPGPTRKHYQPYAPPRDFAAYRS SEQ ID NO. 127: a nucleic acid sequence of a 4-1BB costimulatory factor |
| AAACGGGGCAGAAAGAAACTCCTGTATATATTCAAACAACCATTTATGAGACCAGTACAAA CTACTCAAGAGGAAGATGGCTGTAGCTGCCGATTTCCAGAAGAAGAAGAAGGAGGATGT |
| GAACTG |
| SEQ ID NO. 128: a protein sequence of the 4-1BB costimulatory factor KRGRKKLLYIFKQPFMRPVQTTQEEDGCSCRFPEEEEGGCEL |
| SEQ ID NO. 129: a nucleic acid sequence of a CD3ζ signal transduction region |
| AGAGTGAAGTTCAGCAGGAGCGCAGACGCCCCCGCGTACCAGCAGGGCCAGAACCAG CTCTATAACGAGCTCAATCTAGGACGAAGAGAGGAGTACGATGTTTTGGACAAGAGACG TGGCCGGGACCCTGAGATGGGGGGAAAGCCGAGAAGGAAGAACCCTCAGGAAGGCCT GTACAATGAACTGCAGAAAGATAAGATGGCGGAGGCCTACAGTGAGATTGGGATGAAAG GCGAGCGCCGGAGGGGCAAGGGGCACGATGGCCTTTACCAGGGTCTCAGTACAGCCA CCAAGGACACCTACGACGCCCTTCACATGCAGGCCCTGCCCCCTCGCTAA |
| SEQ ID NO. 130: a protein sequence of the CD3ζ signal transduction region |
| RVKFSRSADAPAYQQGQNQLYNELNLGRREEYDVLDKRRGRDPEMGGKPRRKNPQEGLY NELQKDKMAEAYSEIGMKGERRRGKGHDGLYQGLSTATKDTYDALHMQALPPR |

### Example 4: preparation of retroviruses containing the chimeric antigen receptors

Moloney murine leukemia viruses (MMLV) were prepared by transiently transfecting 293T cells with a three-plasmid system (GFS-CAR, MMLV-gag-pol and RV-RD). CD19.28 CAR was used as a control, and B7-H3.BB CAR and B7-H3.28 CAR derived from a 15C11 humanized antibody were the CARs to be tested. After 293T cells were transfected for 48 and 72 h, supernatant of 293 T cells were collected and used to transduce CD3 and CD28 antibody-stimulated T cells from health individuals.

### Example 5: preparation of T cells expressing the chimeric antigen receptors targeting B7-H3

### 1. T-cell transduction and amplification

Peripheral blood mononuclear cells were isolated from peripheral blood donated by healthy individuals. The mononuclear cells were isolated by Lymphoprep density separation method. The isolated mononuclear cells were activated with CD3 and CD28 antibodies, and T cells were transduced with the retroviral supernatant (CD19.28 CAR, B7-H3.BB CAR, and B7-H3.28 CAR) collected in Example 4. The T cells transduced with the chimeric antigen receptors were further amplified for detection of the expression and tumor-killing effects of the B7-H3 chimeric antigen receptors.

### 2. Detection of the expression of the B7-H3 chimeric antigen receptor

3 days after transduction, B7-H3-targeted chimeric antigen receptor-T cells were collected, and the expression of the B7-H3-targeted chimeric antigen receptor was detected by using a fusion protein of B7-H3 and an antibody Fc fragment and a secondary goat-anti-human Fc antibody labelled with an APC fluorophore. The expression of the B7-H3 chimeric antigen receptors on the T cells was detected by flow cytometry.

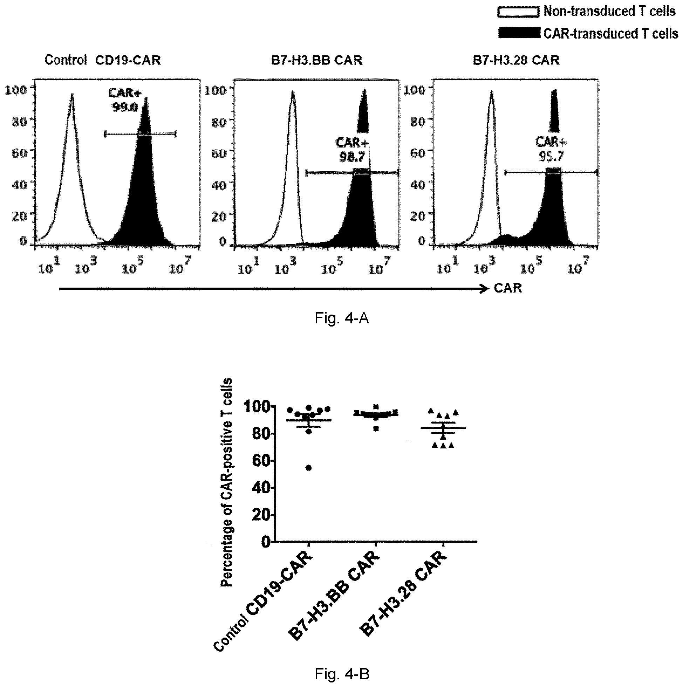

Results: similar to the control CD19.28 CAR, more than 90% of the T cells expressed B7-H3 chimeric antigen receptor (B7-H3.BB CAR or B7-H3.28 CAR) of the present disclosure (see Fig. 4A). The results were also confirmed in T cells from multiple different healthy donors (see Fig. 4B).

### Example 6: killing effects of B7-H3-targeted chimeric antigen receptor-T cells on tumor cells in co-culture systems in vitro

### 1. Killing effect of the B7-H3-targeted chimeric antigen receptor-T cells on tumor cells

To determine whether the chimeric antigen receptor of the present disclosure could mediate T cells to kill tumor cells highly expressing B7-H3, we designed a co-culture experiment of T cells and tumor cells to test the killing effects of the T cells on various tumor cells. After the tumor cell attachment for 24 h, T cells expressing the control (CD19.28 CAR-T) and the B7-H3-targeted chimeric antigen receptor (B7-H3.28 CAR-T) were added, and then killing effects of CAR-T cells on various tumor cells were detected by flow cytometry.

Method: tumor cells (SK-OV-3, LN-18, LN-229, and WM-266-4) labelled with GFP were inoculated into a 24-well plate with a density of 1-2×10⁵ cells per well, and 24 h later, a corresponding number of CAR-T cells transduced for 10 days were added according to a ratio of the T cells to the tumor cells of 1: 1. 7 d later, the cells in the wells were collected, and percentage of remaining tumor cells was detected by flow cytometry. The CAR-T cells were detected by using a mouse-anti-human CD3 fluorescent antibody, and the tumor cells were detected by using spontaneous GFP fluorescence.

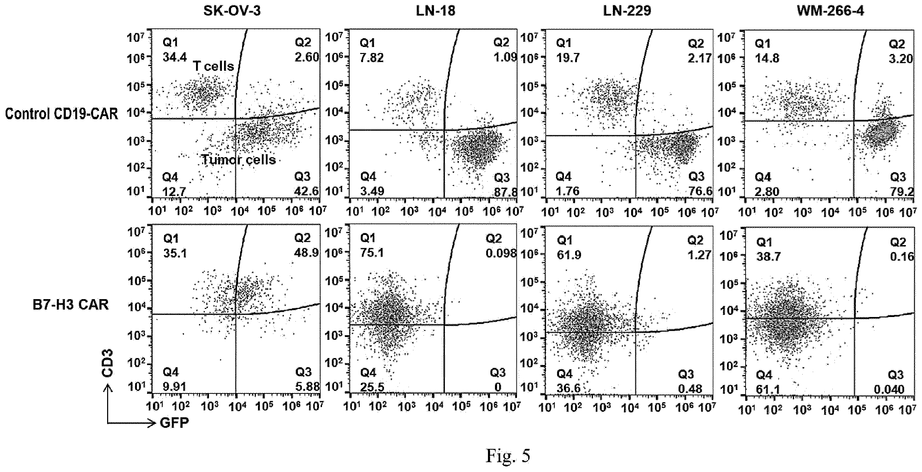

Results: as shown in Fig. 5, the control CAR-T cells could not kill any kind of tumor cells, while the B7-H3 CAR-T cells of the present disclosure could completely kill the tumor cells when co-cultured with various tumor cell lines (SK-OV-3, LN-18, LN-229, and WM-266-4), and meanwhile, the CAR-T cells also exhibited cell proliferation.

### 2. Comparison of the B7-H3-targeted CAR of the present disclosure and other CARs targeting the same antigen: the affinity to the antigen, and cytokine release during tumor cell killing by three kinds of CAR-T cells

In view of two existing other chimeric antigen receptors in the art, we compared the CAR of the present invention with the two CARs (CAR1 of WO2017/044699, and CAR2 of US10,233,226 B2) on the affinity to the B7-H3 antigen and cytokine release during tumor cell killing by the three kinds of CAR-T cells.

### Method: Pull-down assay

293T cells were transfected with B7-H3-FLAG or FLAG empty vector control, respectively. Other 293T cells were transfected with the three kinds of B7-H3-targeted CARs, respectively, and the expression levels were adjusted to the same level. 48 h later, the 293T cells transfected with B7-H3-FLAG or FLAG empty vector control were lysed with RIPA lysis buffer and centrifuged, the supernatant was collected, an anti-FLAG M2 affinity gel was added, incubated at 4°C for 2 h, and washed with RIPA lysis buffer for later use. The 293T cells transfected with different B7-H3-targeted CARs were lysed with RIPA lysis buffer and centrifuged, and supernatants were collected, and respectively incubated with the anti-FLAG M2 gel pull-down products of B7-H3-FLAG or FLAG empty vector control at 4°C for 1 h. The products were then washed with RIPA lysis buffer for 4 times, eluted with elution buffer (0.1 M glycine-HCI, pH 3.5) by shaking at room temperature for 10 min, the supernatants were collected, neutralized with 1 M Tris-HCI (pH 7.5), added with an SDS loading buffer, and denatured at 95°C for 5 min, and the affinity of the CARs to the antigen was analysed by western blot.

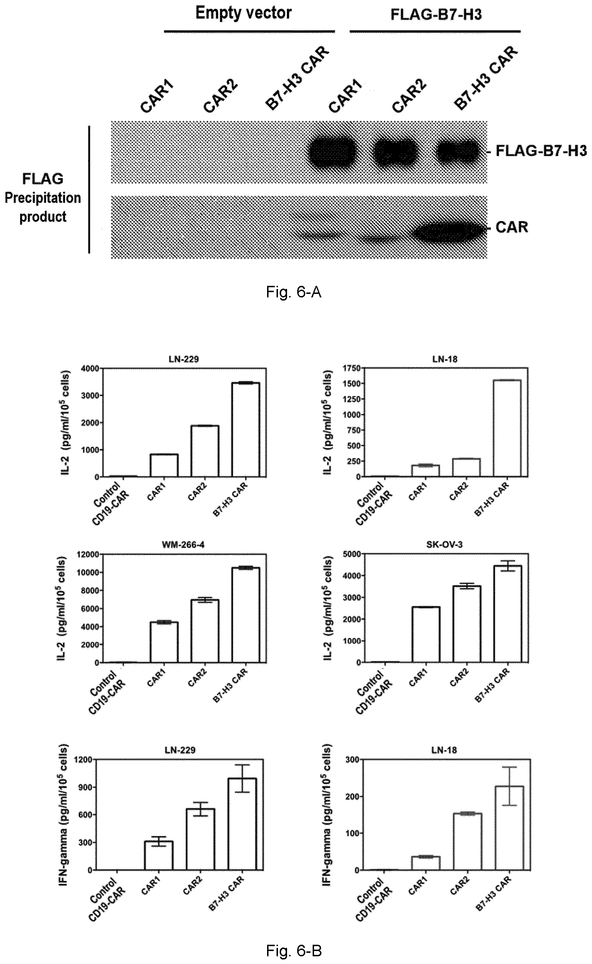

Results: as shown in Fig. 6A, the three kinds of CARs can bind to B7-H3, and compared with the other two kinds of CARs, the B7-H3-targeted CAR of the present disclosure can bind to more B7-H3 antigen proteins, which indicates that the CAR of the present disclosure has higher affinity to the antigen.

### Method: enzyme-linked immunosorbent assay (ELISA)

In the experiment of co-culture of four types of tumor cells (LN-229, LN-18, WM-266-4, and SK-OV-3) with the control CD19.28 CAR-T, CAR1, CAR2, and the B7-H3.28 CAR-T of the present disclosure, after adding the 10-day transduced T cells for 24 h, part of supernatant was collected. Cytokines in the supernatant were determined by using an ELISA kit. Results: as shown in Fig. 6B that the levels of cytokines released by the control CD19.28 CAR-T cells during the co-culture with the tumor cells were below detection limit, while the three kinds of B7-H3-targeted CAR-T cells could release Th1 type cytokines (including IFNgamma, IL-2, etc.) during killing. Compared with CAR1 and CAR2, the B7-H3-targeted CAR-T of the present disclosure could obviously release higher levels of IL-2 and IFNgamma during killing the four types of tumor cells. Because the levels of these cytokines could greatly affect the final killing effect of CAR-T on tumors, the B7-H3-targeted CAR-T of the present disclosure showed significantly better killing effect on tumors compared with the two existing B7-H3-targeted CAR-T.

### Example 7: experiment of the in situ tumor xenograft mouse models

To determine the killing effect of the B7H3 CAR-T cells of the present invention on tumors in vivo, we chose nude mouse models of brain glioma. Human tumor cells (U-87MG or LN-229 cells) labelled with luciferase were injected into the brains of 6-8-week-old nude mice, and after tumors were grafted, the solvent PBS, the control CD19.28 CAR-T cells, the B7-H3.BB CAR-T cells, and the B7-H3.28 CAR-T cells were injected in situ, and the tumor-removal effects of the T cells and survival rates of the treated mice were detected by imaging.

Method: the in vivo experiment of CAR-T cells targeting glioma was completed in 6-8-week-old nude mice. Tumor cell lines (U-87MG cells) labelled with GFP-FFLuc were implanted into the brains of the nude mice through stereotaxis. 2 weeks later, the grafted tumors were determined in an in-vivo-imaging system, and the solvent PBS, and the CD19.28 CAR-T cells, the B7-H3.BB CAR-T cells and the B7-H3.28 CAR-T cells that were transduced for 10 days were injected in situ. Then, imaging was performed once a week to determine the killing effect of the T cells on the tumors.

In an in vivo survival experiment, LN-229 cells labelled with GFP-FFLuc were injected in situ into brains of mice through stereotaxis, and 2 weeks later, the solvent PBS, the control CD19.28 CAR-T cells, the B7-H3.BB CAR-T cells, and the B7-H3.28 CAR-T cells were injected in situ. Then, the body weight of the mice was measured weekly, and the time of death of the mice was recorded.

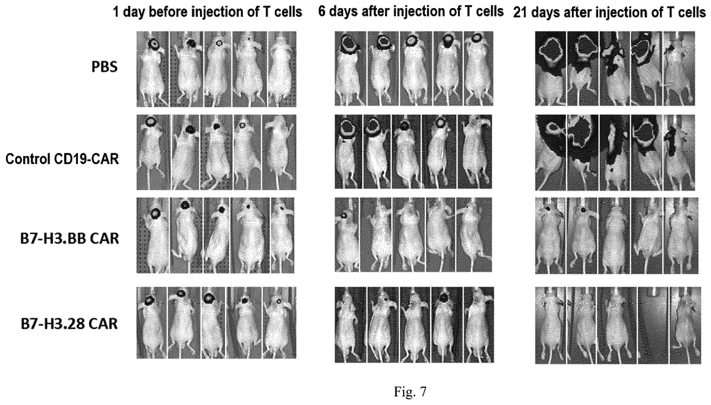

Results: as shown in Fig. 7, compared with the continuously growing tumors in the mice in the PBS group and the control CD19.28 CAR-T group, the B7-H3-targeted CAR-T cells (B7-H3.BB CAR-T and B7-H3.28 CAR-T) could remove the tumors formed by the U-87MG cells in most of the mice, and control tumor recurrence within a certain period. These results indicate that the T cells expressing the B7-H3-targeted chimeric antigen receptor can kill tumors in vivo.

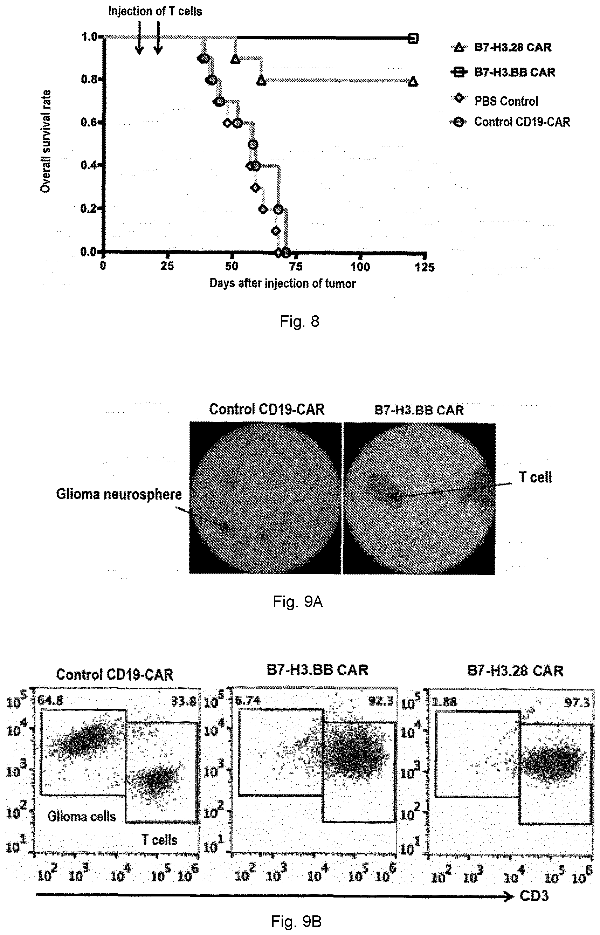

Meanwhile, we determined the survival rates of mice with glioma formed by LN-229 that were treated with the control and the T cells expressing the B7-H3-targeted chimeric antigen receptor. As shown in Fig. 8, compared with the control PBS and CD19.28 CAR-T, the T cells expressing the B7-H3-targeted chimeric antigen receptor (B7-H3.BB CAR-T and B7-H3.28 CAR-T) of the present disclosure significantly prolonged the survival time of mice, which indicate that the T cells expressing the B7-H3-targeted chimeric antigen receptor of the present disclosure can be used as a potential tumor therapy.

### Example 8: Killing effects of the B7-H3-targeted chimeric antigen receptor-T cells on glioma from patients

### 1. Killing effect of the B7-H3-targeted chimeric antigen receptor-T cells on glioma neurospheres from patients in vitro

In order to verify the killing effect of the T cells expressing the B7-H3-targeted chimeric antigen receptor of the present disclosure on tumors from patients, we isolated glioma stem cells from glioma patients and cultured them into glioma neurospheres. The glioma neurospheres were then co-cultured with the T cells expressing the control (CD19-CAR) and the T cells expressing the B7-H3-targeted chimeric antigen receptor (B7-H3.BB CAR or B7-H3.28 CAR), and then killing effects of the T cells on the glioma neurospheres of patients were observed by using a microscope and detected by flow cytometry.

Method: after glioma tissues of patients were obtained, glioma stem cells were isolated by using tissue dissociation kits, and cultured in glioma complete media to obtain glioma neurospheres. The glioma neurosphere cells of the patient were then inoculated into a 24-well plate according to a density of 1-2×10⁵ cells per well, and 24 h later, a corresponding number of CAR-T cells transduced for 10 days were added in a ratio of the T cells to the tumor cells of 1: 1. 5-7 days later, the cells in the wells were observed and collected, and percentage of the remaining tumor cells were determined by flow cytometry. The CAR-T cells were detected by using mouse-anti-human CD3 fluorescent antibodies, and the cells without labelling were glioma neurosphere cells.

Results: as shown in Fig. 9, the control CD19.28 CAR-T cells could not kill the glioma neurospheres, while B7-H3.BB CAR-T and B7-H3.28 CAR-T of the present disclosure could effectively kill the glioma neurospheres, and the CAR-T cells exhibited obvious accumulation and proliferation.

### 2. Killing effects of the B7-H3-targeted chimeric antigen receptor-T cells on glioma neurospheres from patients in vivo

To further confirm in vivo killing effects of the T cells expressing the B7-H3-targeted chimeric antigen receptor on tumors from patients, we used the patient-derived glioma neurospheres to establish in situ tumor xenograft mouse model. The patient-derived glioma neurosphere cells were injected in situ into the brains of nude mice, and 2 weeks later, similarly, the solvent PBS, the control CD19.28 CAR-T cells, the B7-H3.BB CAR-T cell, and the B7-H3.28 CAR-T cells were injected into the brains, and then the survival rates of the treated mice in each group were recorded.

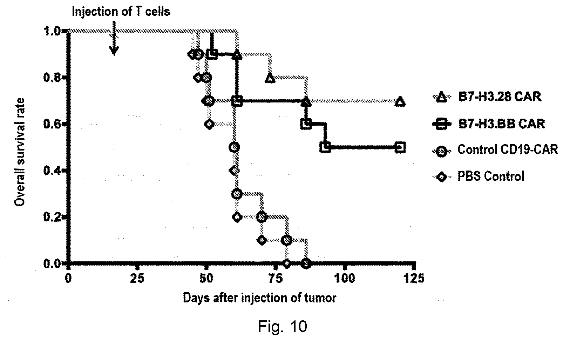

Method: an in vivo experiment of the CAR-T cells targeting patient-derived glioma neurospheres were completed in 6-8-week-old nude mice. Glioma neurosphere cells labelled with GFP-FFLuc were injected in situ into the brains of nude mice by using a stereotaxic instrument, and 2 weeks later, the solvent PBS, the control CD19.28 CAR-T cells, the B7-H3.BB CAR-T cells, and the B7-H3.28 CAR-T cells were injected in situ. Then, the body weight of the mice was measured weekly, and the time of death of the mice was recorded. Results: as shown in Fig. 10, compared with the controls PBS and CD19.28 CAR-T, the T cells expressing the B7-H3-targeted chimeric antigen receptor (B7-H3.BB CAR-T and B7-H3. 28 CAR-T) of the present disclosure could significantly prolong the survival time of the tumor-bearing mice, which indicates that the T cells expressing the B7-H3-targeted chimeric antigen receptor of the present disclosure have the potential to effectively kill patient-derived glioma cells in vivo and prolong the survival time of patients.

### Example 9: killing effects of other 9 kinds of murine-derived B7-H3-targeted chimeric antigen receptor-T cells on tumor cells in the in vitro co-culture system

### 1. Killing effects of 9 kinds of murine-derived B7-H3-targeted chimeric antigen receptor-T cells on tumor cells

In addition to the humanized single-chain antibody cloned from 15C11, the present disclosure also includes other 9 murine-derived single-chain antibodies that recognize the B7-H3 antigen (named as: 53E9D7, 15C11C3, 6E4D9, 31F3E5, 21H2E11, 24A11A11, 42B5C12, 14A7A8, and 41C10E7, respectively). In order to determine that these murine-derived chimeric antigen receptors could also mediate T cells to kill tumor cells highly expressing B7-H3, we designed a co-culture experiment to test and compare killing effects of these murine-derived CAR-T cells on tumor cells (LN-229). After tumor cell attachment, we added the control CD19.28 CAR-T cells and the 9 kinds of murine-derived B7-H3.28 CAR-T cells, and then killing effects of the CAR-T cells on the tumor cells were determined by flow cytometry.

Method: LN-229 cells labelled with GFP were inoculated into a 24-well plate with a density of 2×10⁵ cells per well, and 24 h later, a corresponding number of the CD19.28 CAR-T cells and 9 kinds of different B7-H3.28 CAR-T cells transduced for 3 days were added according to a ratio of T cells to tumor cells of 1: 1. 5 days later, all cells in the wells were collected, and the percentage of the remaining LN-229 cells was determined by flow cytometry. T cells were detected by using mouse-anti-human CD3 fluorescent antibody to bind and stain, and the LN-229 cells were detected by spontaneous GFP fluorescence.

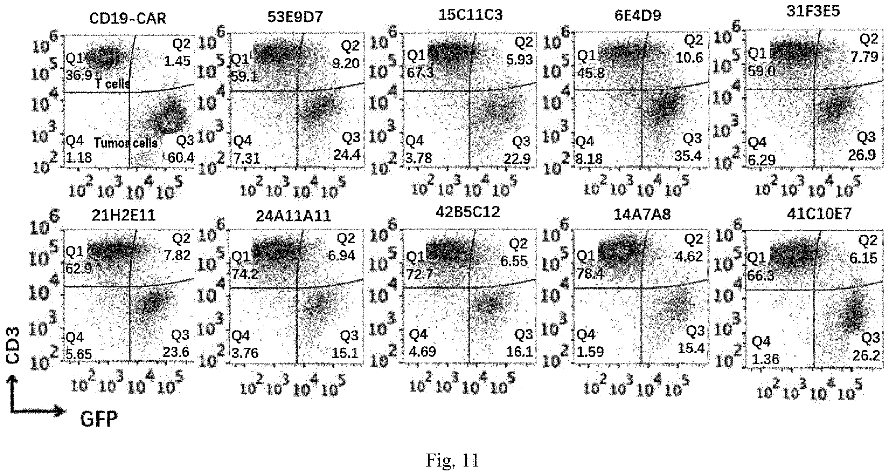

Results: as shown in Fig. 11, the control CD19.28 CAR-T cells did not show any obvious killing effect on the LN-229 cells, and the other 9 kinds of murine-derived B7-H3-targeted chimeric antigen receptor-T cells of the present disclosure showed killing effects on the tumor cells during the co-culture with the LN-229 cells, and meanwhile, the B7-H3-targeted CAR-T cells exhibited some proliferation.

### 2. Cytokine release of the 9 kinds of murine-derived B7-H3-targeted chimeric antigen receptor-T cells during killing tumor cells

In the co-culture experiment of the T cells and the tumor cells, we also detected the cytokine released by the 9 kinds of murine-derived B7-H3-targeted CAR-T cells during killing the tumor cells.

### Method: enzyme-linked immunosorbent assay (ELISA)

The CAR-T cells (CD19.28 CAR-T and 9 kinds of murine-derived B7-H3.28 CAR-T) were added in the co-culture experiments with the tumor cells (LN-229 and WM-266-4), and 24 h later, a part of the culture supernatant was collected. Cytokines in the supernatant were detected by using an ELISA kit.

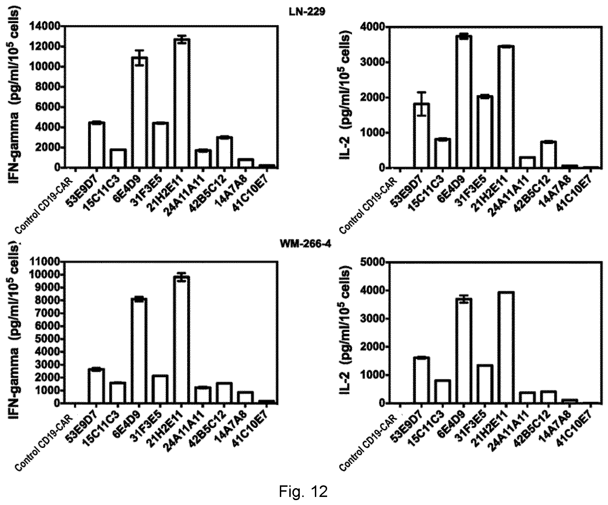

Results: As shown in Fig. 12 that the cytokines released by the control CD19.28 CAR-T cells were below the detection limit, and the 9 kinds of murine-derived B7-H3-targeted chimeric antigen receptor-T cells released different levels of Th1 type cytokines (including IFNgamma, IL-2, etc.) during killing the tumor cells, which indicates that the 9 kinds of CAR-T cells have the ability of killing tumor cells and activating other tumor killing mechanisms.

## Claims

1. A B7-H3-targeting chimeric antigen receptor (CAR), **characterized by** comprising: an anti-B7-H3 binding domain, a hinge region, a transmembrane domain, and a signal transduction domain.

2. The CAR according to claim 1, wherein the anti-B7-H3 binding domain comprises an anti-B7-H3 single-chain antibody or antigen-binding portion, and the antibody or antigen-binding portion comprises a light-chain CDR selected from amino acid sequences of SEQ ID NO. 5-7, 17-19, 29-31, 41-43, 53-55, 65-67, 77-79, 89-91, 101-103, 113-115 or any variant thereof;
and/or, comprises a heavy-chain CDR selected from amino acid sequences of SEQ ID NO. 10-12, 22-24, 34-36, 46-48, 58-60, 70-72, 82-84, 94-96, 106-108, 118-120 or any variant thereof.

3. The CAR according to claim 1 or 2, wherein the anti-B7-H3 binding domain comprises an anti-B7-H3 antibody or antigen-binding portion, and the antibody or antigen-binding portion comprises a light-chain CDR1 selected from amino acid sequences of SEQ ID NO. 5, 17, 29, 41, 53, 65, 77, 89, 101, 113 or any variant thereof, a light-chain CDR2 selected from amino acid sequences of SEQ ID NO. 6, 18, 30, 42, 54, 66, 78, 90, 102, 114 or any variant thereof, a light-chain CDR3 selected from amino acid sequences of SEQ ID NO. 7, 19, 31, 43, 55, 67, 79, 91, 103, 115 or any variant thereof;
and/or, comprises a heavy-chain CDR1 selected from amino acid sequences of SEQ ID NO. 10, 22, 34, 46, 58, 70, 82, 94, 106, 118 or any variant thereof, a heavy-chain CDR2 selected from amino acid sequences of SEQ ID NO. 11, 23, 35, 47, 59, 71, 83, 95, 107, 119 or any variant thereof, a heavy-chain CDR3 selected from amino acid sequences of SEQ ID NO. 12, 24, 36, 48, 60, 72, 84, 96, 108, 120 or any variant thereof.

4. The CAR according to any one of the preceding claims, wherein the anti-B7-H3 binding domain comprises an anti-B7-H3 antibody or antigen-binding portion, and the antibody or antigen-binding portion comprises a light-chain sequence selected from amino acid sequences of SEQ ID NO.4, 16, 28, 40, 52, 64, 76, 88, 100, 112 or any variant thereof; and/or, comprises a heavy-chain sequence selected from amino acid sequences of SEQ ID NO. 9, 21, 33, 45, 57, 69, 81, 93, 105, 117 or any variant thereof.

5. The CAR according to any one of the preceding claims, wherein the anti-B7-H3 binding domain comprises an anti-B7-H3 antibody (scFv) domain; preferably, the anti-B7-H3 single-chain antibody has an amino acid sequence selected from amino acid sequences of SEQ ID NO. 2, 14, 26, 38, 50, 62, 74, 86, 98, 110 or any variant thereof.

6. The CAR according to any one of the preceding claims, wherein the transmembrane domain is selected from one or more of transmembrane domains of α, β, ζ chains of TCR, CD3y, CD3δ, CD3ε, CD3ζ, CD4, CD5, CD8α, CD9, CD16, CD22, CD27, CD28, CD33, CD37, CD45, CD64, CD80, CD86, CD134, 4-1BB, CD152, CD154, PD-1; preferably, the transmembrane domain is selected from one or more of transmembrane domains of CD8α, CD4, CD45, PD-1, CD154, CD28; preferably, the transmembrane domain is selected from one or more of transmembrane domains of CD8α or CD28; more preferably, the transmembrane domain has an amino acid sequence selected from amino acid sequences of SEQ ID NO. 124 or any variant thereof;
the hinge region is selected from one or more of an extracellular hinge region of CD8, a hinge region of IgG1 Fc CH2CH3, a hinge region of IgD, an extracellular hinge region of CD28, a hinge region of IgG4 Fc CH2CH3, and an extracellular hinge region of CD4; preferably, the hinge region is a hinge region of CD8α; more preferably, the hinge region has an amino acid sequence selected from amino acid sequences of SEQ ID NO. 122 or any variant thereof.

7. The CAR according to any one of the preceding claims, wherein the signal transduction domain is selected from one or more of intracellular signaling regions of TCRζ, FcRy, FcRβ, CD3γ, CD3δ, CD3ε, CD5, CD22, CD79a, CD79b, CD278 (ICOS), CD66d, CD3ζ; preferably, the signal transduction domain is selected from an intracellular signaling region of CD3ζ; more preferably, the signal transduction domain has an amino acid sequence selected from amino acid sequences of SEQ ID NO. 130 or any variant thereof;
preferably, the signal transduction domain further comprises one or more costimulatory domains; preferably, the costimulatory domain is selected from one or more of intracellular signaling regions of CARD11, CD2, CD7, CD27, CD28, CD30, CD40, CD54, CD83, OX40, 4-1BB, CD134, CD150, CD152, CD223, CD270, PD-L2, PD-L1, CD278, DAP10, LAT, NKD2C, SLP76, TRIM, FcεRIγ, MyD88, 4-1BB; preferably, the costimulatory domain is selected from intracellular signaling regions of 4-1BB, CD134, CD28, OX40; preferably, the costimulatory domain is selected from one or more of intracellular signaling regions of 4-1BB or CD28; more preferably, the costimulatory domain has an amino acid sequence selected from amino acid sequences of SEQ ID NO. 126, 128 or any variant thereof.

8. An isolated nucleic acid molecule, comprising a polynucleotide sequence encoding the CAR according to any one of claims 1 to 7; preferably, the nucleic acid molecule has a nucleotide sequence selected from nucleotide sequences of SEQ ID NO. 1, 13, 25, 37, 49, 61, 73, 85, 97, 109 or any variant thereof.

9. A nucleic acid construct, comprising the nucleic acid molecule according to claim 8; preferably, the nucleic acid construct is a viral vector; more preferably, the viral vector is one or more of a retroviral vector, a lentiviral vector, an adenoviral vector, an adeno-associated viral vector.

10. A virus, comprising the nucleic acid molecule according to claim 8, or comprising the nucleic acid construct according to claim 9; preferably, the virus is one or more of a retrovirus, a lentivirus, an adenovirus, an adeno-associated virus.

11. Use of the CAR according to any one of claims 1 to 7, the nucleic acid molecule according to claim 8, the nucleic acid construct according to claim 9, or the virus according to claim 10 in preparation of genetically modified immune cells targeting tumor cells expressing B7-H3.

12. An isolated host cell, expressing the CAR according to any one of claims 1 to 7, or comprising the isolated nucleic acid molecule according to claim 8, or comprising the nucleic acid construct according to claim 9, or comprising the virus according to claim 10; preferably, the host cell is a mammalian cell; more preferably, the host cell is one or more of a T cell, an NK cell or cell line, a γδT cell, an NKT cell, a macrophage or cell line, a PG13 cell line; more preferably, the host cell is a T cell; the most preferably, the host cell is a primarily cultured T cell.

13. The host cell according to claim 12, wherein the host cell further expresses other effector molecules, the other effector molecules comprise but are not limited to a cytokine, another CAR, a chemokine receptor, an siRNA reducing the expression of PD-1 or a protein blocking PD-L1, a TCR or a safety switch;
preferably, the cytokine is selected from one or more of TNF-α, TNF-β, VEGF, TPO, NGF-β, PDGF, TGF-α, TGF-β, IGF-I, IGF-II, EPO, M-CSF, IL-1, IL-1α, IL-2, IL-3, IL-4, IL-5, IL-6, IL-7, IL-8, IL-9, IL-10, IL-11, IL-12, IL-13, IL-14, IL-15, IL-16, IL-17, IL-18, IL-21, IL-25, LIF, FLT-3, an interferon, an angiostatin, a thrombospondin, an endostatin;
preferably, the chemokine receptor is selected from one or more of CCR2, CCR5, CXCR2, CXCR4;
preferably, the safety switch is selected from one or more of HSVTK, VZVTK, iCaspase-9, iCaspase-1, iCaspase-8, truncated EGFR, RQR8.

14. A pharmaceutical composition, comprising one or more of the CAR according to any one of claims 1 to 7, the nucleic acid molecule according to claim 8, the nucleic acid construct according to claim 9, the virus according to claim 10, the host cell according to claim 12 or 13, and a pharmaceutically acceptable carrier.

15. Use of the CAR according to any one of claims 1 to 7, the nucleic acid molecule according to claim 8, the nucleic acid construct according to claim 9, the virus according to claim 10, the host cell according to claim 12 or 13, or the pharmaceutical composition according to claim 14 in disease diagnosis, treatment or prevention, the disease is selected from one or more of cancer, an inflammatory disease, an autoimmune disease;
preferably, the disease is a tumor expressing B7-H3; more preferably, the disease is selected from one or more of brain cancer, pancreatic cancer, ovarian cancer, kidney cancer, bladder cancer, skin cancer, stomach cancer, intestinal cancer, head and neck cancer, thyroid cancer, prostate cancer, lung cancer, and Kaposi's sarcoma; more preferably, the brain cancer is selected from one or more of glioblastoma, astrocytoma, meningioma, oligodendroglioma, neuroglioma.

16. Use of the CAR according to any one of claims 1 to 7, the nucleic acid molecule according to claim 8, the nucleic acid construct according to claim 9, the virus according to claim 10, the host cell according to claim 12 or 13, or the pharmaceutical composition according to claim 14 in preparation of a drug; preferably, the drug is used to diagnose, treat or prevent one or more of cancer, an inflammatory disease, an autoimmune disease;
preferably, the drug is used to diagnose, treat or prevent a tumor expressing B7-H3; more preferably, the drug is used to diagnose, treat or prevent one or more selected from brain cancer, pancreatic cancer, ovarian cancer, kidney cancer, bladder cancer, skin cancer, stomach cancer, intestinal cancer, head and neck cancer, thyroid cancer, prostate cancer, lung cancer, and Kaposi's sarcoma; more preferably, the brain cancer is selected from one or more of glioblastoma, astrocytoma, meningioma, oligodendroglioma, neuroglioma.
